# EUROPEAN PATENT SPECIFICATION

(11) **EP 1 363 910 B1**
(45) Date of publication and mention of the grant of the patent: **01.03.2006**
(21) Application number: 01992298.8
(22) Date of filing: 07.11.2001
(51) Int. Cl.: C07D 487/04, C07D 519/00, A61K 31/53, A61P 29/00, C07D 253/00, C07D 209/00, C07D 487/00, C07D 471/00

(54) **METHODS OF TREATING P38 KINASE-ASSOCIATED CONDITIONS AND PYRROLOTRIAZINE COMPOUNDS USEFUL AS KINASE INHIBITORS**
Verfahren zur Behandlung von mit p38-Kinase assoziierten Leiden und Pyrrolotriazin-Verbindungen als Kinaseinhibitoren.
METHODES DE TRAITEMENT DES PATHOLOGIES LIEES A LA KINASE P38 ET COMPOSES DE PYRROLOTIAZINE UTILISES EN TANT QU'INHIBITEURS DE LA KINASE

(30) Priority: 17.11.2000 US 249877 P; 07.08.2001 US 310561 P
(43) Date of publication of application: 26.11.2003
(73) Proprietor: Bristol-Myers Squibb Company, Princeton, NJ 08543-4000 (US)
(72) Inventor: LEFTHERIS, Katerina, Skillman, NJ 08558 (US); BARRISH, Joel, Richboro, PA 18954 (US); HYNES, John, Washington Crossing, PA 18977 (US); WROBLESKI, Stephen, T., Whitehouse Station, NJ 08889 (US)
(74) Representative: Vossius & Partner
(86) International application number: PCT/US2001/049982
(87) International publication number: WO 2002/040486

(56) References cited:
- EP-A- 0 713 876
- WO-A-00/71129
- WO-A-99/24033

## Description

This invention relates to the use of pyrrolotriazine compounds for treating conditions associated with p38α and β kinases and to pyrrolotriazine compounds, more particularly, to pyrrolotriazine carboxamide and benzamide compounds useful for treating p38 kinase-associated conditions.

A large number of cytokines participate in the inflammatory response, including IL-1, IL-6, IL-8 and TNF-α. Overproduction of cytokines such as IL-1 and TNF-α are implicated in a wide variety of diseases, including inflammatory bowel disease, rheumatoid arthritis, psoriasis, multiple sclerosis, endotoxin shock, osteoporosis, Alzheimer's disease, and congestive heart failure, among others [Henry *et al.,* Drugs Fut., 24:1345-1354 (1999); Salituro *et al.,* Curr. Med. Chem., 6:807-823 (1999)]. Evidence in human patients indicates that protein antagonists of cytokines are effective in treating chronic inflammatory diseases, such as, for example, monoclonal antibody to TNF-α (Enbrel) [Rankin *et al.,* Br. J. Rheumatol., 34:334-342 (1995)], and soluble TNF-α receptor-Fc fusion protein (Etanercept) [Moreland *et al.,* Ann. Intern. Med., 130:478-486 (1999)].

The biosynthesis of TNF-α occurs in many cell types in response to an external stimulus, such as, for example, a mitogen, an infectious organism, or trauma. Important mediators of TNF-α production are the mitogen-activated protein (MAP) kinases, and in particular, p38 kinase. These kinases are activated in response to various stress stimuli, including but not limited to proinflammatory cytokines, endotoxin, ultraviolet light, and osmotic shock. Activation of p38 requires dual phosphorylation by upstream MAP kinase kinases (MKK3 and MKK6) on threonine and tyrosine within a Thr-Gly-Tyr motif characteristic of p38 isozymes.

There are four known isoforms of p38, i.e., p38-α, p38β, p38γ, and p38δ. The a and β isoforms are expressed in inflammatory cells and are key mediators of TNF-α production. Inhibiting the p38α and β enzymes in cells results in reduced levels of TNF-α expression. Also, administering p38α and β inhibitors in animal models of inflammatory disease has proven that such inhibitors are effective in treating those diseases. Accordingly, the p38 enzymes serve an important role in inflammatory processes mediated by IL-1 and TNF-α. Compounds that reportedly inhibit p38 kinase and cytokines such as EL-1 and TNF-α for use in treating inflammatory diseases are disclosed in US Pats. Nos. 6,277,989 and 6,130,235 to Scios, Inc; US Pats. Nos. 6,147,080 and 5,945,418 to Vertex Pharmaceuticals Inc; US Pats Nos. 6,251,914, 5,977,103 and 5,658,903 to Smith-Kline Beecham Corp.; US Pats. Nos. 5,932,576 and 6,087,496 to G.D. Searle & Co.; WO 00/56738 and WO 01/27089 to Astra Zeneca; WO 01/34605 to Johnson & Johnson; WO 00/12497 (quinazoline derivatives as p38 kinase inhibitors); WO 00/56738 (pyridine and pyrimidine derivatives for the same purpose); WO 00/12497 (discusses the relationship between p38 kinase inhibitors); and WO 00/12074 (piperazine and piperidine compounds useful as p38 inhibitors).

The present invention relates to the use of certain pyrrolotriazine compounds for treating conditions associated with p38 kinase activity. The invention further provides select pyrrolotriazine compounds, including 5-methyl and 5-trifluoromethyl pyrrolotriazine-6-cardoxamide compounds useful as kinase inhibitors, particularly kinases p38α and β. Pyrrolotdazine compounds useful as tyrosine kinase inhibitors are disclosed in US patent application Serial No. 09/573,829 filed May 18,2000, assigned to the present assignee, and in WO 00/71129. Pyrrolotriazine compounds substituted with an acidic group reportedly having sPLA₂-inhibitory activity are disclosed in WO 01/14378 Al to Shionogi & Co., Ltd, published March 1, 2001 in Japanese. Each of the patent applications, patents and publications referred to herein is incorporated herein by reference.

The instant invention is directed to the use of one or more pharmaceutically-active compounds having the Formula (I): 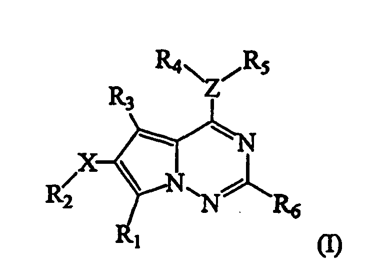 or a pharmaceutically acceptable salt, prodrug, or solvate thereof, wherein:
- R₃: is hydrogen, methyl, perfluoromethyl, methoxy, halogen, cyano, or NH₂;
- X: is selected from -O-, -OC(=O) -S-, -S(=O)-, -SO₂-, -C(=O)-, -CO₂-, -NR₁₀-, NR₁₀C(=O)-, -NR₁₀C(=O)NR₁₁-, -NR₁₀CO₂-, -NR₁₀SO₂-, -NR₁₀SO₂NR₁₁-, -SO₂NR₁₀-, -C(=O)NR₁₀-, halogen, nitro, and cyano, or X is absent;
- Z: is selected from O, S, N, and CR₂₀, wherein when Z is CR₂₀, said carbon atom may form an optionally-substituted bicyclic aryl or heteroaryl with R₄ and R₅;
- R₁: is hydrogen, -CH₃, -OH, -OCH₃, -SH, -SCH₃, -OC(=O)R₂₁, -S(=O)R₂₂, -SO₂R₂₂, -SO₂NR₂₄R₂₅, -CO₂R₂₁, -C(=O)NR₂₄R₂₅, -NH₂, -NR₂₄R₂₅, -NR₂₁SO₂NR₂₄R₂₅, -NR₂₁SO₂R₂₂, -NR₂₄C(=O)R₂₅, -NR₂₄CO₂R₂₅, NR₂₁C(=O)NR₂₄R₂₅, halogen, nitro, or cyano;
- R₂: is selected from:
a) hydrogen, provided that R₂ is not hydrogen if X is -S(=O)-, -SO₂-, -NR₁₀CO₂-, or -NR₁₀SO₂-;
b) alkyl, alkenyl, and alkynyl optionally substituted with up to four R₂₆;
c) aryl and heteroaryl optionally substituted with up to three R₂₇; and
d) heterocyclo and cycloalkyl optionally substituted with keto (=O), up to three R₂₇, and/or having a carbon-carbon bridge of 3 to 4 carbon atoms; or
e) R₂ is absent if X is halogen, nitro, or cyano;
- (i) R₄: is substituted aryl, aryl substituted with NHSO₂alkyl, substituted heteroaryl, or an optionally-substituted bicyclic 7-11 membered saturated or unsaturated carbocyclic or heterocyclic ring, and
R₅ is hydrogen, alkyl, or substituted alkyl, except when Z is O or S, R₅ is absent, or alternatively,
- (ii) R₄: and R₅ taken together with Z form an optionally-substituted bicyclic 7-11 membered aryl or heteroaryl;
- R₆: is hydrogen, alkyl, substituted alkyl, aryl, substituted aryl, heterocyclo, substituted heterocyclo, -NR₇R₈, -OR₇, or halogen;
- R₁₀ and R₁₁: are independently selected from hydrogen, alkyl, substituted alkyl, aryl, substituted aryl, cycloalkyl, substituted cycloalkyl, heterocyclo, and substituted heterocyclo;
- R₇, R₈, R₂₁, R₂₄, and R₂₅: are independently selected from hydrogen, alkyl, substituted alkyl, aryl, substituted aryl, heterocyleo, and substituted heterocyclo;
- R₂₀: is hydrogen, lower alkyl, or substituted alkyl, or R₂₀ may be absent if the carbon atom to which it is attached together with R₄ and R₅ is part of an unsaturated bicyclic aryl or heteroaryl;
- R₂₂: is alkyl, substituted alkyl, aryl, substituted aryl, heterocyclo, or substituted heterocyclo;
- R₂₆: is selected from halogen, trifluoromethyl, haloalkoxy, keto (=O), nitro, cyano, -SR₂₈, -OR₂₈, -NR₂₈R₂₉, -NR₂₈SO₂, -NR₂₈SO₂R₂₉, -SO₂R₂₈, -SO₂NR₂₈R₂₉, -CO₂R₂₈, -C(=O)R₂₈, -C(=O)NR₂₈R₂₉, -OC(=O)R₂₈, -OC(=O)NR₂₈R₂₉, -NR₂₈C(=O)R₂₉, -NR₂₈CO₂R₂₉, =N-OH, =N-O-alkyl; aryl optionally substituted with one to three R₂₇; cycloalkyl optionally substituted with keto(=O), one to three R₂₇, or having a carbon-carbon bridge of 3 to 4 carbon atoms; and heterocyclo optionally substituted with keto (=O), one to three R₂₇, or having a carbon-carbon bridge of 3 to 4 carbon atoms; wherein R₂₈ and R₂₉ are each independently selected from hydrogen, alkyl, alkenyl, aryl, aralkyl, C₃₋₇cycloalkyl, and C₃₋₇heterocycle, or may be taken together to form a C₃₋₇-heterocycle; and wherein each R₂₈ and R₂₉ in turn is optionally substituted with up to two of alkyl, alkenyl, halogen, haloalkyl, haloalkoxy, cyano, nitro, amino, hydroxy, alkoxy, alkylthio, phenyl, benzyl, phenyloxy, and benzyloxy; and
- R₂₇,: is selected from alkyl, R₃₂ and C₁₋₄alkyl substituted with one to three R₃₂ wherein each R₂ group is independently selected from halogen, haloalkyl, haloalkoxy, nitro, cyano, -SR₃₀, -OR₃₀, -NR₃₀R₃₁, -NR₃₀SO₂, -NR₃₀SO₂R₃₁, -SO₂R₃₀, -SO₂NR₃₀R₃₁, -CO₂R₃₀, -C(=O)R₃₀, -C(=O)NR₃₀R₃₁ -OC(=O)R₃₀. -OC(=O)NR₃₀R₃₁, -NR₃₀C(=O)R₃₁, -NR₃₀COCO₂R₃₁, and a 3 to 7 membered carbocyclic or heterocyclic ring optionally substituted with alkyl, halogen, hydroxy, alkoxy, haloalkyl, haloalkoxy, nitro, amino, or cyano, wherein R₃₀ and R₃₁ are each independently selected from hydrogen, alkyl, alkenyl, aryl, aralkyl, C₃₋₇cycloalkyl, and heterocycle, or may be taken together to form a C₃₋₇heterocycle for the manufacture of a medicament for treating one or more conditions associated with p38 kinase activity.

The invention is further directed to compounds having surprisingly advantageous activity as inhibitors of p38 kinases α and β and TNF-α comprising compounds of Formula (II): 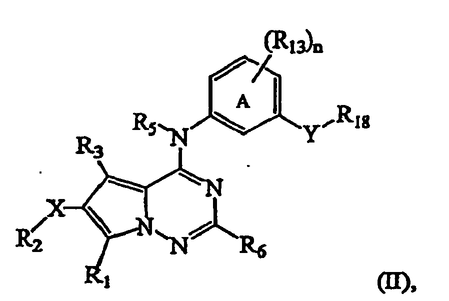 and pharmaceutically acceptable salts, prodrugs or solvates thereof, wherein:
- R₃: is methyl -CF₃, or -OCH₃;
- R₅: is hydrogen or alkyl;
- Y: is -C(=O)NR₂₃-, -NR₂₃C(=O)NR₂₃-, -NR₂₃SO₂-, or -SO₂NR₂₃-;
- R₁₈ and R₂₃: are selected from hydrogen, alkyl, alkoxy, aryl, and aryl substituted with one to three R₁₉, except when Y is -NR₂₃SO₂-, R₁₈ is C₁₋₄alkyl or aryl , optionally substituted with one to three R₁₉;
- R₁₃ and R₁₉: at each occurrence are independently selected from alkyl, halo, trifluoromethoxy, trifluoromethyl, hydroxy, alkoxy, alkanoyl, alkanoyloxy, thiol, alkylthio, ureido, nitro, cyano, carboxy, carboxyalkyl, carbamyl, alkoxycarbonyl, alkylthiono, arylthiono, arylsulfonylamine, alkylsulfonylamine, sulfonic acid, alkysulfonyl, sulfonamido, and aryloxy, wherein each R₁₃ and/or R₁₉ group may be further substituted by hydroxy, alkyl, alkoxy, aryl, or aralkyl; and

X, R₁, R₂, and R₆ are as defined above for compounds of Formula (I); excluding 4-[[3-(Aminosulfonyl)-4-methylphenyl]amino]-5-methylpyrrolo[2,1-f][1,2,4]triazine-6-carboxylic acid methyl ester.

Listed below are definitions of various terms used to describe this invention. These definitions apply to the terms as they are used throughout this specification, unless otherwise limited in specific instances, either individually or as part of a larger group.

The term "alkyl" refers to straight or branched chain unsubstituted hydrocarbon groups of 1 to 20 carbon atoms, preferably 1 to 7 carbon atoms. The expression "lower alkyl" refers to unsubstituted alkyl groups of 1 to 4 carbon atoms. When a subscript is used with reference to an alkyl or other group, the subscript refers to the number of carbon atoms that the group may contain. The term "C₀₋₄alkyl" includes a bond and alkyl groups of I to 4 carbon atoms.

The term "substituted alkyl" refers to an alkyl group substituted by one to four substituents selected from halo, hydroxy, alkoxy, oxo (=O), alkanoyl, aryloxy, alkanoyloxy, amino, alkylamino, arylamino, aralkylamino, disubstituted amines in which the 2 amino substituents are selected from alkyl, aryl or aralkyl; alkanoylamino, aroylamino, aralkanoylamino, substituted alkanoylamino, substituted arylamino, substituted aralkanoylamino, thiol, alkylthio, arylthio, aralkylthio, alkylthiono, arylthiono, aralkylthiono, alkylsulfonyl, arylsulfonyl, aralkylsulfonyl, sulfonamido, *e.g.* SO₂NH₂, substituted sulfonamido, nitro, cyano, carboxy, carbamyl, e.g. CONH₂, substituted carbamyl e.g. CONHalkyl, CONHaryl, CONHaralkyl or cases where there are two substituents on the nitrogen selected from alkyl, aryl or aralkyl; alkoxycarbonyl, aryl, substituted aryl, guanidino and substituted or unsubstituted heterocyclos, such as indolyl, imidazolyl, furyl, thienyl, thiazolyl, pyrrolidyl, pyridyl, pyrimidyl and the like. Where the substituent on the alkyl is further substituted, it will be with alkyl, alkoxy, aryl, or aralkyl.

When the term alkyl is used in connection with another group, as in heterocycloalkyl or cycloalkylalkyl, this means the identified group is bonded directly through an alkyl group which may be branched or straight chain. In the case of substituents, as in "substituted cycloalkylalkyl," the alkyl portion of the group may, besides being branched or straight chain, be substituted as recited above for substituted alkyl groups and/or the connected group may be substituted as recited herein for that group.

The term "halogen" or "halo" refers to fluorine, chlorine, bromine and iodine.

The term "aryl" refers to monocyclic or bicyclic aromatic hydrocarbon groups having 6 to 12 carbon atoms in the ring portion, such as phenyl, naphthyl, biphenyl and diphenyl groups. When the aryl is substituted, each ring of the aryl may be substituted.

The term "substituted aryl" refers to an aryl group substituted by one to four substituents selected from alkyl, substituted alkyl, halo, trifluommethoxy, trifluoromethyl, hydroxy, alkoxy, alkanoyl, alkanoyloxy, amino, alkylamino, aralkylamino, dialkylamino, alkanoylamino, thiol, alkylthio, ureido, nitro, cyano, carboxy, carboxyalkyl, carbamyl, alkoxycarbonyl, alkylthiono, arylthiono, arylsulfonylamine, sulfonic acid, alkysulfonyl, sulfonamido, and aryloxy. The substituent may be further substituted by hydroxy, alkyl, alkoxy, aryl, substituted aryl, substituted alkyl or aralkyl.

The term "aralkyl" refers to an aryl group bonded directly through an alkyl group, such as benzyl, wherein the alkyl group may be branched or straight chain. In the case of a "substituted aralkyl," the alkyl portion of the group may, besides being branched or straight chain, be substituted as recited above for substituted alkyl groups and/or the aryl portion may be substituted as recited for substituted aryl. Thus, the term "optionally substituted benzyl" refers to the group 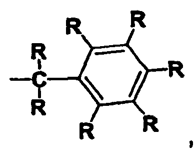 wherein each R group may be hydrogen or may also be selected from alkyl, halogen, cyano, nitro, amino, hydroxy, alkoxy, alkylthio, phenyl, benzyl, phenyloxy, and benzyloxy, and other groups recited above. At least two of these "R" groups should be hydrogen and preferably at least five of the "R" groups is hydrogen. A preferred benzyl group involves the alkyl-portion being branched to define

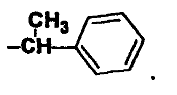

The term "heteroaryl" refers to an aromatic group for example, which is a 4 to 7 membered monocyclic, 7 to 11 membered bicyclic, or 10 to 15 membered tricyclic ring system, which has at least one heteroatom and at least one carbon atom-containing ring. Each ring of the heteroaryl group containing a heteroatom can contain one or two oxygen or sulfur atoms and/or from one to four nitrogen atoms, provided that the total number of heteroatoms in each ring is four or less and each ring has at least one carbon atom. The fused rings completing the bicyclic and tricyclic groups may contain only carbon atoms and may be saturated, partially saturated, or unsaturated. The nitrogen and sulfur atoms may optionally be oxidized and the nitrogen atoms may optionally be quaternized. Heteroaryl groups which are bicyclic or tricyclic must include at least one fully aromatic ring but the other fused ring or rings may be aromatic or non-aromatic. The heteroaryl group may be attached at any available nitrogen or carbon atom of any ring.

A "substituted heteroaryl" has one to four substituents on any one or more of the rings comprising the heteraryl group. The substituents may be selected from those recited below for heterocycle groups.

Exemplary monocyclic heteroaryl groups include pyrrolyl, pyrazolyl, pyrazolinyl, imidazolyl, oxazolyl, isoxazolyl, thiazolyl (*i.e*., 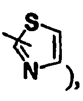 thiadiazolyl, isothiazolyl, furanyl, thienyl, oxadiazolyl, pyridyl, pyrazinyl, pyrimidinyl, pyridazinyl, triazinyl and the like.

Exemplary bicyclic heteroaryl groups include indolyl, benzothiazolyl, benzodioxolyl, benzoxaxolyl, benzothienyl, quinolinyl, tetrahydroisoquinolinyl, isoquinolinyl, benzimidazolyl, benzopyranyl, indolizinyl, benzofuranyl, chromonyl, coumarinyl, benzopyranyl, cinnolinyl, quinoxalinyl, indazolyl, pyrrolopyridyl, furopyridinyl, dihydroisoindolyl, tetrahydroquinolinyl and the like.

Exemplary tricyclic heteroaryl groups include carbazolyl, benzidolyl, phenanthrollinyl, acridinyl, phenanthridinyl, xanthenyl and the like.

The term "alkenyl" refers to straight or branched chain hydrocarbon groups of 2 to 20 carbon atoms, preferably 2 to 15 carbon atoms, and most preferably 2 to 8 carbon atoms, having one to four double bonds.

The term "substituted alkenyl" refers to an alkenyl group substituted by one to two substituents selected from halo, hydroxy, alkoxy, alkanoyl, alkanoyloxy, amino, alkylamino, dialkylamino, alkanoylamino, thiol, alkylthio, alkylthiono, alkylsulfonyl, sulfonamido, nitro, cyano, carboxy, carbamyl, substituted carbamyl, guanidino, and substituted and unsubstituted heterocycles, including indolyl, imidazolyl, furyl, thienyl, thiazolyl, pyrrolidyl, pyridyl, pyrimidyl and the like.

The term "alkynyl" refers to straight or branched chain hydrocarbon groups of 2 to 20 carbon atoms, preferably 2 to 15 carbon atoms, and most preferably 2 to 8 carbon atoms, having one to four triple bonds.

The term "substituted alkynyl" refers to an alkynyl group substituted by a substituent selected from halo, hydroxy, alkoxy, alkanoyl, alkanoyloxy, amino, alkylamino, dialkylamino, alkanoylamino, thiol, alkylthio, alkylthiono, alkylsulfonyl, sulfonamido, nitro, cyano, carboxy, carbamyl, substituted carbamyl, guanidino and substituted or unsubstituted heterocyclo, e.g. imidazolyl, furyl, thienyl, thiazolyl, pyrrolidyl, pyridyl, pyrimidyl and the like.

The term "cycloalkyl" refers to a saturated or partially unsaturated non-aromatic cyclic hydrocarbon ring system, preferably containing 1 to 3 rings and 3 to 7 carbons per ring which may be further fused with an unsaturated C₃-C₇ carbocylic ring. Exemplary groups include cyclopropyl, cyclobutyl, cyclopentyl, cyclohexyl, cycloheptyl, cycloctyl, cyclodecyl, cyclododecyl, and adamantyl. A "substituted cycloalkyl" is substituted with one or more alkyl or substituted alkyl groups as described above, or one or more groups described above as alkyl substituents.

The terms "heterocycle", "heterocyclic" and "heterocyclo" each refer to a fully saturated or unsaturated, aromatic or nonaromatic cyclic group, for example, which is a 4 to 7 membered monocyclic, 7 to 11 membered-bicyclic, or 10 to 15 membered tricyclic ring system, which has at least one heteroatom in at least one carbon atom-containing ring. Thus, the term "heterocycle" includes heteroaryl groups as described above. Each ring of the heterocyclic group containing a heteroatom may have 1, 2 or 3 heteroatoms selected from nitrogen atoms, oxygen atoms and sulfur atoms, where the nitrogen and sulfur heteroatoms may also optionally be oxidized and the nitrogen heteroatoms may also optionally be quaternized. The heterocyclic group may be attached at any heteroatom or carbon atom.

Exemplary monocyclic heterocyclic groups include pyrrolidinyl, pyrrolyl, indolyl, pyrazolyl, oxetanyl, pyrazolinyl, imidazolyl, imidazolinyl, imidazolidinyl, oxazolyl, oxazolidinyl, isoxazolinyl, isoxazolyl, thiazolyl, thiadiazolyl, thiazolidinyl, isothiazolyl, isothiazolidinyl, furyl, tetrahydrofuryl, thienyl, oxadiazolyl, piperidinyl, piperazinyl, 2-oxopiperazinyl, 2-oxopiperidinyl, 2-oxopyrrolidinyl, 2-oxazepinyl, azepinyl, 4-piperidonyl, pyridyl, N-oxo-pyridyl, pyrazinyl, pyrimidinyl, pyridazinyl, tetrahydropyranyl, morpholinyl, thiamorpholinyl, thiamorpholinyl sulfoxide, thiamorpholinyl sulfone, 1,3-dioxolane and tetrahydro-1, 1-dioxotbienyl, dioxanyl, isothiazolidinyl, thietanyl, thiiranyl, triazinyl, and triazolyl, and the like.

Exemplary bicyclic hetrocyclic groups include 2,3-dihydro-2-oxo-1H-indolyl, benzothiazolyl, benzoxazolyl, benzothienyl, quinuclidinyl, quinolinyl, quinolinyl-N-oxide, tetrahydroisoquinolinyl, isoquinolinyl, benzimidazolyl, benzopyranyl, indolizinyl, benzofuryl, chromonyl, coumarinyl, cinnolinyl, quinoxalinyl, indazolyl, pyrrolopyridyl, furopyridinyl (such as furo[2,3-c]pyridinyl, furo[3,1-b]pyridinyl] or furo[2,3-b]pyridinyl), dihydroisoindolyl, dihydroquinazolinyl (such as 3,4-dihydro-4-oxo-quinazolinyl), benzisothiazolyl, benzisoxazolyl, benzodiazinyl, benzofurazanyl, benzothiopyranyl, benzotriazolyl, benzpyrazolyl, dihydrobenzofuryl, dihydrobenzothienyl, dihydrobenzothiopyranyl, dihydrobenzothiopyranyl sulfone, dihydrobenzopyranyl, indolinyl, isochromanyl, isoindolinyl, naphthyridinyl, phthalazinyl, piperonyl, purinyl, pyridopyridyl, quinazolinyl, tetrahydroquinolinyl, thienofuryl, thienopyridyl, thienothienyl, and the like.

Also included are smaller heterocyclos, such as epoxides and aziridines.

A "substituted heterocycle" will be substituted with one or more alkyl or aralkyl groups as described above, and/or one or more groups described above as alkyl substituents.

Unless otherwise indicated, when reference is made to a specifically-named heterocyclo or heteroaryl, the reference is intended to include those systems having the maximum number of non-cumulative double bonds or less than that maximum number of double bonds. Thus, for example, the term "isoquinoline" refers to isoquinoline and tetrahydroisoquinoline. The term "diazepine" refers to a heterocyclo ring having at least one seven atom ring with two nitrogen atoms in the seven membered ring, including a fully saturated or unsaturated diazepine.

The term "heteroatoms" shall include oxygen, sulfur and nitrogen.

The term "haloalkyl" means an alkyl having one or more halo substituents

The term "perfluoromethyl" means a methyl group substituted by one, two, or three fluoro atoms, *i.e.,* CH₂F, CHF₂ and CF₃. The term "perfluoroalkyl" means an alkyl group having from one to five fluoro atoms, such as pentafluoroethyl.

The term "haloalkoxy" means an alkoxy group having one or more halo substituents. For example, "haloalkoxy" includes -OCF₃.

The term "carbocyclic" means a saturated or unsaturated unsaturated monocyclic or bicyclic ring in which all atoms of all rings are carbon. Thus, the term includes cycloalkyl and aryl rings. The carbocyclic ring may be substituted in which case the substituents are selected from those recited above for cycloalkyl and aryl groups.

When the term "unsaturated" is used herein to refer to a ring or group, the ring or group may be fully unsaturated or partially unsaturated.

Definitions for the various other groups that are recited above in connection with substituted alkyl, substituted alkenyl, substituted alkynyl, substituted aryl, substituted heterocycle, substituted cycloalkyl, and so forth, are as follows: alkoxy is -OR^{a}, alkanoyl is -C(=O)R^{a}, aryloxy is -OAr, alkanoyloxy is -OC(=O)R^{a}, amino is -NH₂, alkylamino is -NHR^{a}, arylamino is -NHAr, aralkylamino is -NH-R^{b}-Ar, disubstituted amine or dialkylamino is NR^{c}R^{d}, alkanoylamino is -NH-C(=O)R^{a}, aroylamino is NH-C(=O)Ar, aralkanoylamino is -NH-C(-O)R^{b}-Ar, thiol is -SH, alkylthio is -SR^{a}, arylthio is -SAr, aralkylthio is -S-R^{b}-Ar, alkylthiono is -S(-O)R^{a}, arylthiono is -S(=O)Ar, aralkylthiono is -S(=O)R^{b}-Ar, alkylsulfonyl is -SO_{(q)}R^{a}, arylsulfonyl is -SO_{(q)}Ar, arylsulfonylamine is -NHSO_{(q)}Ar, alkylsulfonylamine is -NHSO₂R^{a}, aralkylsulfonyl is -SO_{(q)}R^{b}Ar, sulfonamido is -SO₂NH₂, nitro is -NO₂, carboxy is -CO₂H, carbamyl is -CONH₂, substituted carbamyl is -C(=O)NHR^{c}= or -C(=O)NR^{c}R^{d}, alkoxycarbonyl is -C(=O)OR^{a}, carboxyalkyl is -R^{b}-CO₂H, , sulfonic acid is -SO₃H, arylsulfonylamine is -NHSO_{(q)}Ar, guanidino is 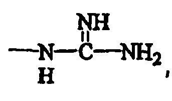 and ureido is 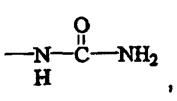 wherein R^{a} is alkyl as defined above, R^{b} is alkylene as defined above, R^{c} and R^{d} are selected from alkyl, aryl, and aralkyl, Ar is an aryl as defined above, and q is 2 or 3.

Throughout the specification, groups and substituents thereof may be chosen by one skilled in the field to provide stable moieties and compounds.

The compounds of Formula (I) may form salts which are also within the scope of this invention. Pharmaceutically acceptable (*i.e*. non-toxic, physiologically acceptable) salts are preferred, although other salts are also useful, e.g., in isolating or purifying the compounds of this invention. All references to compounds of Formula (I) herein are intended to include without limitation compounds of Formulae (Ia) to (Ii) as well as compounds of Formula (II) and (IIa)-(IIh). All references to compounds of Formula (II) are intended to include compounds of Formulae (IIa) to (IIh).

The compounds of Formula (I) may form salts with alkali metals such as sodium, potassium and lithium, with alkaline earth metals such as calcium and magnesium, with organic bases such as dicyclohexylamine, tributylamine, pyridine and amino acids such as arginine, lysine and the like. Such salts can be formed as known to those skilled in the art.

The compounds for Formula (I) may form salts with a variety of organic and inorganic acids. Such salts include those formed with hydrogen chloride, hydrogen bromide, methanesulfonic acid, sulfuric acid, acetic acid, trifluoroacetic acid, oxalic acid, maleic acid, benzenesulfonic acid, toluenesulfonic acid and various others (e.g., nitrates, phosphates, borates, tartrates, citrates, succinates, benzoates, ascorbates, salicylates and the like). Such salts can be formed as known to those skilled in the art.

Salt forms of the compounds may be advantageous for improving the compound dissolution rate and oral bioavailability. For select compounds of Formula (1), mesylate and/or bisulfate salts were successfully obtained *(see, e*.*g*., Example 125 herein). Both mesylate and bisulfate salts were found to be non-hygroscopic, highly water soluble, and stable in solid state

In addition, zwitterions ("inner salts") may be formed.

All stereoisomers of the compounds of the instant invention are contemplated, either in admixture or in pure or substantially pure form. The definition of compounds according to the invention embraces all the possible stereoisomers and their mixtures. It embraces the racemic forms and the isolated optical isomers having the specified activity. The racemic forms can be resolved by physical methods, such as, for example, fractional crystallization, separation or crystalliztion of diastereomeric derivatives or separation by chiral column chromatography. The individual optical isomers can be obtained from the racemates from the conventional methods, such as, for example, salt formation with an optically active acid followed by crystallizanon.

Compounds of the Formula (I) may also have prodrug forms. Any compound that will be converted in vivo to provide the bioactive agent (*i.e*., the compound for formula I) is a prodrug within the scope and spirit of the invention.

Various forms of prodrugs are well known in the art. For examples of such prodrug derivatives, see:
a) **Design of Prodrugs,** edited by H. Bundgaard, (Elsevier,1985) and **Methods in Enzymology,** Vol.42, p. 309-396, edited by K. Widder, et al. (Acamedic Press, 1985);
**b) A Textbook of Drug Design and Development, edited by Krosgaard-**Larsen and H. Bundgaard, Chapter 5, "Design and Application of Prodrugs," by H. Bundgaard, p. 113-191 (1991); and
c) H. Bundgaard, **Advanced Drug Delivery Reviews, 8, 1-38** (1992), each of which is incorporated herein by reference.

It should further be understood that solvates (e.g., hydrates) of the compounds of Formula (I) are also with the scope of the present invention. Methods of solvation are generally known in the art.

Preferred embodiments of the invention comprise preferred compounds of Formulae (I) and (II), and their use for treating conditions associated with p38 kinase activity. Preferred compounds are those having Formula (I), 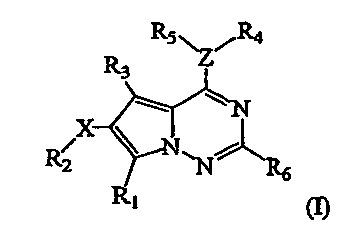 and pharmaceutically acceptable salts, prodrugs, or solvates thereof, wherein:
- R₃: is methyl, -CF₃, or -OCF₃,
- X: is selected from -C(=O)-, -CO₂-, NR₁₀C(=O)-, and -C(=O)NR₁₀-, or X is absent;
- Z: is N;
- R₁: is hydrogen, -CH₃, -OH, -OCH₃, -SH, -SCH₃, -OC(=O)R₂₁, -S(=O)R₂₂, -SO₂R₂₂, -SO₂NR₂₄R₂₅, -CO₂R₂₁, -C(=O)NR₂₄R₂₅, -NH₂, -NR₂₁SO₂NR₂₄R₂₅, -NR₂₁SO₂R₂₂, -NR₂₄C(=O)R₂₅, -NR₂₄CO₂R₂₅, -NR₂₁C(=O)NR₂₄R₂₅, halogen, nitro, or cyano;
- R₂: is hydrogen, C₂₋₆alkyl, substituted C₁₋₄alkyl, aryl, aralkyl, substituted aryl, substituted aralkyl, cycloalkyl, substituted cycloalkyl, heterocycle, or substituted heterocycle, or optionally-substituted cycloalkylalkyl or heterocycloalkyl;
- R₄: is aryl or heteroaryl substituted with one R₁₂ and zero to three R₁₃;
- R₃ and R₁₀: independently are selected from hydrogen and lower alkyl;
- R₆: is hydrogen, alkyl, substituted alkyl, aryl, substituted aryl, heterocyclo, substituted heterocyclo, -NR₇R₈, -OR₇, or halogen;
- R₁₂: is carbamyl, sulfonamido, arylsulfonylamine, or ureido, each of which is optionally substituted with up to two of hydroxy, alkyl, substituted alkyl, alkoxy, aryl, substituted aryl, and aralkyl, or R₁₂ is alkylsulfonylamine;
- R₁₃: at each occurrence is independently selected from alkyl, substituted alkyl, halo, trifluoromethoxy, trifluommethyl, -OR₁₄, -C(=O)alkyl, -OC(-O)alkyl, -NR₁₅R₁₆, -SR₁₅, -NO₂, -CN, -CO₂R₁₅, -CONH₂, -SO₃H, -S(=O)alkyl, -S(=O)aryl, -NHSO₂-aryl-R₁₇, NHSO₂-alkyl, -SO₂NHR₁₇, -CONHR₁₇, and NHC(-O)NHR₁₇;
- R₁₄: is hydrogen, alkyl, or aryl;
- R₁₅: is hydrogen or alkyl;
- R₁₆: is hydrogen, alkyl, aralkyl, or alkanoyl;
- R₁₇: is hydrogen, hydroxy, alkyl, substituted alkyl, alkoxy, aryl, substituted aryl, or aralkyl;
- R₇, R₈, R₁₀, R₁₁, R₂₁, R₂₄, and R₂₅: are independently selected from hydrogen and alkyl; and R₂₂ is alkyl or substituted alkyl.

In compounds of Formula (I), preferably the group R₃ is methyl, trifluoromethyl, or methoxy, most preferably methyl; X is preferably -CO₂-, NR₁₀C(=O)-, or -C(=O)NR₁₀-, more preferably -C(=O)NH-; Z is preferably N; R₄ is preferably substituted aryl or substituted heteroaryl, more preferably phenyl substituted with at least one of carbamyl, substituted carbamyl, arylsulfonylamido, substituted arylsulfonylamido, ureido, or substituted ureido, and optionally substituted with one or two C₁₋₄alkyl or halogen. Most preferably R₄ is phenyl substituted with at least one of -C(=O)NHO(C₁₋₄alkyl) or -C(=O)NH(optionally substituted phenyl), and also is optionally substituted with C₁₋₄alkyl. R₅ is preferably hydrogen or lower alkyl, more preferably hydrogen.

In preferred compounds, R₁ and R₆ may be selected from groups of substituents as defined herein; however, advantageously they are selected from hydrogen, CH₃, -OH, -OCH₃, halogen, nitro, and cyano, and most preferably R₁ and R₆ are hydrogen. R₂ preferably is alkyl aryl, substituted aryl, aralkyl, substituted aralkyl, heteroaryl, or substituted heteroaryl, more preferably straight or branched C₂. ₆alkyl or optionally-substituted benzyl. The mesylate salt is the preferred form of salt.

Accordingly, preferred compounds further comprise those having the Formula (II), 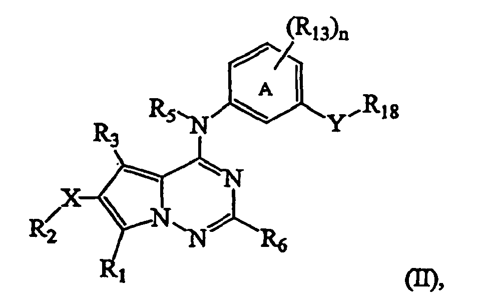 and pharmaceutically acceptable salts, prodrugs, or solvates thereof, wherein:
- R₃: is methyl, -CF₃, or -OCH₃;
- X: is -C(=O)NR₁₀-, -NR₁₀C(=O)-, -C(=O)-, or -CO₂-;
- Y: is -C(=O)NH-, -NHC(=O)NH-, or -NHSO₂-;
- R₁₀: is hydrogen or lower alkyl;
- R₁₈: is selected from hydrogen, alkyl, alkoxy, aryl, and aryl substituted with one to three R₁₉, except that when Y is -NHSO₂-, R₁₈ is -C₁₄alkyl, aryl or aryl substituted with R₁₉;
- R₁₃: is attached to any available carbon atom of phenyl ring A and at each occurrence is independently selected from alkyl, substituted alkyl, halo, trifluoromethoxy, trifluoromethyl, -OR₁₄, -C(=O)alkyl, -OC(=O)alkyl, -NR₁₅R₁₆, -SR₁₅, -NO₂, -CN, -CO₂R₁₅, -CONH₂, -SO₃H, -S(=O)alkyl, -S(=O)aryl, NHSO₂-aryl- R₁₇, -SO₂NHR₁₇, -CONHR₁₇, and NHC(=O)NHR₁₇;
- R₁₄, R₁₅, R₁₆ and R₁₇: are hydrogen or alkyl;
- R₁₉: at each occurrence is selected from alkyl, halo, trifluoromethoxy, trifluoromethyl, hydroxy, alkoxy, alkanoyl, alkanoyloxy, thiol, alkylthio, ureido, nitro, cyano, carboxy, carboxyalkyl, carbamyl, alkoxycarbonyl, alkylthiono, arylthiono, arylsulfonylamine, sulfonic acid, alkysulfonyl, sulfonamido, and aryloxy, wherein each group R₁₉ may be further substituted by hydroxy, alkyl, alkoxy, aryl, or aralkyl;
- *n*: is 0, 1 or 2, and
- R₁, R₂ and R₆: are as defined above for compounds of Formula (I).

More preferred are compounds having the Formula (IIa) or (IIb): 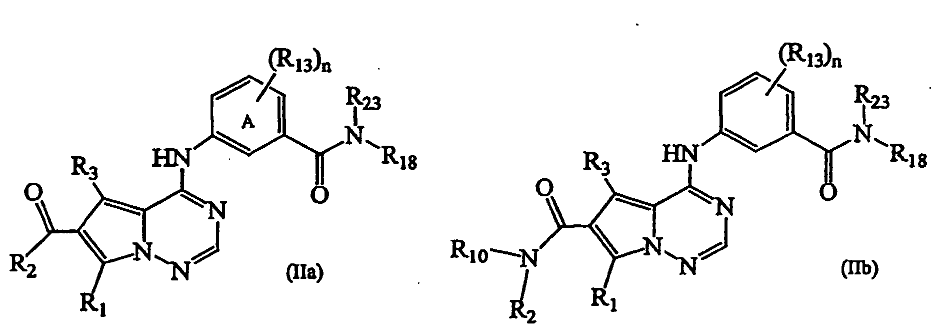 and pharmaceutically acceptable salts, prodrugs, or solvates thereof, wherein:
- R₃: is methyl;
- R₁ and R₁₀: are hydrogen or -CH₃;
- R₂: is selected from hydrogen; straight or branched C₂₋₆alkyl; cycloalkyl optionally substituted with keto and/or up to two R₂₇; phenyl optionally substituted with up to two R₂₇; heterocycle optionally substituted with keto and/or up to two R₂₇; and C₁₋₄alkyl substituted with up to three of halogen, trifluoromethyl, cyano, OR₂₈, NR₂₈R₂₉, CO₂R₂₈, aryl, heterocycle, and/or cycloallryl, wherein the aryl, heterocycle, and/or cycloalkyl in turn are optionally substituted with up to two of halogen, hydroxy, alkoxy, haloalkyl, haloalkoxy, nitro, cyano and alkyl;
- R₁₈: is hydroxy, C₁₋₄alkoxy, phenyl, or phenyl substituted with one or two R₁₉;
- R₁₃ and R₁₉: are selected from lower alkyl, halogen, trifluoromethoxy, trifluoromethyl, hydroxy, C₁₋₄alkoxy, nitro, and cyano;
- R₂₇: at each occurrence is independently selected from hydrogen, alkyl, trifluoromethyl, trifluoromethoxy, halogen, cyano, nitro, amino, hydroxy, alkoxy, phenyl, benzyl, phenyloxy, and benzyloxy;
- R₂₈ and R₂₉: at each occurrence are independently selected from hydrogen, alkyl, alkenyl, phenyl, and benzyl; and
- *n*: is 0,1 or 2.

When R₂ is a heterocyclo, advantageously it is selected from diazepinyl, morpholinyl, piperidinyl, and pyrrolidinyl, said heterocycle being optionally substituted with C₁₋₄alkyl, phenyl, and/or benzyl.

Most preferred are compounds having the formula, 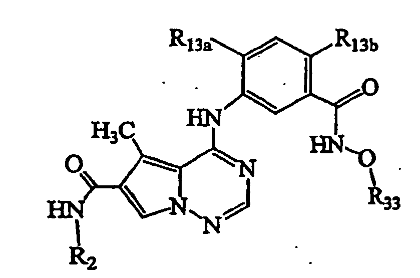 in which R₁₃ₐ and R_{13b} are hydrogen, CH₃, OR, OCH₃, CF₃, cyano, or halogen, R₂ is C₂₋₆alkyl or optionally substituted benzyl, R₃₃ is lower alkyl, and n is 0 or 1.

The compounds of the invention are selective inhibitors of p38 kinase activity, and in particular, isoforms p38α and p38β. Accordingly, compounds of formula (I) have utility in treating conditions associated with p38 kinase activity. Such conditions include diseases in which cytokine levels are modulated as a consequence of intracellular signaling via p38, and in particular, diseases that are associated with an overproduction of cytokines IL-1, IL-4, IL-8, and TNF-α. As used herein, the terms "treating" or "treatment" encompass either or both responsive and prophylaxis measures, *e.g*., designed to inhibit or delay the onset of the disease or disorder, achieve a full or partial reduction of the symptoms or disease state, and/or to alleviate, ameliorate, lessen, or cure the disease or disorder and/or its symptoms. When reference is made herein to inhibition of "p-38α/β kinase," this means that either p38α and/or p38β kinase are inhibited. Thus, reference to an IC₅₀ value for inhibiting p-38α/β kinase means that the compound has such effectiveness for inhibiting at least one of, or both of, p38α and p38β kinases.

In view of their activity as inhibitors of p-38α/β kinase, compounds of Formula (I) are useful in treating p-38 associated conditions including, but not limited to, inflammatory diseases, autoimmune diseases, destructive bone disorders, proliferative disorders, angiogenic disorders, infectious diseases, neurodegenerative diseases, and viral diseases.

More particularly, the specific conditions or diseases that may be treated with the inventive compounds include, without limitation, pancreatitis (acute or chronic), asthma, allergies, adult respiratory distress syndrome, chronic obstructive pulmonary disease, glomerulonephritis, rheumatoid arthritis, systemic lupus erythematosis, scleroderma, chronic thyroiditis, Grave's disease, autoimmune gastritis, diabetes, autoimmune hemolytic anemia, autoimmune neutropenia, thrombocytopenia, atopic dermatitis, chronic active hepatitis, myasthenia gravis, multiple sclerosis, inflammatory bowel disease, ulcerative colitis, Crohn's disease, psoriasis, graft vs. host disease, inflammatory reaction induced by endotoxin, tuberculosis, atherosclerosis, muscle degeneration, cachexia, psoriatic arthritis, Reiter's syndrome, gout, traumatic arthritis, rubella arthritis, acute synovitis, pancreatic β-cell disease; diseases characterized by massive neutrophil infiltration; rheumatoid spondylitis, gouty arthritis and other arthritic conditions, cerebral malaria, chronic pulmonary inflammatory disease, silicosis, pulmonary sarcoisosis, bone resorption disease, allograft rejections, fever and myalgias due to infection, cachexia secondary to infection, meloid formation, scar tissue formation, ulcerative colitis, pyresis, influenza, osteoporosis, osteoarthritis and multiple myeloma-related bone disorder, acute myelogenous leukemia, chronic myelogenous leukemia, metastatic melanoma, Kaposi's sarcoma, multiple myeloma, sepsis, septic shock, and Shigellosis; ALzheimer's disease, Parkinson's disease, cerebral ischemias or neurodegenerative disease caused by traumatic injury; angiogenic disorders including solid tumors, ocular neovasculization, and infantile haemangiomas; viral diseases including acute hepatitis infection (including hepatitis A, hepatitis B and hepatitis C), HIV infection and CMV retinitis, AIDS< ARC or malignancy, and herpes; stroke, myocardial ischemia, ischemia in stroke heart attacks, organ hyposia, vascular hyperplasia, cardiac and renal reperfusion injury, thrombosis, cardiac hypertrophy, thrombin-induced platelet aggregation, endotoxemia and/or toxic shock syndrome, and conditions associated with prostaglandin endoperoxidase syndase-2.

In addition, p38 inhibitors of this invention inhibit the expression of inducible pro-inflammatory proteins such as prostaglandin endoperoxide synthase-2 (PGHS-2), also referred to as cyclooxygenase-2 (COX-2). Accordingly, additional p38-associated conditions include edema, analgesia, fever and pain, such as neuromuscular pain, headache, pain caused by cancer, dental pain and arthritis pain. The inventive compounds also may be used to treat veterinary viral infections, such as lentivirus infections, including, but not limited to equine infectious anemia virus; or retro virus infections, including feline immunodeficiency virus, bovine immunodeficiency virus, and canine immunodeficiency virus.

When the terms "p38 associated condition" or "p38 associated disease or disorder" are used herein, each is intended to encompass all of the conditions identified above as if repeated at length, as well as any other condition that is affected by p38 kinase activity.

The present invention thus refers to the use of at least one compound of Formula (I) or a salt thereof for treating such conditions. The treatment of p38 kinase-associated conditions may comprise administering compounds of Formula (I) alone or in combination with each other and/or other suitable therapeutic agents useful in treating such conditions. Exemplary of such other therapeutic agents include corticosteroids, rolipram, calphostin, CSAIDs, 4-substituted imidazo [1,2-A]quinoxalines as disclosed in US Pat. No. 4,200,750 and in S. Ceccarelli *et al, "Imidazo[1,2-α]quinoxalin-4-amines: A Novel Class of Nonxanthine A*_{*1*}*-Adenosine Receptor Antagonists,"* European Iournal of Medicinal Chemistry VoL 33, (1998), at pp. 943-955; Interleukin-10, glucocorticoids, salicylates, nitric oxide, and other immunosuppressants; nuclear translocation inhibitors, such as deoxyspergualin (DSG); non-steroidal antiinflammatory drugs (NSAIDS) such as ibuprofen, celecoxib and rofecoxib; steroids such as prednisone or dexamethasone; antiviral agents such as abacavir; antiproliferative agents such as methotrexate, leflunomide, FK506 (tacrolimus, Prograf); cytotoxic drugs such as azathiprine and cyclophosphamide; TNF-α inhibitors such as tenidap, anti-TNF antibodies or soluble TNF receptor, and rapamycin (sirolimus or Rapamune) or derivatives thereof.

The above other therapeutic agents, when employed in combination with the compounds of the present invention, may be used, for example, in those amounts indicated in the Physicians' Desk Reference (PDR) or as otherwise determined by one of ordinary skill in the art. In the methods of the present invention, such other therapeutic agent(s) may be administered prior to, simultaneously with, or following the administration of the inventive compounds.

The present invention also provides pharmaceutical compositions capable of treating p38-kinase associated conditions, including TNF-α, IL-1, and/or IL-8 mediated conditions, as described above. The inventive compositions may contain other therapeutic agents as described above and may be formulated, for example, by employing conventional solid or liquid vehicles or diluents, as well as pharmaceutical additives of a type appropriate to the mode of desired administration (e.g., excipients, binders, preservatives, stabilizers, flavors, etc.) according to techniques such as those well known in the art of pharmaceutical formulation.

The compounds of Formula (I) may be administered by any means suitable for the condition to be treated, which may depend on the need for site-specific treatment or quantity of drug to be delivered. Topical administration is generally preferred for skin-related diseases, and systematic treatment preferred for cancerous or pre-cancerous conditions, although other modes of delivery are contemplated. For example, the compounds may be delivered orally, such as in the form of tablets, capsules, granules, powders, or liquid formulations including syrups; topically, such as in the form of solutions, suspensions, gels or ointments; sublingually; bucally; parenterally, such as by subcutaneous, intravenous, intramuscular or intrasternal injection or infusion techniques (*e.g*., as sterile injectable aq. or non-aq. solutions or suspensions); nasally such as by inhalation spray; topically, such as in the form of a cream or ointment; rectally such as in the form of suppositories; or liposomally. Dosage unit formulations containing non-toxic, pharmaceutically acceptable vehicles or diluents may be administered. The compounds may be administered in a form suitable for immediate release or extended release. Immediate release or extended release may be achieved with suitable pharmaceutical compositions or, particularly in the case of extended release, with devices such as subcutaneous implants or osmotic pumps.

Exemplary compositions for topical administration include a topical carrier such as PLASTIBASE® (mineral oil gelled with polyethylene).

Exemplary compositions for oral administration include suspensions which may contain, for example, microcrystalline cellulose for imparting bulk, alginic acid or sodium alginate as a suspending agent, methylcellulose as a viscosity enhancer, and sweeteners or flavoring agents such as those known in the art; and immediate release tablets which may contain, for example, microcrystalline cellulose, dicalcium phosphate, starch, magnesium stearate and/or lactose and/or other excipients, binders, extenders, disintegrants, diluents and lubricants such as those known in the art. The inventive compounds may also be orally delivered by sublingual and/or buccal administration, e.g., with molded, compressed, or freeze-dried tablets. Exemplary compositions may include fast-dissolving diluents such as mannitol, lactose, sucrose, and/or cyclodextrins. Also included in such formulations may be high molecular weight excipients such as celluloses (AVICEL®) or polyethylene glycols (PEG); an excipient to aid mucosal adhesion such as hydroxypropyl cellulose (HPC), hydroxypropyl methyl cellulose (HPMC), sodium carboxymethyl cellulose (SCMC), and/or maleic anhydride copolymer (e.g., GANTREZ®); and agents to control release such as polyacrylic copolymer (e.g., CARBOPOL 934®). Lubricants, glidants, flavors, coloring agents and stabilizers may also be added for ease of fabrication and use.

Exemplary compositions for nasal aerosol or inhalation administration include solutions which may contain, for example, benzyl alcohol or other suitable preservatives, absorption promoters to enhance absorption and/or bioavailability, and/or other solubilizing or dispersing agents such as those known in the art.

Exemplary compositions for parenteral administration include injectable solutions or suspensions which may contain, for example, suitable non-toxic, parenterally acceptable diluents or solvents, such as mannitol, 1,3-butanediol, water, Ringer's solution, an isotonic sodium chloride solution, or other suitable dispersing or wetting and suspending agents, including synthetic mono- or diglycerides, and fatty acids, including oleic acid.

Exemplary compositions for rectal administration include suppositories which may contain, for example, suitable non-irritating excipients, such as cocoa butter, synthetic glyceride esters or polyethylene glycols, which are solid at ordinary temperatures but liquefy and/or dissolve in the rectal cavity to release the drug.

The effective amount of a compound of the present invention may be determined by one of ordinary skill in the art, and includes exemplary dosage amounts for a mammal of from about 0.05 to 100 mg/kg of body weight of active compound per day, which may be administered in a single dose or in the form of individual divided doses, such as from 1 to 4 times per day. It will be understood that the specific dose level and frequency of dosage for any particular subject may be varied and will depend upon a variety of factors, including the activity of the specific compound employed, the metabolic stability and length of action of that compound, the species, age, body weight, general health, sex and diet of the subject, the mode and time of administration, rate of excretion, drug combination, and severity of the particular condition. Preferred subjects for treatment include animals, most preferably mammalian species such as humans, and domestic animals such as dogs, cats, horses, and the like. Thus, when the term "patient" is used herein, this term is intended to include all subjects, most preferably mammalian species, that are affected by mediation of p38 enzyme levels.

Compounds of formula (I), including the compounds described in the examples hereof, have been tested in one or more of the assays described below and have shown activity as inhibitors of p38α/β enzymes and TNF-α.

### Biological Assays

### Generation of p38 Kinases

cDNAs of human p38α,β and γ isozymes were cloned by PCR. These cDNAs were subcloned in the pGEX expression vector (Pharmacia). GST-p38 fusion protein was expressed in E. Coli and purified from bacterial pellets by affinity chromatography using glutathione agarose. p38 fusion protein was activated by incubating with constitutively active MKK6. Active p38 was separated from MKK6 by affinity chromatography. Constitutively active MKK6 was generated according to Raingeaud et al. [*Mol*. Cell. *Biol.,* 1247-1255 (1996)].

### TNF-α Production by LPS-Stimulated PBMCs

Heparinized human whole blood was obtained from healthy volunteers. Peripheral blood mononuclear cells (PBMCs) were purified from human whole blood by Ficoll-Hypaque density gradient centrifugation and resuspended at a concentration of 5 x 10⁶/ml in assay medium (RPMI medium containing 10% fetal bovine serum). 50 ul of cell suspension was incubated with 50 ul of test compound (4X concentration in assay medium containing 0.2% DMSO) in 96-well tissue culture plates for 5 minutes at RT. 100 ul of LPS (200 ng/ml stock) was then added to the cell suspension and the plate was incubated for 6 hours at 37°C. Following incubation, the culture medium was collected and stored at 20°C. TNF-α concentration in the medium was quantified using a standard ELISA kit (Pharmingen-San Diego, CA). Concentrations of TNF-α and IC₅₀ values for test compounds (concentration of compound that inhibited LPS-stimulated TNF-α production by 50%) were calculated by linear regression analysis.

### p38 Assay

The assays were performed in V-bottomed 96-well plates. The final assay volume was 60 *µ*l prepared from three 20 *µ*l additions of enzyme, substrates (MBP and ATP) and test compounds in assay buffer (50 mM Tris pH 7.5, 10 mM MgCl₂, 50 mM NaCl and 1 mM DTT). Bacterially expressed, activated p38 was pre-incubated with test compounds for 10 min. prior to initiation of reaction with substrates. The reaction was incubated at 25°C for 45 min. and terminated by adding 5 *µ*l of 0.5 M EDTA to each sample. The reaction mixture was aspirated onto a pre-wet filtermat using a Skatron Micro96 Cell Harvester (Skatron, Inc.), then washed with PBS. The filtermat was then dried in a microwave oven for 1 min., treated with MeltilLex A scintillation wax (Wallac), and counted on a Microbeta scintillation counter Model 1450 (Wallac). Inhibition data were analyzed by nonlinear least-squares regression using Prizm (GraphPadSoftware). The final concentration of reagents in the assays are ATP, 1 *µ*M; [γ-³³P]ATP, 3 nM,; MBP (Sigma, #M1891), 2µg/well*;* p38, 10 nM; and DMSO, 0.3%.

### TNF-α Production by LPS-Stimulated Mice

Mice (Balb/c female, 6-8 weeks of age, Harlan Labs; n=8/treatment group) were injected intraperitoneally with 50ug/kg lipopolysaccharide (LPS; *E coli* strain 0111:B4, Sigma) suspended in sterile saline. Ninety minutes later, mice were sedated by CO₂:O₂ inhalation and a blood sample was obtained. Serum was separated and analyzed for TNF-alpha concentrations by commercial ELISA assay per the manufacturer's instructions (R&D Systems, Minneapolis, MN).

Test compounds were administered orally at various times before LPS injection. The compounds were dosed either as suspensions or as solutions in various vehicles or solubilizing agents.

### Abbreviations

For ease of reference, the following abbreviations are employed herein, including the methods of preparation and Examples that follow:
Ph = phenyl
Bz = benzyl
t-Bu = tertiary butyl
Me = methyl
Et = ethyl
Pr = propyl
Iso-P = isopropyl
MeOH = methanol
EtOH = ethanol
EtOAc = ethyl acetate
Boc = tert-butyloxycarbonyl
CBZ = carbobenzyloxy or carbobenzoxy or benzyloxycarbonyl
DCM = dichloromethane
DCE =1,2-dichloroethane
DMF = dimethyl formamide
DMSO = dimethyl sulfoxide
TFA = trifluoroacetic acid
THF = tetrahydrofuran
HATU = *O*-(7-Azabenzotriazol-1-yl-*N,N,N;N'*-tetramethyluronim
hexafluorophosphate
KOH = potassium hydroxide
K₂CO₃ = potassium carbonate
POCl₃ =phosphorous oxychloride
KOtBu = potassium t-butoxide
EDC or EDCI = 1-(3-Dimethylaminopropyl)-3-ethylcarbodiimide hydrochloride
DIPEA = diisopropylethylamine
HOBt= 1-hydroxybenzotriazole hydrate
m-CPBA = m-chloroperbenzoic acid
NaH = sodium hydride
NaOH = sodium hydroxide
Na₂S₂O₃ = sodium thiosulfate
Pd = palladium
Pd/C = palladium on carbon
min = minute(s)
L = liter
mL = milliliter
µL = microliter
g = gram(s)
mg = milligram(s)
mol = moles
mmol = millimole(s)
meq = milliequivalent
RT or rt = room temperature
ret. t. = HPLC retention time (minutes)
sat or sat'd = saturated
aq. = aqueous
TLC = thin layer chromatography
HPLC = high performance liquid chromatography
RP HPLC = reverse phase HPLC
LC/MS = high performance liquid chromatography/mass spectrometry
MS = mass spectrometry
NMR = nuclear magnetic resonance
mp = melting point

In the Examples, "HPLC Condition A" refers to YMC S5 ODS 4.6 x 50 mm Ballistic column, 4 mL/min flow rate, 4 min linear gradient elution (Start solvent %B = 0; Final solvent %B = 100), solvent A =10% MeOH / 90% H₂O / 0.2% H₃PO₄.

### Methods of Preparation

Compounds of formula I may generally be prepared according to the following schemes and the knowledge of one skilled in the art. In the schemes, the groups R,-R₆, R₁₀, R₁₃, R₁₈, R₂₃, X and Z are as described herein for compounds of Formula (I).

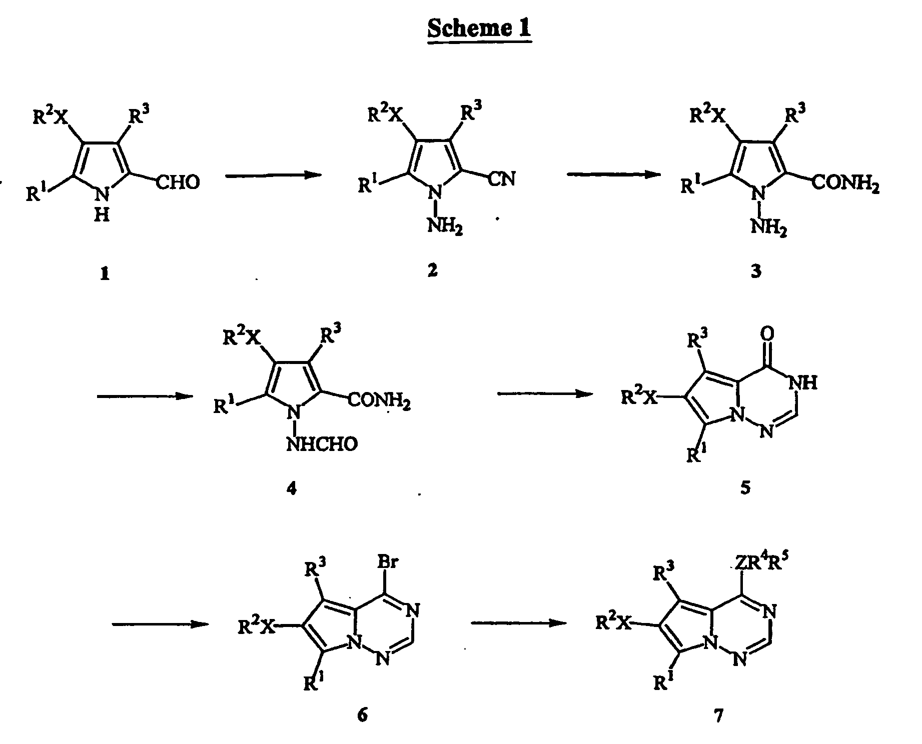

An optionally substituted 2-formylpyrrole (1) is reacted with an aminating reagent, such as hydroxylamine-O-sulfonic acid, in an aq. solvent at rt, followed by treatment under cooling with a base such as KOH, to form compound (2).

Compound (2) is reacted with an aq. base such as KOH at rt to form compound (3). Compound (3) is reacted with an acylating agent, such as formic acid, in an aq. solvent, to form compound (4). Compound (4) is cyclized with a base such as sodium methoxide in MeOH with heating to form compound (5). Compound (5) is halogenated, *e.g*., with phosphorus oxybromide at elevated temperature, to form compound (6). Compound (6) is reacted with an amine such as an aniline in an organic solvent, such as acetonitrile, to form product (7) of Scheme 1.

Compound (7) of Scheme 1 where R₁ = halogen can be prepared from compound (7) of Scheme 1 where R₁ = hydrogen by reaction with a halogenating agent such as bromine in a suitable solvent such as acetic acid.

Compounds (1) may be obtained from substituted pyrroles by formylation, e.g., by reaction with phosphorus oxychloride and DMF. A methylpyrrole may be obtained by reduction of a formylpyrrole, *e.g.*, by reaction with lithium aluminum hydride.

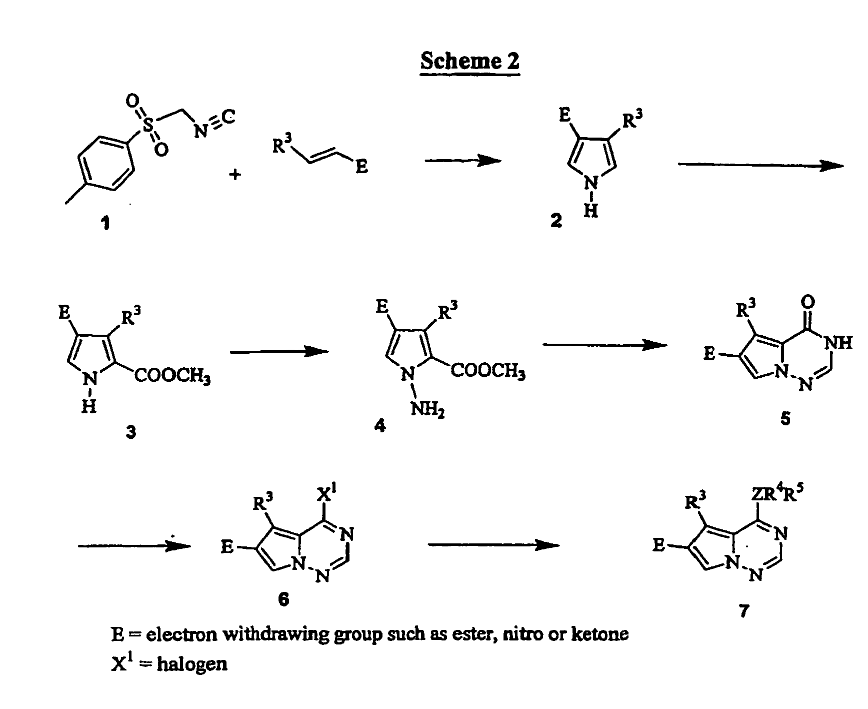

Reacting an anion of tosylmethyl isocyanide (TosMIC) (1) with a Michael acceptor such as ethyl crotonate provides disubstituted pyrrole (2). Treatment of pyrrole (2) with an acylating agent such as trichloroacetyl chloride in the presence of a Lewis acid such as aluminum chloride at from rt to 50 °C, followed by treatment with sodium methoxide, affords trisubstituted pyrrole (3). Compound (3) can be obtained by warming an aldehyde, such as acetaldehyde, with 2 equivalents of ethyl isocyanoacetate in the presence of a base, such as DBU, in an organic solvent, such as THF. Alternatively, compound (3) can be obtained following the procedure of M. Suzuki, M. Miyoshi, and K. Matsumoto J. Org. Chem. 1974, 39 (1980).

Pyrrole (3) can be aminated by an aminating reagent, such as diphenyl phosphoryl hydroxylamine, in the presence of a base, such as NaH, at rt in organic solvents, such as DMF, to form N-aminated pyrrole (4). Compound (4) is cyclized by heating at from 120 to 195 °C with formamide to afford 1,2,4-triazine (5). Treatment of compound (5) with a halogenating agent, such as phosphorous oxybromide, at from 60 to 115°C, in the presence or absence of a co-solvent such as DCE, affords compound (6).

Compound (6) is reacted with an amine, such as an aniline in an organic solvent, such as DMF, to obtain compound (7). Alternatively, compound (7) can be obtained by treating (6) with an anion of a heterocyclic compound, such as oxindole, in an organic solvent such as THF.

An anion of TosMIC (1) can be made by treating a solution of it in DMSO with a base such as NaH at rt or a solution of it in THF with lithium hexamethyldisilazane at -78 °C.

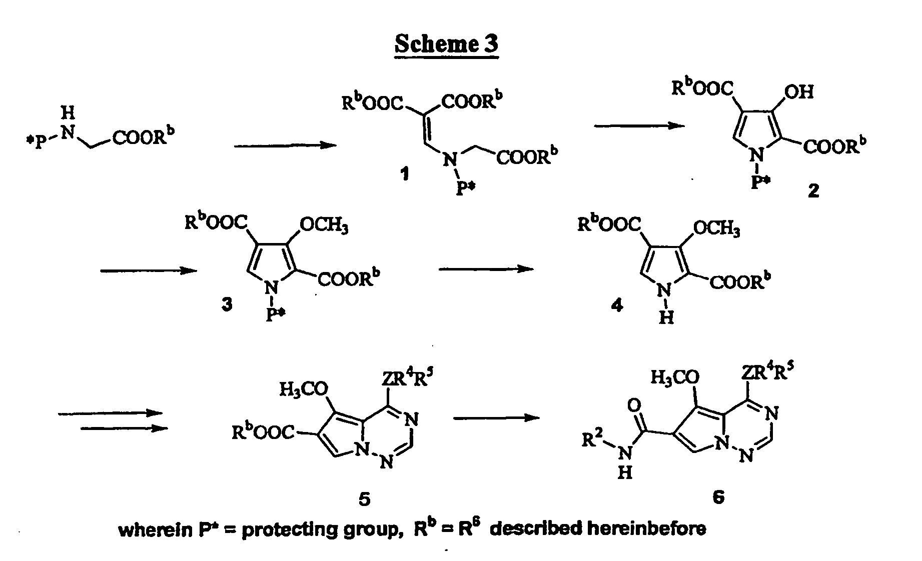

A suitably N-protected ester of glycine, such as with benzyl group, can be added to dialkyl methylene malonate at from rt to 80°C to obtain compound (1). Compound (1) is cyclized to form pyrrole (2) upon treatment with a strong base, such as lithium hexamethyldisilazane, at from -78°C to rt in an organic solvent such as THF. Pyrrole (2) is alkylated by treatment with an alkylating agent, such as iodomethane or dimethyl sulfate, in the presence of a base, such as K₂CO₃, in an organic solvent, such as acetone or DMF to yield compound (3).

Deprotection of compound (3) can be achieved, when optionally protected by groups such as benzyl, by hydrogenation over a catalyst, such as Pd, in the presence of ammonium formate. Compound (4) is converted to compound (5) via cyclization as described for compound (5) of Scheme 2.

Hydrolysis of the ester group in compound (5) can be achieved by treatment with a base such as aq. KOH. The resulting acid can be coupled with an amine in the presence of a coupling agent, such as DCC or PyBrop.

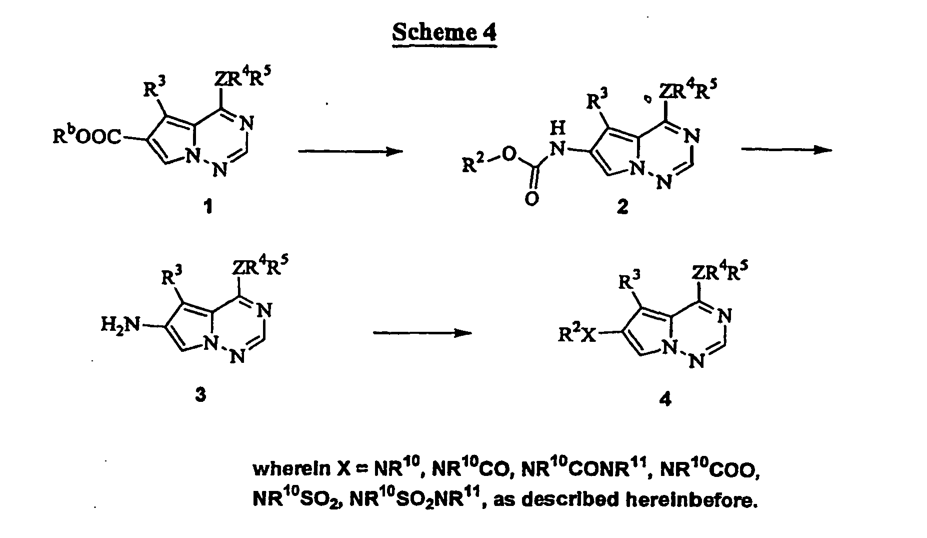

Compound (5) from Scheme 3 can be converted to carboxylic acid (1) (wherein R₃ is methoxy or is as otherwise defined herein) by treatment with a base such as aq. KOH. This acid undergoes Curtis rearrangement by treatment with diphenyl phosphoryl azide in the presence of an alcohol, such as benzyl alcohol, in an organic solvent, such as 1,4-dioxane, to afford compound (2).

The carbamate group of compound (2) can be deprotected, when optionally protected by groups such as CBZ, by hydrogenation over a catalyst, such as Pd, to obtain compound (3). The amino group of compound (3) can be acylated to form compound (4), e.g., by treatment with a carboxylic acid in the presence of a coupling agent such as DCC, or sulfonylated, e.g., by treatment with a sulfonyl chloride. Alternatively, the amino group of compound (3) may be alkylated with alkyl halides or may undergo reductive amination with aldehydes in the presence of a reducing agent, such as sodium cyanoborohydride.

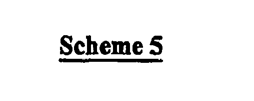

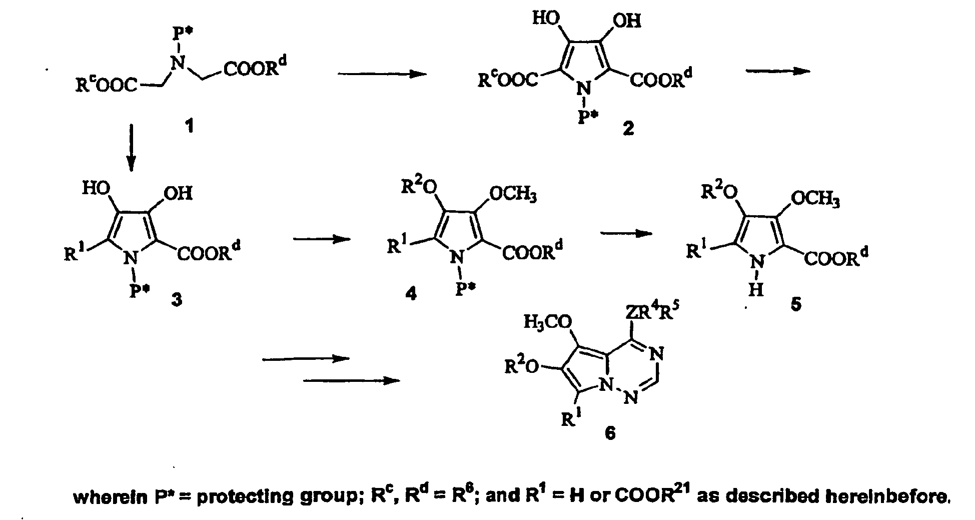

Suitably protected compound (1) (imino dicarboxylate) can be cyclized by treatment with dialkyl oxalate in the presence of a base, such as sodium methoxide, in an organic solvent, such as MeOH. Compound (2) upon selective deprotection, such as with TFA when optionally protected by tert-butyl ester, undergoes decarboxylation to afford compound (3) where R¹= H. This step is omitted to form compound (3) where R¹ = COOR²¹.

The hydroxy group of compound (3) can be etherified by reaction with an alkylating agent, such as dimethyl sulfate. Compound (4) can be deprotected by hydrogenation, when optionally protected such as with a benzyl group, to obtain compound (5). Compound (5) is then converted to compound (6) in an analogous manner to that described for compound (4) of Scheme 3 and compounds (4) through (7) of Scheme 2.

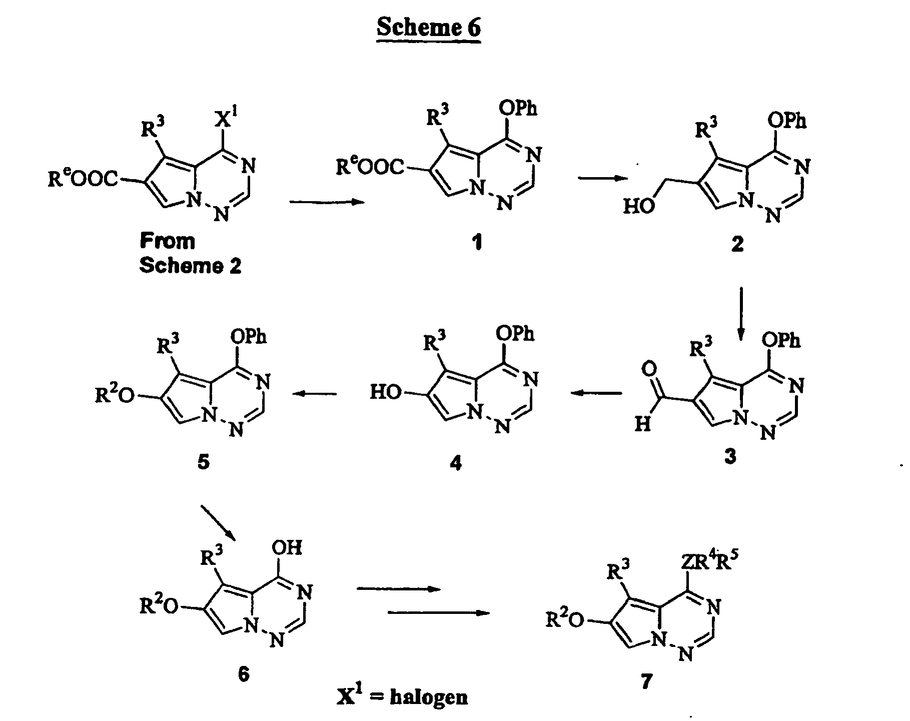

Compound (6) of Scheme 2 can be etherified at the 4-position, e.g., by treatment with phenoxide anion to form compound (1). Reduction of compound (1) with a reducing agent, such as DIBAL, in an organic solvent, such as toluene, affords alcohol (2). Oxidation of the alcohol (2) can be achieved by treatment with MnO₂ at an elevated temperature in an organic solvent, such as toluene, to form (3). Treatment of compound (3) with an oxidant, such as m-CPBA in an organic solvent, such as DCM, followed by aq. hydrolysis with a base, such as potassium bicarbonate, affords the hydroxy compound (4).

Alkylation of the phenolic group of compound (4) with an agent, such as iodomethane, in the presence of a base, such as NaH, at from rt to 100°C, affords compound (5). Hydrolysis of compound (5) can be achieved by treatment with an acid, such as aq. HCl, at an elevated temperature to afford (6). Compound (6) can be converted to compound (7) with procedures analogous to those described in Scheme 2.

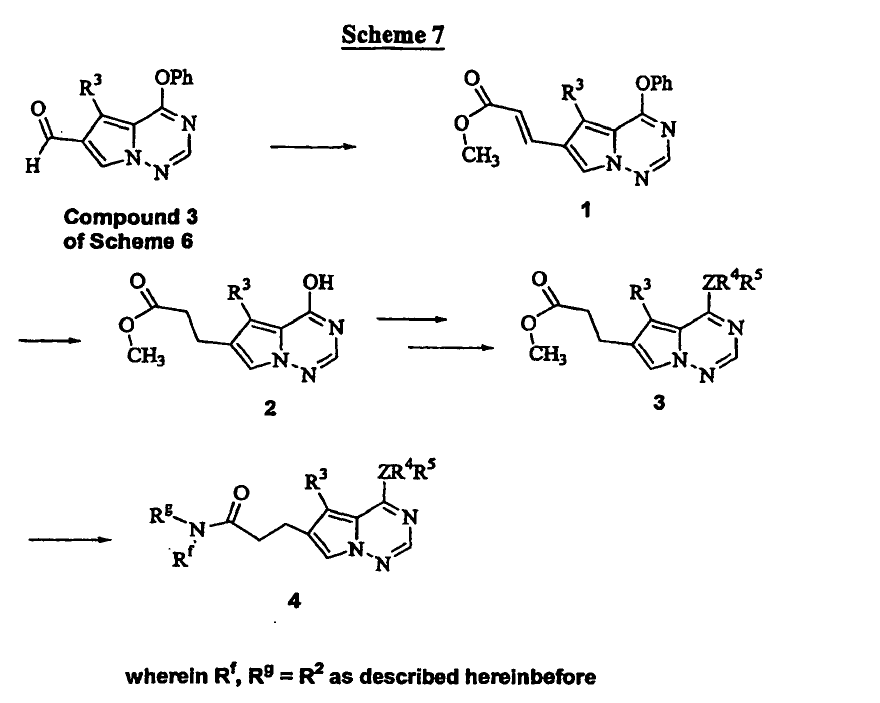

Compound (3) from Scheme 6 can undergo a Wittig reaction, *e.g.,* with phosphonates such as methyl diethylphosphonoacetate, in an organic solvent, such as DCE, in the presence of a base, such as NaH, to afford compound (1). The double bond of compound (1) can be hydrogenated by treatment with hydrogen in the presence of a catalyst, such as Pd. Compound (2) can be converted to (3) by procedures described in Scheme 2.

Hydrolysis of the ester, as described hereinbefore, followed by coupling of the resulting acid with an amine in the presence of a coupling agent, such as DCC, affords compound (4).

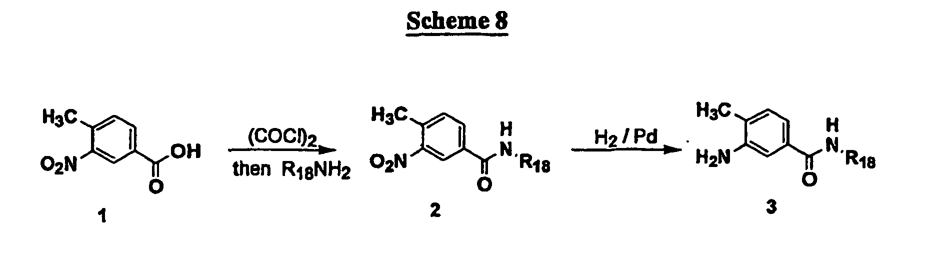

Commercially-available compound (1) can be reacted with oxalyl chloride with heating and then concentrated *in vacuo* and reacted with an amine R₁₈NH₂ in the presence of a base, such as diisopropylamine, in an organic solvent, such as DCM to yield compound (2). Compound (2) can be reacted with hydrogen in the presence of a catalyst, such as Pd, in an alcoholic solvent, such as EtOH, at rt to afford compound (3). Compound (3) can then be used as in Scheme 9 to produce compounds (6) of Scheme 9.

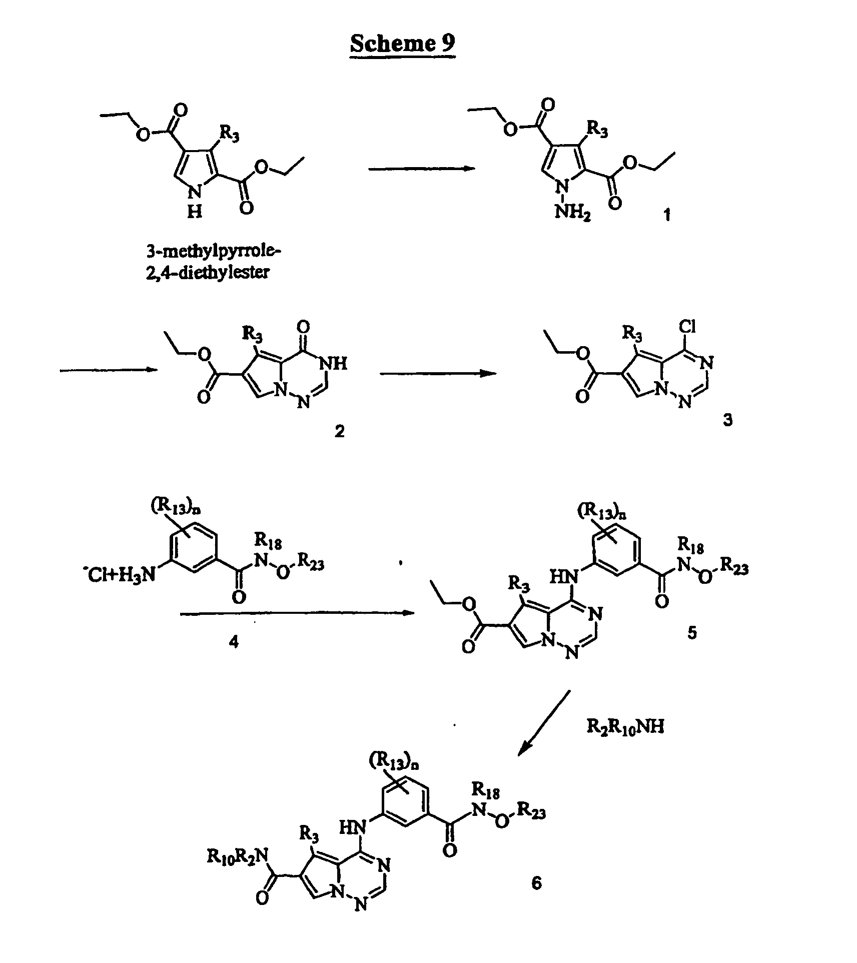

3-methyl-1-pyrrole-2,4-diethyl ester can be reacted with chloramine in ether to produce compound (1). Reacting compound (1) in formamide with acetic acid produces compound (2). Compound (2) can be reacted with DIPEA and POCl₃ in toluene to produce compound (3). Compound (3) can be reacted with DIPEA and compound (4) in DMF to produce compound (5). Compound (5) can be reacted in THF with NaOH to produce an acid intermediate which upon treatment with HOBt, EDCI and the appropriate amine (NR₂R₁₀ in DMF produces compounds (6).

Compound (4) can be prepared by 1) reacting commercially-available 4-amino-3-methylbenzoic acid and N*-*(*tert*-butoxycarbonyl)anhydride in THF to produce a BOC-protected aniline intermediate; 2) reacting the aniline intermediate with -(3-dimethylaminopropyl)-3-ethylcarbodiimide hydrochloride, HOBt, and DMF, followed by addition of methoxyamine hydrochloride and DIPEA to produce a BOC-protected N-methoxyamide intermediate; and 3) reacting that methoxyamide intermediate in a solution of HCl in dioxane to produce compound (4) as a hydrochloride salt. Alternatively, compound (4) can be prepared as shown in Scheme 8.

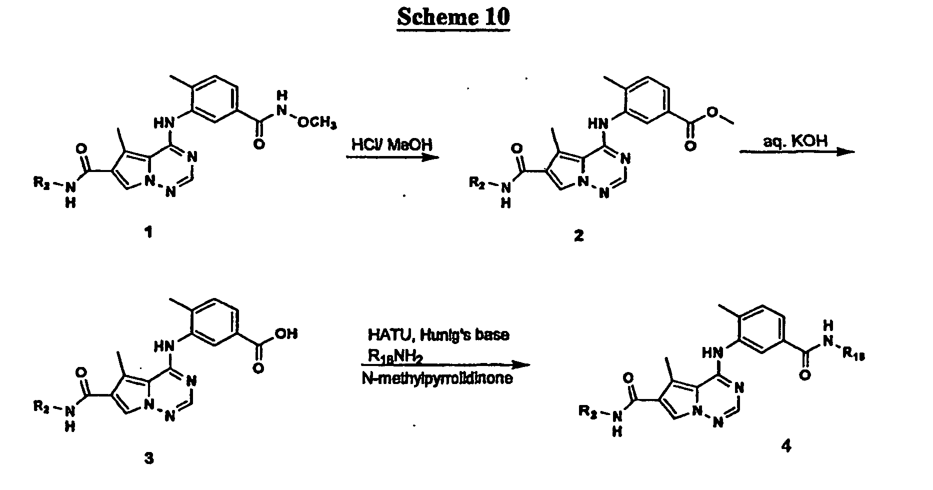

A substituted hydroxamate (1) can be reacted with acid, such as HCl, in anhydrous MeOH, to afford compound (2). Compound (2) can be reacted with an aq. base such KOH with heating to form compound (3). Compound (3) is reacted with an amine R₁₈NH₂ in the presence of a coupling reagent, such as HATU, and a base such as diisopropylamine, in an organic solvent, such as N-methylpyrrolidinone to afford compounds (4). Alternatively, compounds (4) may generally be prepared as outlined in Schemes 8 and 9.

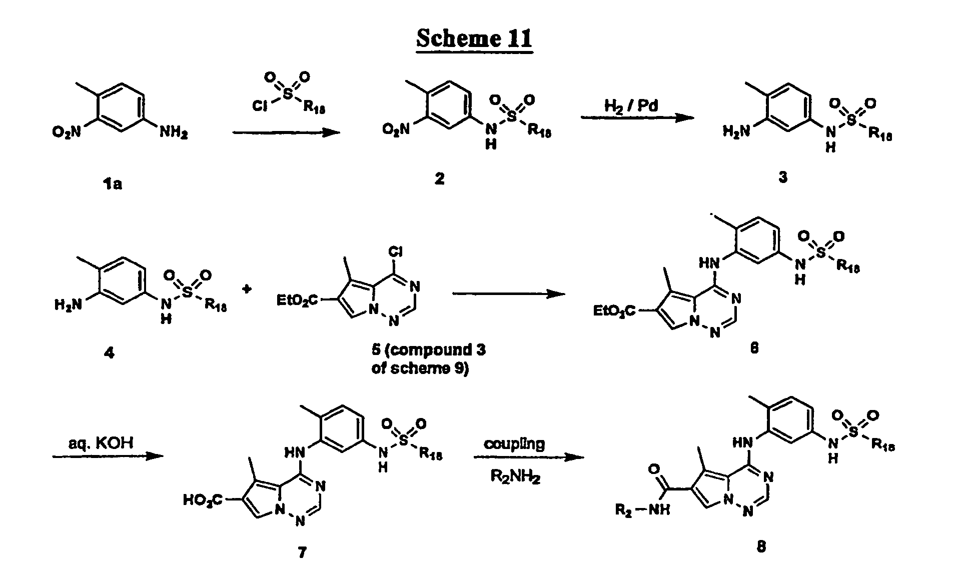

Commercially-available compound (1a) can be reacted with a sulfonyl chloride in the presence of a base, such as TEA, in an organic solvent, such as DCM to yield compound (2). Reaction of compound (2) with hydrogen in the presence of a catalyst, such as Pd in a solvent, such as MeOH, yields compound (3). Reaction of compound (3) with chloride (5) (compound 3 of scheme 9) in an organic solvent, such as DMF, at rt affords compound (6).

Reaction of compound (6) with aq. KOH with heating affords compound (7). Compound (7) can be reacted with an amine R₂NH₂ in the presence of a coupling reagent, such as EDCI, and a base such as diisopropylamine, in an organic solvent, such as DMF to afford compound (8).

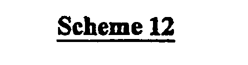

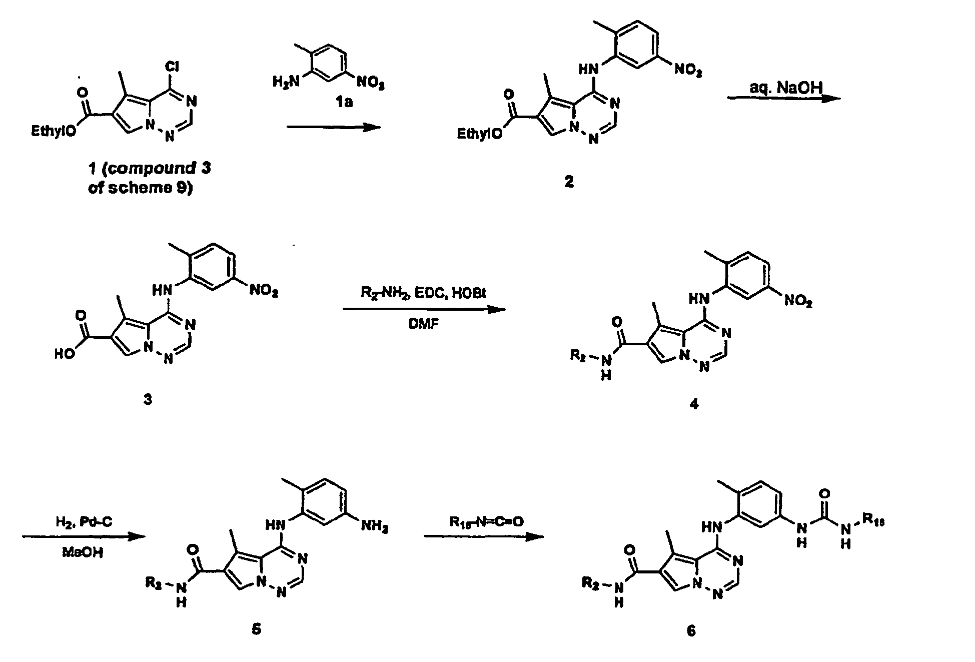

*Chloropyrrolotriazine* (1) (compound 3 of Scheme 9) can be reacted with an aniline (1a) (*e.g*., compound 1 of Scheme 11) in anhydrous DMF at rt to afford compound (2). Reaction of compound (2) with an aq. base such as NaOH with heating affords compound (3). Compound (3) can be reacted with an amine R₂NH₂ in the presence of a coupling reagent, such as HOBt, with or without a base such as diisopropylamine, in an organic solvent, such as DMF to afford compound (4). Compound (4) can be reacted with hydrogen in the presence of a catalyst, such as Pd/C, in an organic solvent, such as MeOH to afford compound (5). Reaction of compound (5) with an isocyanate in an organic solvent, such as DCE affords compound (6).

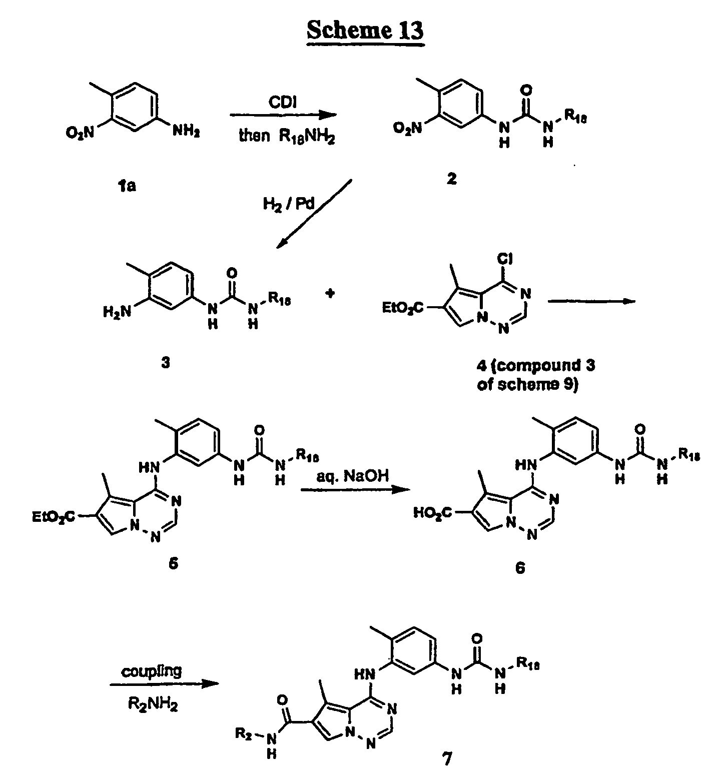

Commercially-available compound (1a) (compound 1a of Schemes 11 and 12), can be reacted with carbonyl diimidazole and with an amine R₁₈NH₂ in an organic solvent, such as DCE, to yield compound (8). Reaction of compound (8) with hydrogen in the presence of a catalyst, such as Pd, in an alcoholic solvent such as EtOH affords compound (9). Reaction of (9) with chloride (1) in an organic solvent, such as DMF, affords compound (10). Reaction of (10) with aq. NaOH with heating affords product (11). Product (11) can be reacted with an amine R₂NH₂ in the presence of a coupling reagent, such as EDCI, and a base such as diisopropylamine, in an organic solvent, such as DMF to afford compound (7).

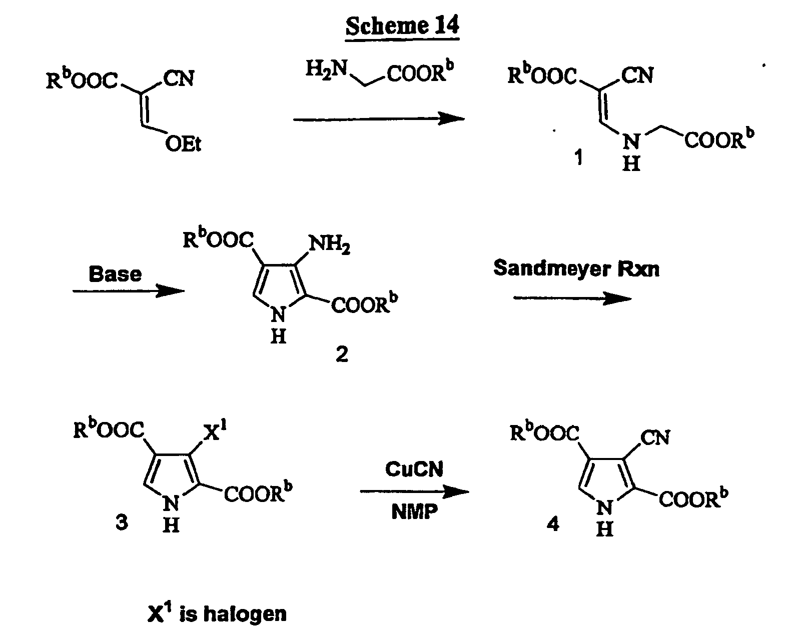

Glycine ethyl ester can be added to an alkyl alkoxy methylene cyanoacetate at from rt to 80°C to obtain compound (1). Compound (1) is cyclized to form pyrrole (2) upon treatment with a strong base, such as lithium hexamethyldisilazane, at from - 78°C to rt in an organic solvent such as THF. Pyrrole (2) is converted to a halide using sodium nitrite in an organic solvent, such as DMF, and a halide source, such as CuBr to yield compound (3). Compound (3) can be converted to compound (4) using CuCN in an organic solvent such as NMP at elevated temperatures. Alternatively, compound (2) can be directly converted to compound (4) using sodium nitrite in an organic solvent, such as DMF, and a cyanide source such as CuCN. Compounds (3) and (4) can then be used as described in previous schemes (*e.g*., as compound 3 of Scheme 2), to form compounds of Formula (I) wherein R₃ is halogen or cyano.

In addition, other compounds of formula I may be prepared using procedures generally known to those skilled in the art. In particular, the following examples provide additional methods for the preparation of the compounds of this invention.

The invention will now be further described by the following working examples, which are preferred embodiments of the invention. HPLC purifications were done on C18 reverse phase (RP) columns using water MeOH mixtures and TFA as buffer solution. These examples are illustrative rather than limiting. There may be other embodiments that fall within the spirit and scope of the invention as defined by the appended claims.

### Example 1

### 1-[2,3-Dihydro-6-(pyrrolo[2,1-f][1,2,4]triazin-4-ylamino)-1H-indol-1-yl]ethanone

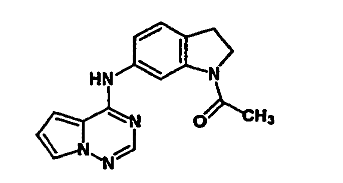

### A. 4-Bromo-pyrrolo[2,1-f][1,2,4]triazine

A mixture of 50 mg (0.37 mmol) of pyrrolo[2,1-f][1,2,4]triazin-4(3H)-one [prepared as described in S.A.Patil, B.A. Otter and R.S. Klein, *J. Het. Chem.,* 31, 781-786 (1994)] and 0.5 g of phosphorus oxybromide was heated and kept at 60°C for 20 min under argon. A clear orange melt was initially obtained which solidified to a yellow solid on continued heating. Ice was added with vigorous stirring. The mixture was extracted twice with EtOAc. The combined extracts were washed with sat. NaHCO₃ and brine, dried (MgSO₄), and the solvent removed to afford 63 mg of crude Compound A as an orange oil which crystallized on standing. (M+H)⁺ = 198⁺, 200⁺.

### B. Example 1

A solution of 60 mg (0.3 mmol) of Compound A and 1-acetyl-6-aminoindoline in 1.5 ml of acetonitrile was stirred overnight at rt under argon. A white precipitate was obtained which was removed by filtration The filter cake was suspended in 10% isopropanol/methylene chloride for extraction. Sat'd NaHCO₃ was added and the mixture stirred until a solution was obtained. The organic layer was separated and washed with brine, dried (MgSO₄), and the solvent removed. Purification by chromatography on silica gel with EtOAc yielded 4% of Example 1 as a white solid. (M+H)⁺ = 294.

### Example 2

### 4-(2,3-Dihydro-1H-indol-1-yl)-5-methylpyrrolo[2,1-f][1,2,4]triazine-6-carboxylic acid methyl ester

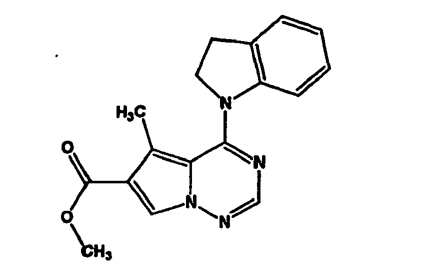

### A. Methylpyrrole-3-carboxylic acid methyl ester

To a 1.0 M solution of lithium hexamethyldisilazide in THF (41 mL, 41 mmol) at -78°C was added dropwise over 45 min, a solution of tosylmethyl isocyanide (8.1 g, 41 mmol) in THF. After the reaction was stirred for an additional 45 min., a solution of methyl crotonate in THF was added over 40 min. The reaction was warmed to 25°C and stirred for 5 h. The reaction was diluted with EtOAc and washed with sat. aq. NaHCO₃. The aqueous layer was extracted three times with EtOAc, dried (Na₂SO₄), concentrated, and purified by chromatography on silica gel eluting with a gradient of 20-30% EtOAc in hexanes to provide Compound A.

### B. 3-Methylpyrrole-2,4-dicarboxylic acid dimethyl ester

To a suspension of aluminum chloride (106.4 g, 798 mmol) in DCE (700 mL) at -40°C under nitrogen was added dropwise trichloroacetyl chloride (89 mL, 798 mmol). A solution of Compound A (37 g, 266 mmol) in DCE (200 mL) was added. The reaction mixture was gradually warmed to rt and stirred over the weekend (65 hr). A cold and pre-prepared mixture of aluminum chloride (53.2 g) and trichloroacetyl chloride (44.6 g) in DCE (450 mL) was added to the reaction mixture. After an additional 24 hr, the mixture was carefully poured into an ice-water bath (2 L) and the pH of the solution adjusted to 2.0. The organic layer was separated and the aqueous layer extracted with DCM. The combined organic extracts were washed with 3 N HCl, brine, dried (Na₂SO₄), and concentrated *in vacuo* to give a dark oil. This oil was dissolved in MeOH (400 mL), and the resulting solution was cooled to 0°C under nitrogen. To this solution was added sodium methoxide (25 % in MeOH) until the pH of the solution was 10. After 1 hr, the mixture was concentrated and then diluted with ice water (1L) and the pH of the mixture was adjusted to 6. The mixture was extracted with DCM (3 x 1L). The combined extracts were washed with NaHCO₃, brine, dried (Na₂SO₄), and concentrated *in vacuo.* The brown solid obtained was purified by chromatography on silica gel eluting with EtOAc in hexanes to provide 44.3 g (84%) of Compound B. MS: [M+H]⁻=196.

### C. 1-Amino-3-methylpyrrole-2,4-dicarboxylic acid dimethyl ester

To a suspension of NaH (60% in oil, 33 mg, 0.83 mmol) in DMF (5 mL) at 0°C was added Compound B (46 g, 213 mmol) in DMF (3 mL). After 10 min. at 0°C, diphenyl phosphoryl hydroxylamine (0.19 g, 0.83 mmol) was added neat followed by DMF (3 mL). The reaction mixture was stirred for 2 hrs at 25°C and then quenched with pH 7 phosphate buffer (15 mL). The mixture was extracted with EtOAc (4 x 20 mL). The combined extracts were dried (Na₂SO₄) and after purification by chromatography on silica gel eluting with 25-30% EtOAc in hexanes, 38 g (84%) of Compound C was obtained as white solid. ESI [M+H]⁻ = 213.1.

### D. 5-Methylpyrrolo[2,1-f][1,2,4]triazin-4(3H)-one-6-carboxylic acid methyl ester

Compound C (38 g, 179 mmol) was combined with formamide (400 mL) and heated to 165°C for 6 hr. The reaction was diluted with water (5 mL), extracted with EtOAc (3 x 10 mL), dried (Na₂SO₄), and concentrated. The crude material was purified by washing with ether/hexanes (7/3) to provide 33.4 g (90%) of Compound D as a white solid. ESI MS: [M-H]⁻= 206.0

### E. 4-Chloro-5-methyl-6-carbomethoxypyrrolo[2,1-f][1,2,4]triazine

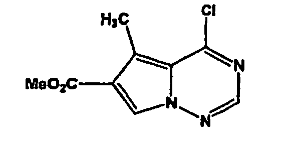

Phosphorous oxychloride (2.5 mL) was combined with Compound D (100 mg, 0.483 mmol) and heated at 100°C overnight. The melt was allowed to cool to rt and dissolved in EtOAc. The mixture was neutralized with aq. NaHCO₃ and extracted twice with EtOAc. The combined organic washes were dried (Na₂SO₄), and concentrated to provide 101 mg (93%) of Compound E. MS: (M+H)⁺ = 226.6.

### F. Example 2

A mixture of Compound E (20 mg, 0.09 mmol) and indoline (21 mg, 0.177 mmol) in CH₃CN (1mL) was shaken for 4 hrs. Then DMF (0.2 mL) was added, and the crude mixture was purified by preparative HPLC to provide 12.2 mg (45 %) of Example 2 as a white solid. [M+H]⁺=309.2; ¹H NMR (CDCl₃): δ 8.06 (s, 1H), 7.91 (s, 1H), 7.20 (m, 1H), 7.01 (m, 1H), 6.93-6.91 (m, 2H), 4.15 (t, *J*=7.8 Hz, 2H), 3.81 (s, 3H), 3.09 (t, *J*=7.8 Hz, 2H), 2.35 (s, 3H).

### Examples 3-6

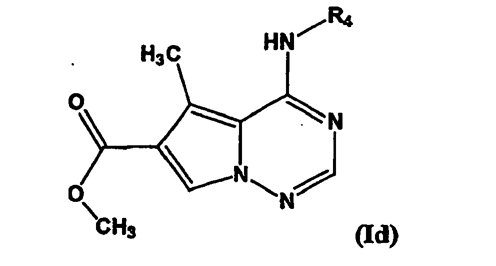

Compounds having the formula (Id), wherein the group R₄ has the values listed in Table 1, were prepared following the method of Example 2, using an appropriately-selected amine compound in place of indoline in step F.

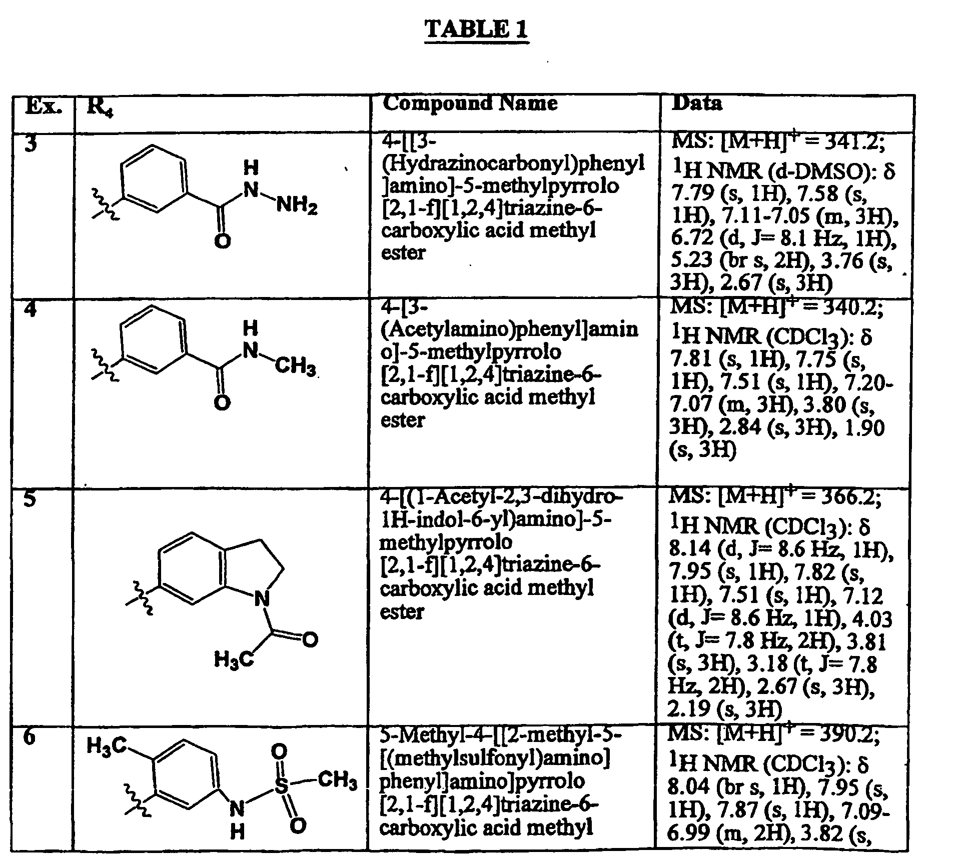

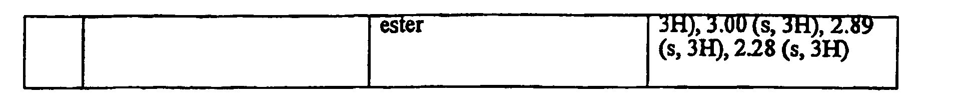

### Example 7

### 4-(1H-Indazol-6 ylamino)-5-methylpyrrolo[2,1-f][1,2,4]triazine-6-carboxylic acid methyl ester

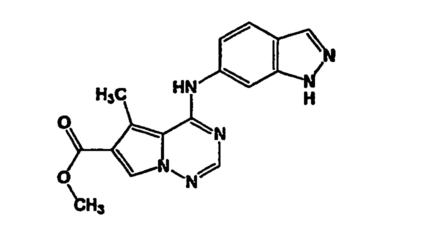

A mixture of Compound E from Example 2 (20 mg, 0.09 mmol) and 6-aminoindazole (18 mg, 0.13 mmol) in CH₃CN (1 mL) and DMSO (0.5 mL) was shaken for 4 hrs. The mixture was filtered, washed with CH₃CN, and the crude material was purified by preparative HPLC to provide Example 7 as a white solid (13 mg, 45 %). [M+H]⁺=323.1; ¹H NMR (CDCl₃): δ 8.37 (s, 1H), 7.99 (s, 1H), 7.94 (d, *J*=7.4 Hz, 1H), 7.68 (d, *J* = 4.2 Hz,1H), 7.00 (dd, *J*=7.4, 4.2 Hz, 1H), 3.83 (s, 3H). 2.91 (s, 3H).

### Example 8

### 5-Methyl-4-[[3-[(methylsulfonyl)amino]phenyl]amino]pyrrolo[2,1-f][1,2,4]triazine-6-carboxylic acid methyl ester

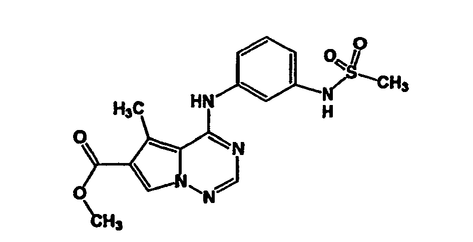

Compound E of Example 2 was dissolved in DMF (2 mL), and then 3-(methylsulfonylamino)aniline(54 mg, 0.3 mmol) was added. The reaction mixture was stirred for 4 hrs under argon at 25°C. The crude reaction mixture was purified by preparative HPLC. The material obtained appeared to be a salt of the desired compound. The material was dissolved in EtOAc and washed with sat'd NaHCO₃. Evaporating the solvent gave 24 mg (40 %) of Example 8. MS: [M+H]⁺= 376.2; ¹H NMR (*d*-DMSO): δ 8.89 (s, 1H), 8.14 (s, 1H), 7.95 (s, 1H), 7.55 (s, 1H), 7.38 (s, 1H), 7.34-7.32 (m, 2H), 7.01 (d, *J*=8.0 Hz, 1H), 3.81 (s, 3H), 3.02 (s, 3H), 2.82 (s, 3H).

### Example 9

### 4-[[3-(Aminosulfonyl)phenyl]amino]-5-methylpyrrolo[2,1-f][1,2,4]triazine-6-carboxylic acid methyl ester

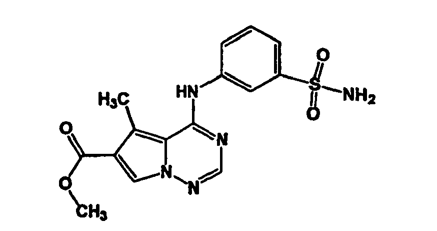

Compound E from Example 2 (20 mg, 0.09 mmol) was mixed with 3-aminobenzenesulfonamide (23 mg, 0.13 mmol) in DMF (1 mL) and shaken for 4 hrs. Evaporating the extracting solvent and purification by preparative HPLC gave 8.6 mg (58 %) of Example 9 as a solid. MS: [M+H]⁺= 362; ¹H NMR (*d*-DMSO): δ 9.08 (s, 1H), 8.18 (s, 2H), 7.98 (s, 1H), 7.91-7.89 (m, 1H), 7.62-7.58 (m, 2H), 7.42 (s, 2H), 3.81 (s, 3H), 2.84 (s, 3H)

### Examples 10-14

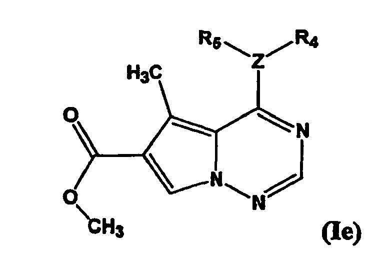

Compounds having the formula (Ie), wherein the groups Z, R₄, and R₅ together have the values listed in Table 2, were prepared following the method of Example 9, except instead of 3-aminobenzenesulfonamide, an appropriately-selected amino compound was used.

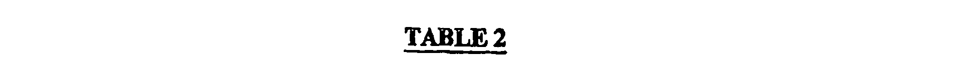

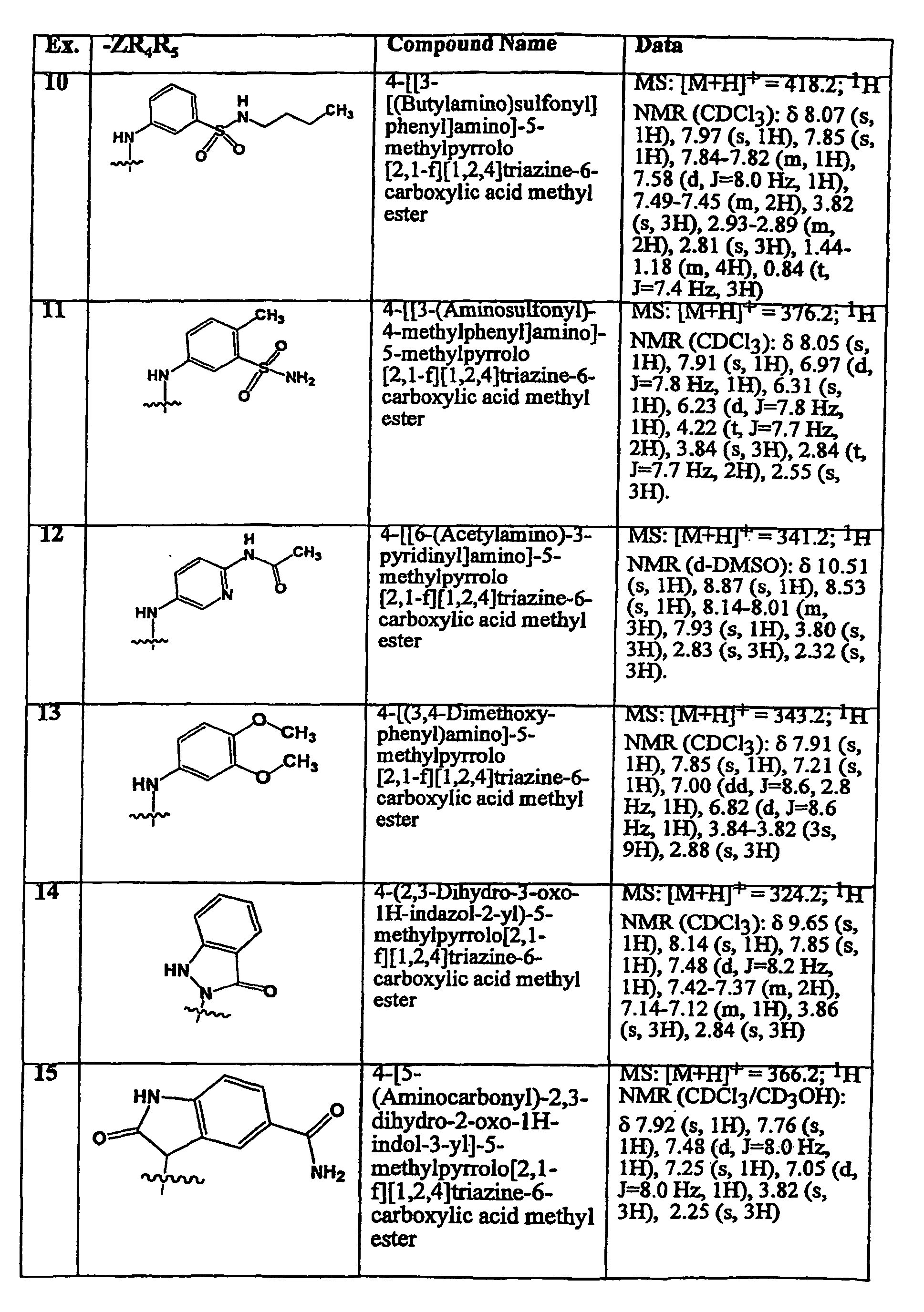

### Example 16

### 4-(2,3- Dihydro-2-oxo-1H-indol-3-yl)-5-methylpyrrolo[2,1-f] [1,2,4]triazine-6-carboxylic acid methyl ester

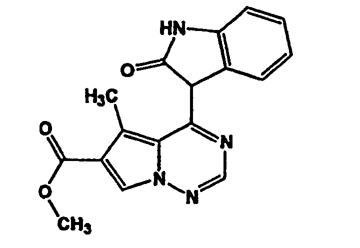

A solution of oxindole (5.32 g, 40 mmol) in THF (150 ml) and DMF (35 ml) was deoxygenated by purging with argon. This mixture was placed in an ice bath, and NaH (60% in oil, 1.7 g, 42 mmol) was added. After 30 min, 4-Chloro-5-methyl-6-carbomethoxypyrrolo[2,1-f][1,2,4]triazine (Compound E of Example 2) (3.38 g, 15 mmol) was added. After 1 hr at rt, the resulting mixture was neutralized with acetic acid. The solvent was removed *in vacuo.* The residue was dissolved in DCM, washed with brine, and dried (MgSO₄). The solution was concentrated to a solid residue which was triturated with DCM and diethyl ether to afford the title compound as an orange solid (3.5 g, 72%). MS: [M+H]⁺ = 323.1; ¹H NMR (CDCl₃): δ 7.86 (s, 1H), 7.74 (s, 1H), 7.37 (s, 1H), 7.19 (d, *J*=7.6 Hz, 1H), 7.11 (t, *J*=7.6 Hz,1H), 7.10 (t, *J*=7.6 Hz, 1H), 7.06 (d, *J*=7.6 Hz, 1H), 3.84 (s, 3M, 2.34 (s, 3H).

### Example 17

### 4-(2,3-Dihydro-3-ozo-1H-indazol-1-yl)-5-methylpyrrolo[2,1-f][1,2,4]triazine-6-carboxylic acid methyl ester

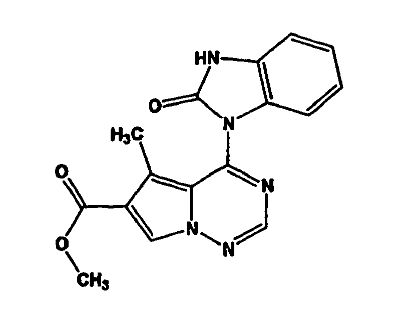

Example 17 above was prepared following the method of Example 16, except 3-indazolinone was used in place of oxindole. [M+H]⁺= 324; ¹H NMR (CDCl₃): δ 8.16 (s, 1H), 8.02-7.99 (m, 2H), 7.83-7.81 (m, 1H), 7.58-7.54 (m, 1H), 7.46 (s, 1H), 3.87 (s, 3H), 2.66 (s, 3H).

### Example 18

### 4-(2,3-Dihydro-1-methyl-2-oxo-1H-indol-3 yl)-5-methylpyrrolo[2,1-f][1,2,4]triazine-6-carboxylic acid methyl ester

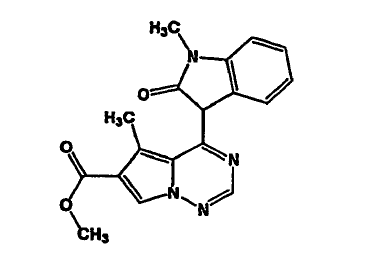

To a 0°C mixture of NaH (60%, 5 mg, 0.106 mmol) in DMF (0.5 mL) was added N-methyloxindole (22 mg, 0.15 mmol). The reaction mixture was stirred for 10 min. at 0°C. Compound E of Example 2 (22 mg, 0.10 mmol) in DMF (1 mL) was added. The reaction mixture was stirred for 45 min. at 25°C and then quenched with pH 7 phosphate buffer. The mixture was extracted with EtOAc. The combined extracts were dried (Na₂SO₄) and purified by preparative HPLC to provide Example 18 as a yellow solid. MS: [M+H]⁺= 337.2; ¹H NMR (CDCl₃): δ 7.90 (s, 1H), 7.40 (s, 1H), 7.16 (m, 2H), 7.02 (t, *J*=7.6 Hz, 1H), 6.90 (d, *J*=7.6 Hz, 1H), 5.28 (s, 1H), 3.86 (s, 3H), 3.36 (s, 3H), 2.37 (s, 3H).

### Examples 19-24

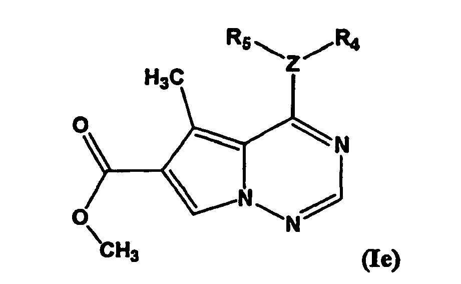

Compounds having the formula (Ie), wherein the groups Z, R₄, and R₅ together have the values listed in Table 3, were prepared following the method of Example 18, except instead of oxindole, an appropriately-substituted oxindole was used, and for Examples 19 and 20, 2,3-dihydro-2-oxo-1H-benzimidazol (40.2 mg, 0.3 mmol) and (methylsulfonyl)amino]-2-oxo-1H-indole (90 mg, 0.4 mmol) were used, respectively, in place of oxindole. After purification by preparative HPLC, the desired material can be collected, concentrated, and neutralized with aq. NaHCO₃ or desilylated with tetrabutylammonium fluoride.

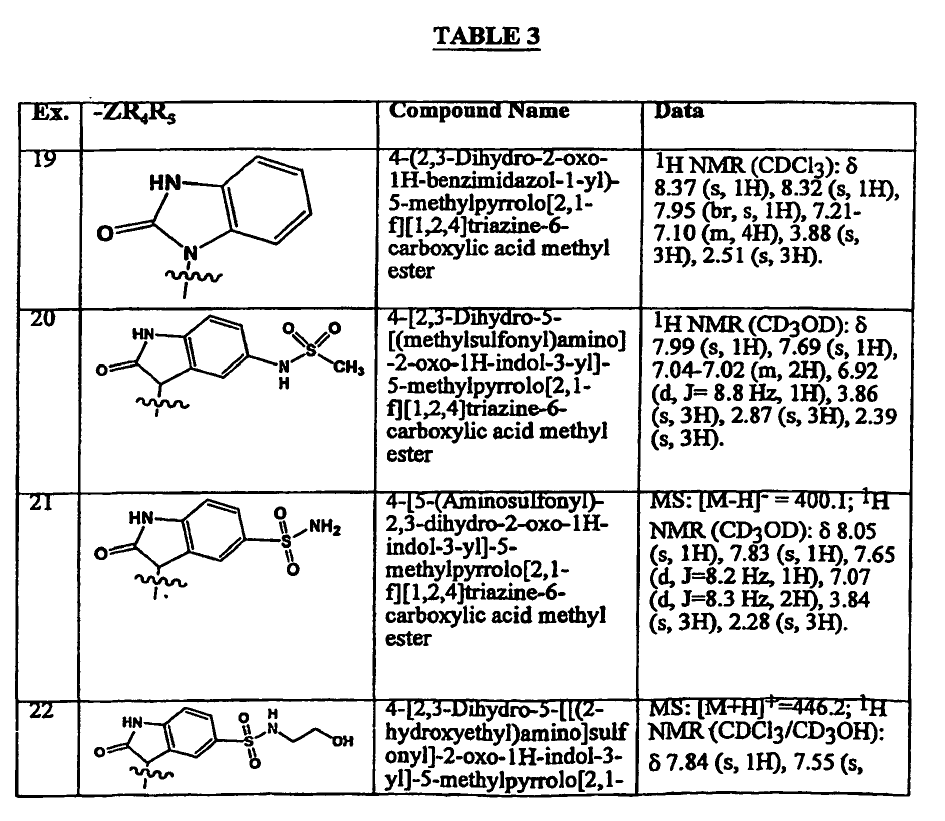

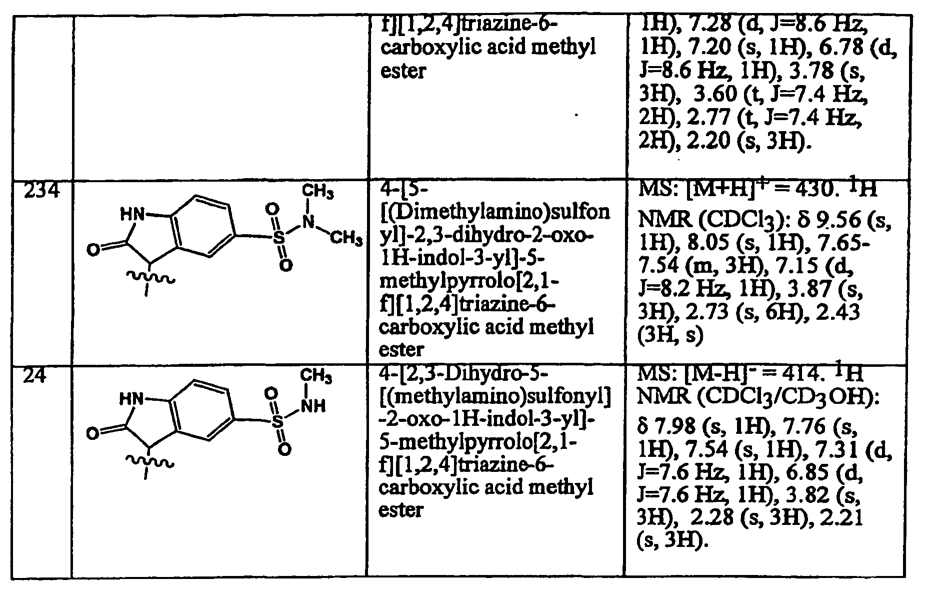

### Example 25

### 5-Methyl-4-(1,2,3,4-tetrahydro-3-oxo-1-quinoxalinyl)pyrrolo[2,1-f] [1,2,4]triazine-6-carboxylic acid methyl ester

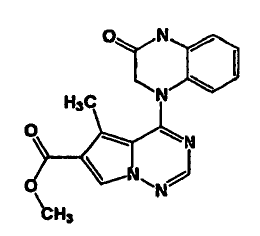

Example 2, Compound E (23 mg, 0.1 mmol) was stirred with 1,2,3,4-tetrahydroquinoxalin-2-one (44.4 mg, 0.3 mmol) in DMF (0.5 mL) for 1 hr at 50°C. Water was added, and the resulting solid material was collected, washed with water, and dried. The material was triturated with MeOH, filtered, and dried again to provide 20 mg (59%) of Example 25 as a white solid. ¹H NMR (d-DMSO): δ 8.30-8.25 (m, 2H), 7.12-7.03 (m, 2H), 6.83 (br s, 2H), 4.38 (s, 2H), 3.73 (s, 3H), 2.49 (s, 3H), 1.72 (s, 3H).

### Example 26

### 4-(2,3-Dihydro-2-oxo-1H-indol-3-yl)-5-methylpyrrolo[2,1-f] [1,2,4]triazine-6-carboxylic acid

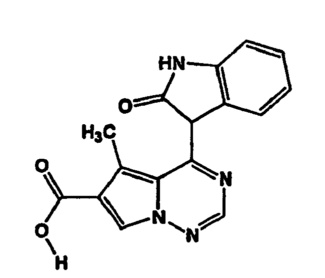

To a solution of Example 16 (3.3 g, 10.2 mmol) in MeOH (600 mL) was added KOH (1N aq. solution, 200 mL), and the mixture was deoxygenated by purging with argon. The reaction mixture was heated to 60°C for 20 hrs, then cooled and concentrated to about 50 mL. The residue was acidified with concentrated HCl to pH 4. The yellow solid was collected, washed with water, and dried *in vacuo* to afford the title compound (2.9 g, 92%). MS: [M+H]⁺= 307.1; ¹H NMR (CD₃OD): δ 7.94 (s, 1H), 7.71 (s, 1H), 7.18-7.10 (m, 2H), 6.94-6.86 (m, 2H), 2.45 (s, 3H).

### Example 27

### 4-(2,3-Dihydro 2-ozo-1H-indol-3 yl)-5-methozypyrrolo[2,1-f][1,2,4]triazine-6-carboxylic acid ethyl ester

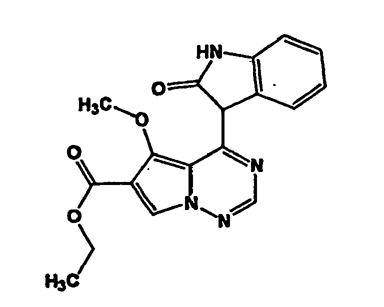

### A. [[(2-Ethoxy-2-oxoethyl)(phenylmethyl)amino]methylene] propanedioic acid diethyl ester

N-benzylglycine ethyl ester (5.79 g, 30 mmol) was combined with diethyl ethoxymethylene malonate (6.48 g, 30 mmol) and stirred at 120°C for 1 hr. The crude material was used directly for the next reaction.

### B. 1-Phenylmethyl-3-hydroxypyrrole-2,4-dicarboxylic acid diethyl ester

To a suspension ofNaH (60% in oil, washed with hexanes, 500 mg, 12.5 mmol) in toluene (10 mL) was added Compound A (3.63 g, 10 mmol) in toluene (30 mL) dropwise at 50°C. After 2 hr, the mixture was poured into ice water and acidified with 1 N aq. HCl. The mixture was extracted three times with EtOAc. The combined organic extracts were dried (MgSO₄) and concentrated *in vacuo.* The crude material was purified by chromatography on silica gel eluting with 50% EtOAc in hexanes to provide 2.70 g (85%) of Compound B as a pink oil.

### C. 1-Phenylmethyl-3-methoxypyrrole-2,4-dicarboxylic acid diethyl ester

Compound B (634 mg, 2 mmol) was stirred in acetone for 10 hrs at rt with methyl iodide (300 mg, 2.1 mmol) and K₂CO₃ (500 mg). The mixture was filtered, concentrated, and purified by chromatography on silica gel eluting with 33% EtOAc in hexanes to provide 470 mg (71 %) of Compound C as a gel.

### D. 3-methoxypyrrole-2,4-dicarboxylic acid diethyl

Compound C (27 g, 81.5 mmol) in EtOH (1 L) was mixed with Pd/C (10%, 4 g) and ammonium formate (28 g) and hydrogenated at 40 psi at 90°C for 18 hrs. The reaction mixture was cooled to rt, filtered, and concentrated. The crude material (brown oil) was purified by chromatography on silica gel eluting with 25% EtOAc in hexanes to provide 13 g (66%) of tan solid.

### E. 1-Amino-3-methoxypyrrole-2,4-dicarboxylic acid diethyl ester

To a stirred suspension ofNaH (60% in oil, 1.76 g, 70 mmol) in DMF (350 mL) under nitrogen at 0° C was added dropwise a solution of Compound D (13 g, 54 mmol) in DMF (200 mL). After 30 min, the mixture was diluted with DMF (750 mL), and then diphenyl phosphoryl hydroxylamine (15.7 g, 67.4 mmol) was added and the reaction mixture was allowed to warm to rt. After 6 hrs, the mixture was concentrated and the residue diluted with water (1 L) and extracted with EtOAc (3x1 L). The combined organic extracts were dried (MgSO₄), concentrated, and purified by chromatography on silica gel eluting with 20% EtOAc in hexanes to provide 13 g (93 %) of solid.

### F. 4-Hydroxy-5-methozypyrrolo[2,1-f] [1,2,4]triazine-6-carboxylic acid ethyl ester

Compound E (100 mg, 0.39 mmol) was combined with formamide (1 mL) and heated at 180°C for 6 hrs. The reaction mixture was cooled to rt and diluted with water (5 mL). The solid which formed was collected, washed with water, and dried to provide 70 mg (76%) of Compound F.

### G. 4-Chloro-5-methoxypyrrolo[2,1-f][1,2,4]triazine-6-carboxylic acid ethyl ester

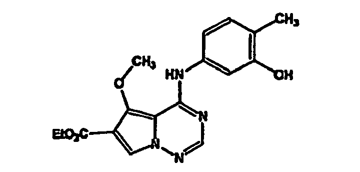

Phosphorous oxychloride (1 mL) was combined with Compound F (23.7 mg, 0.1 mmol) and heated at reflux for 2 hrs. The melt was allowed to cool to rt and phosphorous oxychloride was removed on rotary evaporator.

### H. Example 27

To a suspension ofNaH (60%, 44 mg, 1.1 mmol) in THF (1 mL) was added oxindole (133 mg, 1 mmol). The reaction mixture was stirred for 20 min. at rt and Compound G (0.1 mmol) was added. The reaction was stirred for 2 hrs at 25°C. The crude material was purified by preparative HPLC followed by chromatography on silica gel eluting with EtOAc to provide 5.5 mg (16%) of Example 27 as a yellow solid. MS: [M+H]⁺= 353; ¹H NMR (CDCl₃): δ 8.42 (s, 0.4H), 8.10 (s, 0.6H), 7.79 (s, 1H), 7.75-6.88 (m, 4H), 4.33 (m, 2H), 3.57 (s, 3H), 1.37 (m, 3H).

### Example 28

### 5-Methyl-4-[[4-methyl-3-[(methylsulfonyl)amino]phenyl]amino]pyrrolo[2,1-f][1,2,4]triazine-6-carboxylic acid methyl ester

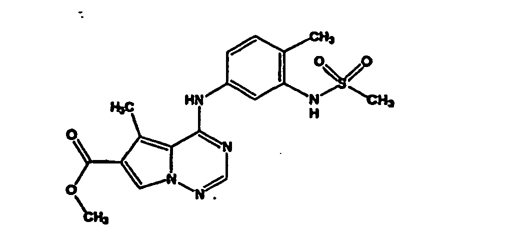

To a solution of Example 6 (16 mg, 51 µmol) in pyridine (1 mL) at 0°C was added 4-methyl-3-[(methylsulfonyl)aniline (4.4 µL, 87 µmol). The reaction mixture was stirred for 1 hr at 0°C and then warmed to 25°C and stirred for 4 hrs. Water (5 mL) was added and the mixture was extracted with EtOAc (3 x 5 mL). The combined organic extracts were washed with water (10 mL) and brine (10 mL) and dried (Na₂SO₄). The crude material was purified by chromatography on silica gel eluting with 2% MeOH in chloroform to provide 6.9 mg (30%) of solid. MS: [M+H]⁺= 390.2; ¹H NMR (CDCl₃): δ 7.93 (s, 1H), 7.86 (s, 1H), 7.77 (s, 1H), 7.35 (d, *J*=8.2 Hz, 1H), 7.27 (s, 1H), 7.18 (d, *J*=8.2 Hz, 1H), 6.25 (s, 1H), 3.82 (s, 3H), 3.03 (s, 3H), 2.86 (s, 3H), 2.24 (s, 3H)

### Example 29

### [4-(2,3-Dihydro-2-oxo-1H-indol-3-yl)-5-methylpyrrolo[2,1-f] [1,2,4]triazin-6-yl]carbamic acid phenylmethyl ester

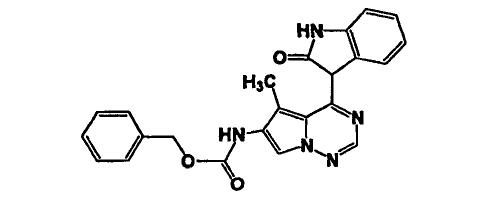

To a solution of Example 26 (29 mg, 0.09 mmol) in 1,4-dioxane (0.6 mL) under Ar with powdered 4 Å molecular sieves was added TEA (10 µL, 71µmol), diphenylphosphoryl azide (15 µL, 71 µmol) and benzyl alcohol (12 _{J}.lL. 0.12 mmol). The reaction was warmed at 50°C for 15 hrs. The mixture was concentrated *in vacuo* and chromatographed directly on silica gel eluting with a gradient of 2-5% MeOH in chloroform to provide 8 mg (50%) of an intermediate product as a white solid. Phosphorous oxybromide (5 eq.) was combined with this intermediate (16 mg, 0.054 mmol) and heated to 60°C for 20 min. The melt was poured into ice water and extracted with EtOAc (4 x 5 mL). The extracts were washed with aq. NaHCO₃, dried (Na₂SO₄), and concentrated *in vacuo.* The residue was dissolved in a mixture of CH₃CN (0.5 mL) and DMF (0.1 mL), and then 5-amino-o-cresol (10 mg, 0.081 mmol) was added. The reaction mixture was stirred overnight under argon at 25°C. Solvent was removed *in vacuo,* and the crude material was purified by rotary chromatography on a 1 mm silica gel plate eluting with 2% MeOH in chloroform to afford the title compound as yellow oil (5 mg, 13%). MS: [M+H]⁺= 414; ¹H NMR (CDCl₃): δ 7.94 (s, 1H), 7.82 (s, 1H), 7.41-7.34 (m, 5H), 7.17-7.14 (m, 1H), 7.04-7.02 (m, 1H), 6.93-6.90 (m, 2H), 6.44 (s, 1H), 5.23 (s, 2H), 2.12 (s, 3H).

### Example 30

### 4-(2,3-Dihydro-methyl-2-oxo-1H-pyrazolo[2,3-d]pyrimidin-3-yl)-5-methylpyrrolo[2,1-f][1,2,4]triazine-6-carboxylic acid methyl ester

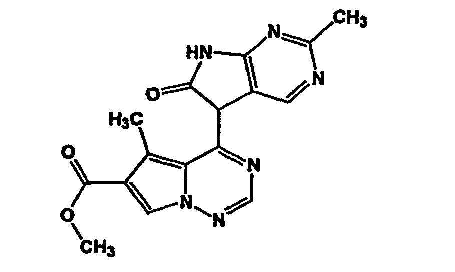

### A. 6-Methyl-5,7-diazaoxindole

To a solution of ethyl (4-amino-2-methylpyrimidin-5-yl) acetate (WO 99/10349, 0.975 g, 5 mmol) in THF (30 ml), was slowly added potassium t-butoxide (1 M in THF, 5 mL). After one hour, the mixture was neutralized with acetic acid to pH 5. The volatiles were removed *in vacuo* and the residue purified by flash column chromatography (silica gel, 5-8 % MeOH in DCM) to afford a yellow solid (680 mg, 91 %).

### B. Methylpyrrolo[2,1-f][1,2,4]triazine-6-carboxylic acid methyl ester

To a solution of 6-methyl-5,7-diazaoxindole (67 mg, 0.45 mmol) in DMF (2 ml) and THF (1 ml) was added NaH (60% in oil, 20 mg, 0.5 mmol). After stirring for 20 min, Compound E of Example 2 (34 mg, 0.15 mmol) was added. The mixture was stirred at rt overnight and then neutralized with acetic acid. DCM (10 ml) was added and the resulting precipitate was collected and washed with small amounts of DCM and water and then dried *in vacuo* to give the title compound as an orange solid (32 mg,63%). MS: (M+H) = 359

### Example 31

### 4-(2,3-Dihydro-2-oxo-1H-pyrazolo[2,3-b]pyridin-3-yl)-5-methylpyrrolo[2,1-f][1,2,4]triazine-6-carbozylic acid methyl ester

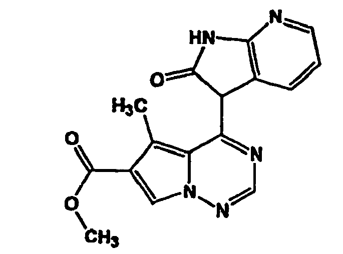

To a solution of 7-azaoxindole (*see* Tetrahedron.Lett., Vol. 28 (1987), at p. 4027) (60 mg, 0.45 mmol) in DMF (2 mL) and THF (1 mL) was added NaH (60% in oil, 20 mg, 0.5 mmol). After stirring for 20 min, Compound E of Example 2 (34 mg, 0.15 mmol) was added, and the mixture was stirred at rt overnight. The solution was neutralized with acetic acid, and then DCM (10 ml) was added to the mixture. The resulting solid was collected, washed with small amounts of DCM and water and dried *in vacuo* to give a yellow solid (35 mg, 72%). LC-MS: (M+H)⁺ = 324.

### Example 32

### 4-(2,3-Dihydro-2-oxo-1H-indol-3-yl)pyrrolo[2,1-f][1,2,4]triazine-6-carboxylic acid methyl ester

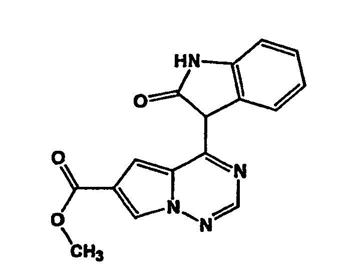

Example 2 was following using 2-methoxycarbonyl pyrrole as the starting pyrrole to afford 4-chloro-6-carbomethoxypyrrolo[2,1-f][1,2,4]triazine, which was then converted to Example 32 using the same or similar procedure of Example 31.

### Examples 33-36

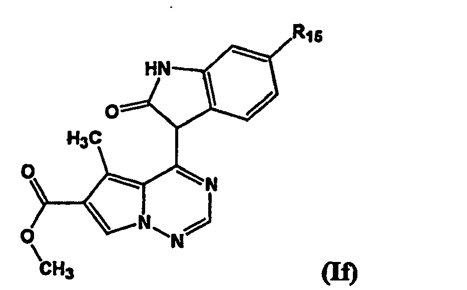

Compounds having the formula (If), wherein R₁₅ has the values listed in Table 4, were prepared by following the procedure of Example 32 using appropriate reagents known in the literature (see, WO 97/42187).

**TABLE 4**

| **EX.** | **R**_{**15**} | **Compound name** |
|---|---|---|
| 33 | F | 4-[6-Fluoro-2-hydroxy-1H-indol-3-yl]-5-methylpyrrolo[2,1-f][1,2,4]triazine-6-carboxylic acid methyl ester |
| 34 | Br | 4-[6-Bromo-2-hydroxy-1H-indol-3-yl]-5-methylpyrrolo[2,1-f][1,2,4]triazine-6-carboxylic acid methyl ester |
| 35 | CF₃ | 4-[2,3-Dihydro-2-oxo-6-(trifluoromethyl)-1H-indol-3-yl]-5-methylpyrrolo[2,1-f][1,2,4]triazine-6-carboxylic acid methyl ester |
| 36 | SO₂Me | 4-[2,3-Dihydro-6-(methylsulfonyl)-2-oxo-1H-indol-3-yl]-5-methylpyrrolo[2,1-f][1,2,4]triazine-6-carboxylic acid methyl ester |

### Examples 37-49

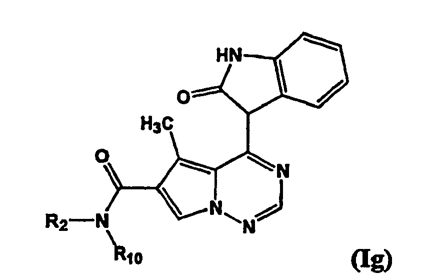

To Example 26 (50 mg, 0.16 mmol) in DMF (1 mL) and DCM (0.5 mL) were added PyBrop (113 mg, 0.24 mmol) and DIPEA (0.08 mL, 0.5 mL). After 10 min, an appropriate amine was added. After 15 h, the reaction mixture was purified by preparative RP HPLC to provide the compounds of formula (Ig), above, wherein R₂ and R₁₀ have the values listed in Table 5.

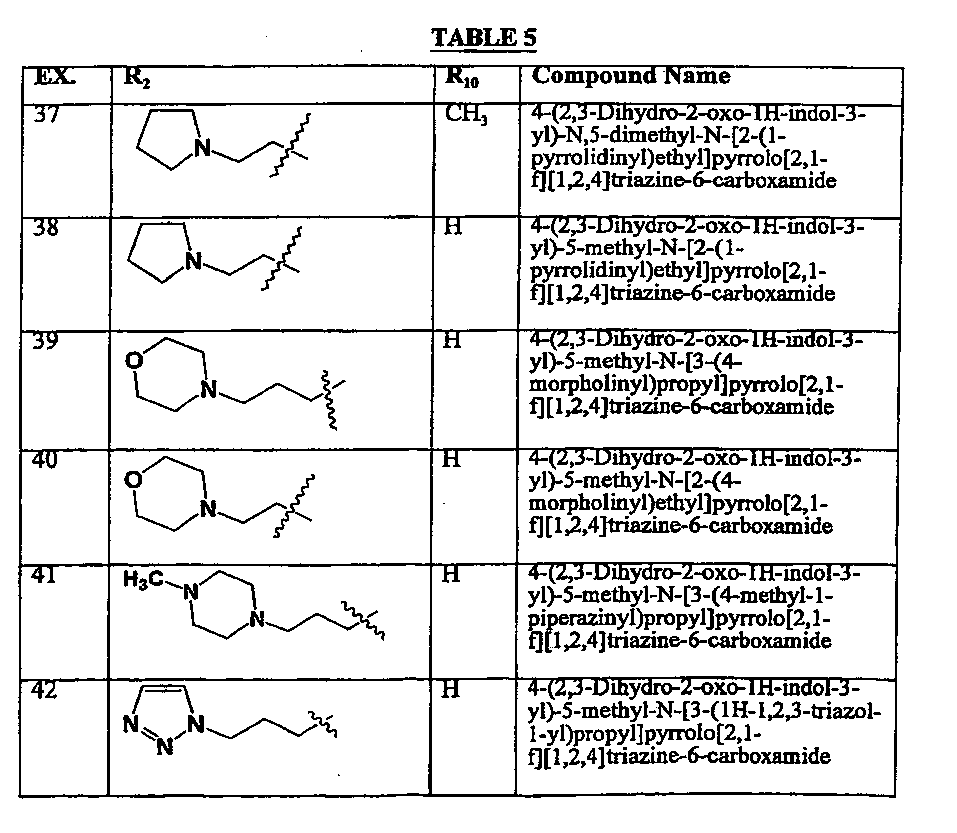

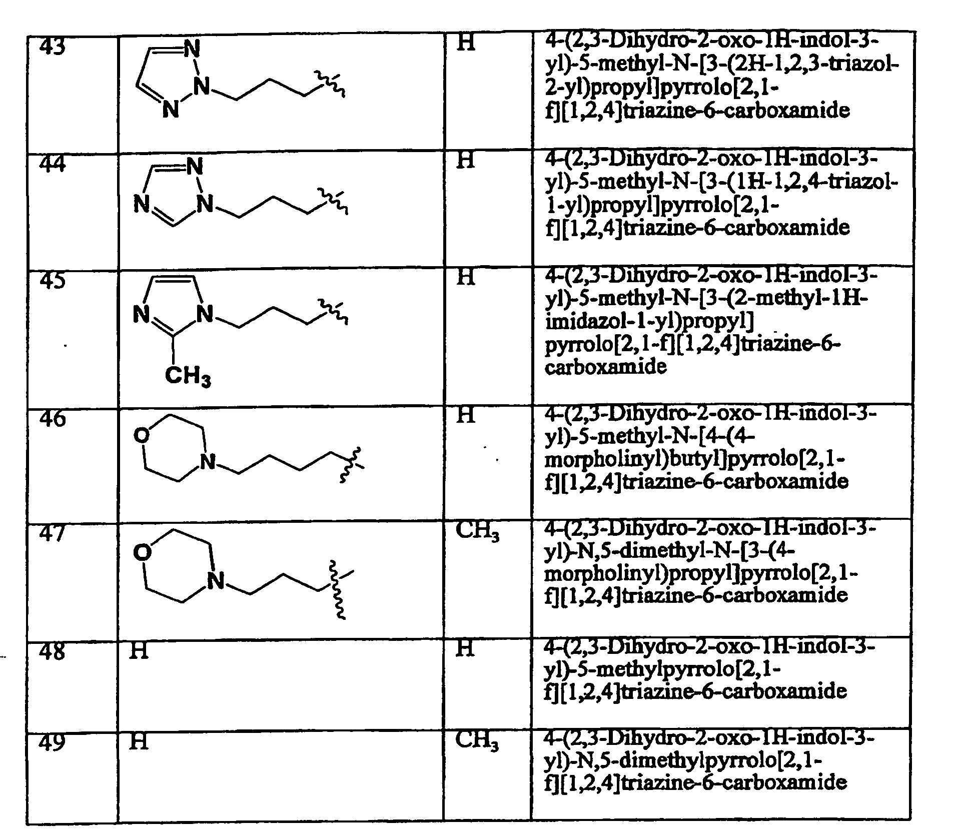

### Examples 50-52

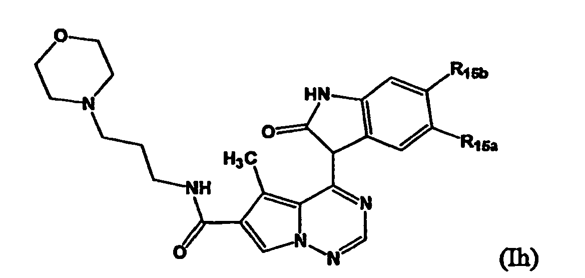

Compounds having formula (Ih), wherein R₁₅ₐ and R_{15b} have the values listed in Table 6 below, were prepared by the following process, using the appropriately substituted oxindole in step C.

### A. 5-Methylpyrrolo[2,1-f][1,2,4]triazin-4(3H)-one-6-carboxylic acid

To a solution of 5-Methylpyrrolo[2,1-f][1,2,4]triazin-4(3H-one-6-carboxylic acid methyl ester (Compound D from Example 2,1.035 g, 5.00 mmol) in a mixture of THF/MeOH/water (50 mL, 3:1:1) was added lithium hydroxide (2.062 g, 49.1 mmol). The reaction mixture was stirred at 55°C for 12 h, then cooled to 0°C and neutralized by 3N HCl. The organic solvents were removed and the aqueous solution brought to pH 4 with 1 N HCl. The resulting precipitate was filtered, rinsed with cold water, and air dried to afford Compound A as an off-white solid (0.965 g, 100%).

### B. 4-Chloro-5-methyl-N-[3-(4-morpholinyl)propyl]pyrrolo[2,1-f][1,2,4]triazine-6-carboxamide

A suspension of Compound A (2.00 g, 10.4 mmol) in phosphorous oxychloride (8 mL) was stirred at 100°C over 4 h. The solvent was removed *in vacuo* using toluene to assist in the removal. The resulting green solid was suspended in acetonitrile (20 mL) at 0°C and treated with sufficient TEA (5 mL) to bring the solution to pH 10. 4-(3-aminopropyl)morpholine (1.5 mL, 10.3 mmol) was added, and the solution was allowed to stir at rt over 1 h. The reaction mixture was poured into saturated sodium bicarbonate solution and extracted with EtOAc. The organic layer was dried (MgSO₄) and the volatiles were removed *in vacuo* to afford Compound B as a yellow solid (1.75 g, 50%).

### C. Examples 50-52

To a solution of an appropriately substituted oxindole, *i.e.,* 5-fluoro oxindole for Example 50 (36 mg, 0.24 mmol) in DMF (1 mL) was added NaH (5.9 mg, 0.23 mmol). After 30 min at rt, a solution of Compound B (24 mg, 0.072 mmol) in DMF (1 mL) was added and the resulting mixture was stirred at rt over 1 h. The solvent was removed *in vacuo* and the mixture purified by RP HPLC. MeOH in the desired HPLC fractions was removed *in vacuo* and the resulting aq. solution neutralized using sat'd sodium bicarbonate solution, then extracted with EtOAc. The organic layer was dried (MgSO₄) and the volatiles were removed *in vacuo.* The solid obtained was dissolved in acetonitrile/MeOH and treated with 1 N HCl in diethyl ether. The mixture was stirred at rt over 1 h and the solvents removed *in vacuo.* The HCl salt of the title compound was obtained as an orange solid (18 mg, 51%).

**TABLE 6**

| **Ex.** | **R**_{**15a**} | **R**_{**15b**} | **Compound Name** | **Data** |
|---|---|---|---|---|
| 50 | F | H | 4-(5-Fluoro-2,3-dihydro-2-oxo-1H-indol-3-yl)-5-methyl-N-[3-(4-morpholinyl)propyl]pyrrolo[2,1-f][1,2,4]triazine-6-carboxamide | MS: (M+H)⁺ = 453. |
| 51 | H | F | 4-(6-Fluoro-2,3-dihydro-2-oxo-1H-indol-3-yl)-5-methyl-N-[3-(4- | - |

| | | | | |
|---|---|---|---|---|
| | | | morpholinyl)propyl]pyrrolo[2,1-f][1,2,4]triazine-6-carboxamide; | |
| 52 | -SO₂NH₂ | H | 4-[5-(Aminosulfinyl)-2,3-dihydro-2-oxo-1H-indol-3-yl]-5-methyl-N-[3-(4-morpholinyl)propyl]pyrrolo[2,1-f][1,2,4]triazine-6-carboxamide | |

### Example 53

### 4-(2,3-Dihydro-2-oxo-1H-indol-3-yl)-5-methylpyrrolo[2,1-f][1,2,4]triazine-6-propanoic acid methyl ester

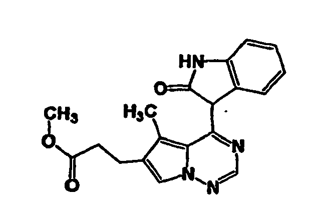

### A. 4-Phenoxy-5-methyl-6-carbomethoxypyrrolo[2,1-f][1,2,4]triazine

To a solution of phenol (705 mg, 7.5 mmol) in a mixture of THF (10 mL) and DMF (10 mL) was added NaH (60% in oil, 300 mg, 7.5 mmol). After 30 min, 4-chloro-5-methyl-6-carbomethoxypyrrolo[2,1-f][1,2,4]triazine (675 mg, 3.0 mmol - Compound E from Example 2) was added. After 1 h, the solvent was removed and the residue poured into 5% aq. K₂CO₃ solution. The precipitate was collected, washed with water, and dried *in vacuo* to afford Compound A as white solid (800 mg, 94%). MS: (M+M)⁺ = 284.

### B. 4-Phenoxy-5-methyl-6-hydroxymethylpyrrolo[2,1-f][1,2,4]triazine

To a solution of Compound A (700 mg, 2.47 mmol) in toluene (20 mL) at -60 °C, was added DIBAL (1.5 M in toluene, 6 mmol). After stirring at 0°C for 1 h, aq. 1N HCl (30 mL) was added and the mixture stirred for 30 min. The mixture was then diluted with DCM. The organic layer was separated, dried (MgSO₄), and concentrated. The residue was purified by flash column chromatography (silica gel, 2 % MeOH in DCM) to afford Compound B as a solid (610 mg, 96%). MS: (M+H)⁺= 256.

### C. 4-Phenoxy-5-methylpyrrolo[2,1-f] [1,2,4]triazine-6-carboxaldehyde

A mixture of Compound B (500 mg, 1.96 mmol) and MnO₂ (3.0 g) in toluene (30 mL) was heated at 60°C for 1h. After cooling to rt, the mixture was filtered through a pad of silica gel and washed with EtOAc. After concentration *in vacuo,* Compound C was obtained as a white solid (420 mg, 85%). MS: (M+H)⁺ = 254

### D. 4-Phenoxy-5-methylpyrrolo[2,1-f][1,2,4]triazine-6-propenoic acid methyl ester.

DBU (1.42 mL, 9.49 mmol) was added to a solution of Compound C (600 mg, 2.37 mmol) and methyl diethylphosphonoacetate (1.74 mL, 9.49 mmol) in DCE (20 mL). After stirring at rt overnight, the reaction mixture was diluted with DCM and washed with aq. 2% citric acid, brine, dried (MgSO₄), and concentrated. The organic extract was concentrated and the residue purified by chromatography on silica gel and elution with 20% EtOAc/DCM to afford a white solid (710 mg. 97%). MS: (M+H)⁺ = 310.

### E. 4-Hydroxy-5-methylpyrrolo[2,1-f][1,2,4]triazine-6-propanoic acid methyl ester.

Pd/C (10%, 70 mg) was added to a solution of Compound D (710 mg, 2.30 mmol) in a solvent mixture EtOAc/MeOH/THF/AcOH (100 mL/100 mL/20 mL/2 mL). The suspension was stirred under hydrogen for 2h. The reaction mixture was passed through Celite; the Celite was washed with MeOH, and the filtrate was concentrated *in vacuo* to give crude product. Trituration with hexanes afforded Compound E as a white solid (430 mg, 88%). MS:(M+H)⁺ = 236.

### F. 4-Chloro-5-methylpyrrolo[2,1-f][1,2,4]triaxine-6-propanoic acid methyl ester.

A mixture of DIPEA (0.24 mL, 1.4 mmol), Compound E (220 mg, 0.94 mmol) and POCl₃ (3 mL) was heated in a sealed bottle at 80°C. After 2 h, the mixture was cooled down to rt and concentrated *in vacuo* to give a residue. The residue was partitioned between DCM and aq. NaHCO₃ solution. The DCM layer was separated, dried (MgSO₄), and concentrated *in vacuo* to give a dark green solid. Purification by chromatography on silica gel and elution with 20% EtOAc/DCM afforded Compound F as a yellow solid (220 mg, 92%). MS: (M+H)⁺ = 254.

### G. 4-(2,3-Dihydro-2-oxo-1H-indol-3-yl)-5-methylpyrrolo[2,1-f][1,2,4]triazine-6-propanoic acid methyl ester.

NaH (60% in oil, 28 mg, 0.71 mmol) was added to a solution of oxindole (94 mg, 0.71 mmol) in DMF (2 mL) under argon. The mixture was stirred for 10 min. and Compound F (60 mg, 0.24 mmol) was added. After 1 h at RT, the reaction was quenched by the addition of acetic acid and diluted with DCM. The organic solution was washed with water, dried (MgSO₄), and concentrated *in vacuo* to give crude product. Purification by chromatography on silica gel and elution with 20% EtOAc/DCM afforded the title compound as a pure yellow solid (78 mg, 94%). MS: (M+H)⁺ = 351.

### Example 54

### 1,3-Dihydro-3-[5-methoxy-6-(phenylmethoxy)pyrrolo[2,1-f][1,2,4]triazin-4-yl]-2H-indol-2-one

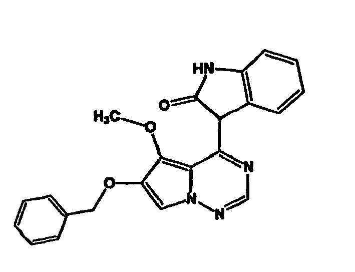

### A. 4-Hydroxy-5-methoxypyrrolo[2,1-f] [1,2,4]triazine-6-methanol

4-Hydroxy-5-methoxypyrrolo[2,1-f][1,2,4]triazine-6-carboxylic acid ethyl ester (Compound F of Example 27 -3.56 g, 15 mmol) was combined with lithium tri-*tert*-butoxyaluminohydride (1M solution in THF, 60 mL, 60 mmol) and heated at reflux overnight. The reaction mixture was allowed to cool to rt and quenched with 1 N aq. HCI. The mixture was concentrated to remove volatiles and the remaining material was combined with 100 g of silica gel and applied to a flash silica gel column which was eluted with EtOAc to provide 2.65 g (90%) of Compound A. MS: [[M+H]⁺= 196.

### B. 2,2-Dimethylpropanoic acid [6-(hydroxymethyl)-5-methoxy-4-oxopyrrolo[2,1-f][1,2,4]triazin-3(4H)-yl]methyl ester

Compound A (195 mg, 1 mmol) was dissolved in 1.5 mL of DMF. NaH (60% in oil, 48 mg, 1.2 mmol) was added and the reaction mixture was stirred at rt for 0.5 hr. Chloromethyl pivalate (181 mg, 1.2 mmol) was added and the mixture was stirred for 1 hr. Water was added and the mixture was extracted with EtOAc (3 x 10 mL). The combined extracts were dried (Na₂SO₄), concentrated *in vacuo* and purified by flash column chromatography on silica gel eluting with 33% EtOAc in hexanes to provide 260 mg (84%) of Compound B as a solid. MS: [M+H]⁺= 310.

### C. 2,2-Dimethylpropanoic acid [6-formyl-5-methoxy-4-oxopyrrolo[2,1-f][1,2,4]triazin-3(4H)-yl]methyl ester

Compound B (740 mg, 2.39 mmol) was suspended in toluene (10 mL) with manganese dioxide (835 mg, 9.6 mmol) and heated at 100°C for 3 hr. The reaction mixture was cooled to rt, filtered, and the precipitate was washed with EtOAc. The filtrate was concentrated *in vacuo* to provide 660 mg (90%) of Compound C as a solid. MS: [M+H]⁺= 308.

### D. 2,2-Dimethylpropanoic acid [6-formyloxy-5-methoxy-4-oxopyrrolo[2,1-f][1,2,4]triazin-3(4H)-yl]methyl ester

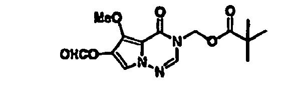

Compound C (660 mg, 2.15 mmol) was dissolved in CH₂Cl₂ (10 mL). M-chloroperoxybenzoic acid (57%, 745 mg, 2.46 mmol) was added with MgSO₄ (2.0 g), and the reaction mixture was stirred at rt for 5 hr. The mixture was filtered and the filtrate washed with aq. NaHCO₃ solution twice, dried (MgSO₄), and concentrated to provide 680 mg (98%) of Compound D as a solid. MS: [M+H]⁺= 324.

### E. 2,2-Dimethylpropanoic acid [5-methoxy-4-oxo-6-(phenylmethoxy}pyrrolo[2,1-f][1,2,4]triazin 3(4H)-yl]methyl ester

Compound D (680 mg, 2.10 mmol, 1 eq) was dissolved in acetone (10 mL) followed by the addition of benzyl bromide (430 mg, 2.5 mmol) and K₂CO₃ (1.0 g, 7.25 mmol). The reaction mixture was stirred at 60°C for 10 hr, cooled to rt, and filtered. The filtrate was concentrated and purified by flash silica gel chromatography eluting with 25% EtOAc in hexanes to provide 485 mg (60%) of Compound E as a gel. MS: [M+H]⁺= 386;

### F. 5-Methoxy-6-(phenylmethoxy)pyrrolo[2,1-f] [1,2,4]triazin-4(3Hone

Compound E (65 mg, 0.17 mmol) was stirred at rt in a mixture of MeOH (1 mL) and ammonium hydroxide (0.2 mL) for 6 hrs. The mixture was concentrated *in vacuo,* dissolved in CH₂Cl₂, and purified by flash silica gel chromatography eluting with 33% EtOAc in hexanes to provide 45 mg (97%) of compound F as a solid. MS: [M+H]⁺=272.

### G. 4-Chloro-5-methoxy-6-(phenylmethoxy)pyrrolo[2,1-f][1,2,4]triazine

Compound F (44 mg, 0.16 mmol) was stirred with POCl₃ (0.5 mL) at 60°C for 3 hr. The mixture was concentrated *in vacuo,* dissolved in CH₂Cl₂ (2 mL), and stirred with solid NaHCO₃ for 10 min. The mixture was filtered and concentrated to provide 46 mg (99%) of Compound G as a solid. MS: [M+H]⁺= 286. (The Cl substituent may be replaced with OCH₃ upon standing in MeOH); R.T.=3.265 min (YMC S5 ODS column 4.6 x 50 mm, 10-90% aq. MeOH over 4 minutes containing 0.2% phosphoric acid, 4 mL/min, monitoring at 220 nm); ¹H NMR (CDCl₃): δ 8.01 (s, 1H), 7.45-7.30 (m, 6 H), 5.15 (s, 2H), 4.03 (s, 3H).

### H. 1,3-Dihydro-3-[5-methoxy-6-(phenylmethoxy)pyrrolo [2,1-f][1,2,4]triazin-4-yl]-2H-indol 2-one

To a suspension of NaH (60% in oil, 19.2 mg, 0.48 mmol) in DMF (0.5 mL) was added oxindole (63.4 mg, 0.48 mmol). The reaction mixture was stirred for 1 hr at RT. Compound G (38 mg, 0.16 mmol, 1 eq) was added, and the mixture was stirred for 1 hr. The mixture was diluted with water and filtered. The resulting solid was triturated with MeOH and dried to provide 38 mg (62%) of the titled compound. MS: [M+H]⁺= 387; ¹H NMR (*d*-DMSO): δ 12.83 (br s, 1H), 10.64 (br s, 1H), 7.78 (s, 1H), 7.60 (s, 1H), 7.50-7.31 (m, 6H), 7.02-6.94 (m, 1H), 6.89-6.82 (m, 2H), 5.10 (s, 2H), 3.55 (s, 3H).

### Examples 55-60

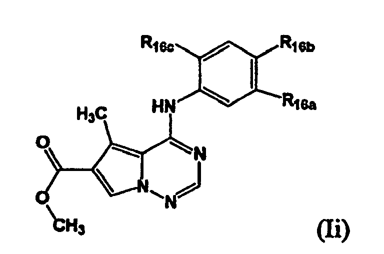

Compounds having the formula (Ii), wherein R₁₆ₐ, R_{16b}, and R_{16c} have the values listed in Table 7, below, were prepared following the procedure described for Example 54, except using a different phenylamine.

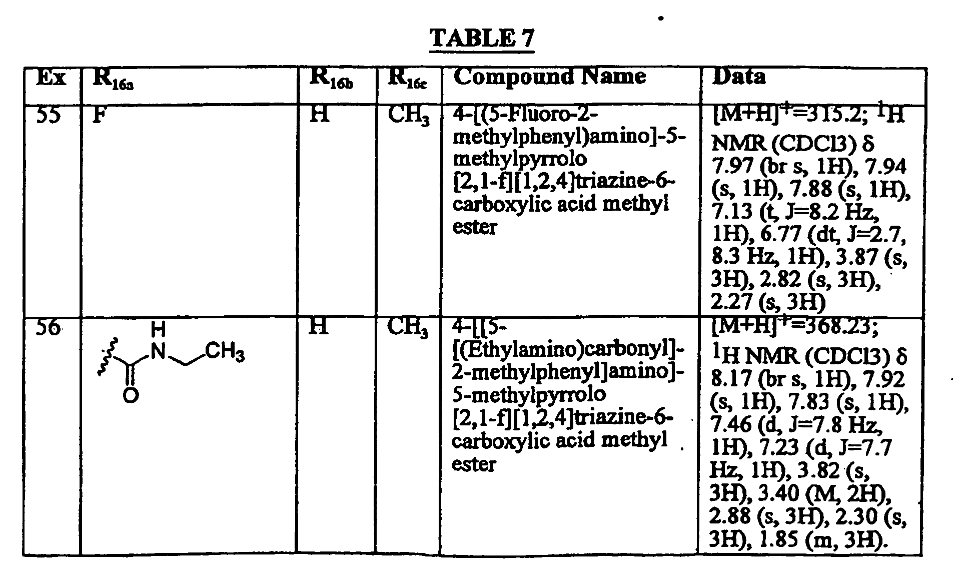

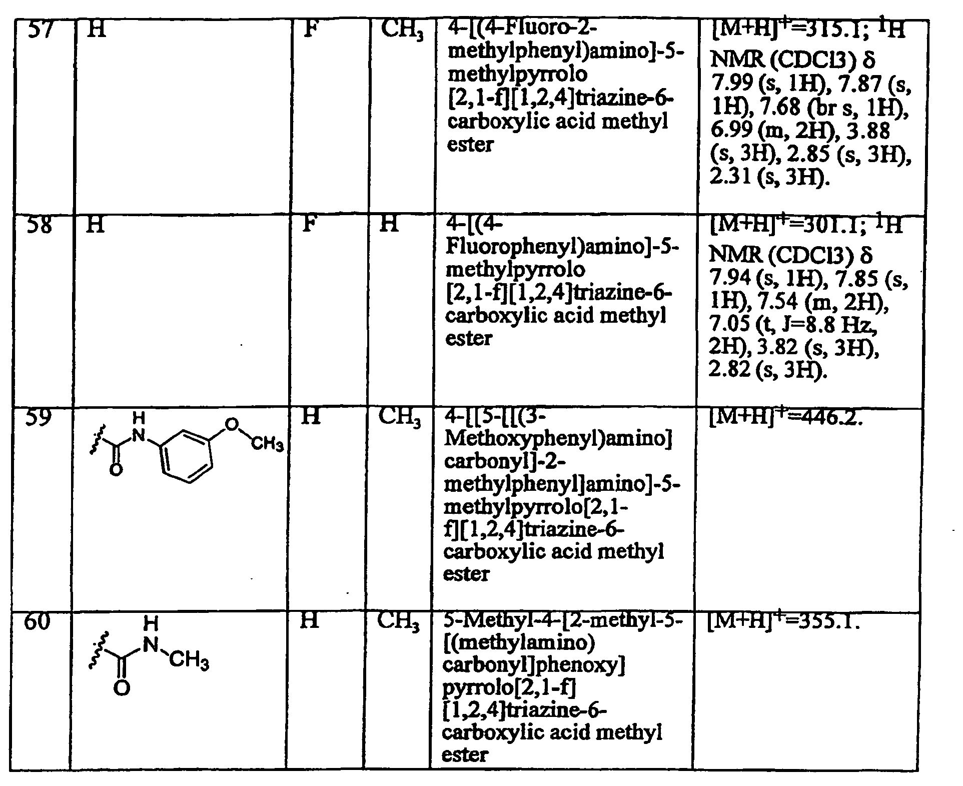

### Example 61

### N-Cyclobutyl-4-[[5-[(methoxyamino)carbonyl-2-methylphenyl]amino]-5-methylpyrrolo[2,1-f] [1,2,4]triazine-6-carboxamide

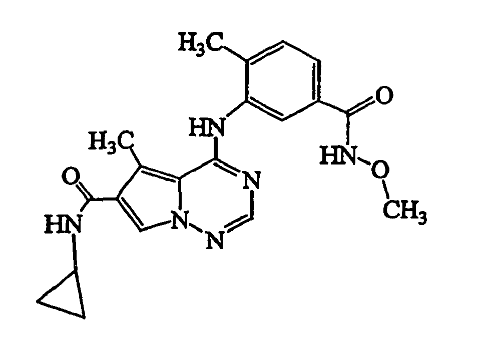

The compound of Example 61 was prepared as set forth below, using the Scheme 9 described above, wherein Compounds (A)-(E) have the structures indicated below.
A. 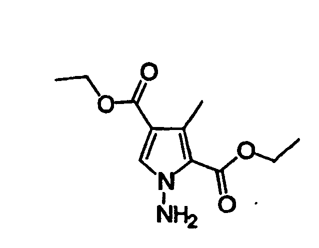
   To a solution of the 3-methyl-1-pyrrole-2,4-diethyl ester (100 mg) (J. Heterocyclic Chem Vol. 34 (1997), at pp. 177-193; Heterocycles, Vol. 50 (1999), at pp. 853-866; Synthesis (1999), at pp. 479-482) in DMF (0.44M) was added either NaH or KOtBu (1.2 equiv) at rt. This solution was stirred for 30-45 minutes. Chloramine in ether (ca. 0.15M, 1 eq.) was added via syringe. The solution was stirred for 1.5 h or until starting material was converted to product as judged by HPLC analysis. The reaction was then quenched with aq. Na₂S₂O₃ and extracted with EtOAc or Et₂O. The organic extracts were washed with water and brine and then dried over sodium sulfate. Compound A was obtained in >90% yield. NH₂Cl in ether was prepared according to the procedure of Nunn, J. Chem. Soc. (C), (1971) at p. 823.
B. 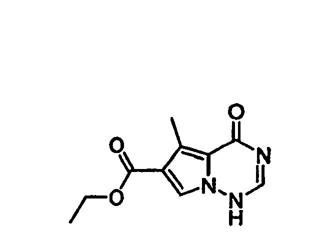
   To a solution of Compound A (2 g) in formamide (8 mL) was added acetic acid (20% by weight), and the mixture was heated at 120°C for 24h. The reaction mixture was cooled and water added (32 mL) to precipitate the product The solids were collected by filtration and washed with EtOAc to furnish Compound B as a yellow solid (90%).
C. 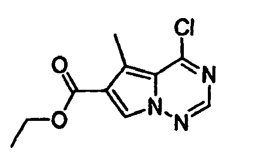
   To a solution of Compound B (10 g, 45.2 mmol) in toluene (150 mL) was added DIPEA (6.31 mL, 36.2 mmol, 0.8 equiv) and POCl₃ (5.05 mL, 54.2 mmol, 1.2 equiv) and the reaction mixture heated at 120-125 °C (oil bath temp) for 20 h. The reaction mixture was cooled and poured into ice cold sat. NaHCO₃-water-toluene (450 mL-450 mL-150 mL) and stirred rapidly to assure quenching of the excess POCl₃. The layers were separated (filtered through celite if a suspension forms) and the organic layer was washed again with sat. NaHCO₃. The organic layer was dried over Na₂SO₄, filtered and concentrated *in vacuo* to afford Compound C as a tan yellow solid (9.9g, 95%).
D. 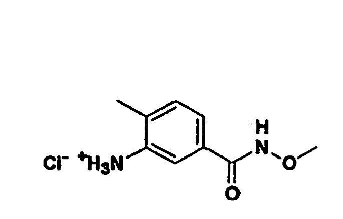
   A mixture of commercially-available 4-amino-3-methylbenzoic acid (100 g, 0.66 mol) and N-(*tert*-butoxycarbonyl)anhydride (150 g, 0.68 mol) in THF (1000 mL) was slowly heated to 50°C overnight. The resulting mixture was cooled to rt and the solvent was removed on a rotary evaporator. The resulting solids were triturated with hexanes and dried *in vacuo* to afford 151 g (91%) of the crude BOC-protected aniline intermediate as a light pink solid.
   To the above, light-pink solid was added EDCI (127 g, 0.66 mol), HOBt (90 g, 0.66 mol), and DMF (1000 ml), and the resulting mixture was stirred at rt for 30 minutes followed by addition of methoxyamine hydrochloride (55 g, 0.66 mol) in one portion. After stirring for 10 min, the mixture was cooled using an ice bath. DIPEA (250 ml, 1.4 mol) was added at such a rate so as to maintain the internal reaction temperature below 25°C. After the addition was complete, the ice bath was removed and the reaction was stirred overnight at rt. The reaction mixture was partitioned between 0.5 L of water and 1.5 L of EtOAc and the resulting layers were separated. The aqueous portion was extracted with additional EtOAc (400 mL x 3), and the combined organic extracts were washed with water (300 mL x 3), cold 0.5 N aq. HCl (400 mL x 2), and water (500 mL). The product was then extracted with cold 0.5 N aq. NaOH (300 mL x 3) and the combined basic aqueous extracts were neutralized to pH = 8 by a slow addition of cold 0.5 N aq. HCl. The resulting solid which precipitated was collected by filtration and washed with cold water. The wet solid was decolorized in hot EtOH with active charcoal to give 106 g of white solid as the BOC-protected N-methoxyamide intermediate.
   To a slurry of the above solid (91 g, 0.32 mol) in 1,4-dioxane (400 mL) at rt was added a 4M solution of HCl in dioxane (400 mL), and the resulting mixture was stirred at rt overnight. Diethyl ether (1000 mL) was added and the precipitated solid was collected by filtration and triturated with a hot EtOH/H₂O mixture (4:1 v/v). Drying the resulting solid *in vacuo* afforded 53 g of the pure hydrochloride salt (Compound D) as a white solid. ₁H NMR (d₆-DMSO): δ 9.5-9.9 (br. s, 1H), 7.75 (s, 1H), 7.55 (d, 1H), 7.36 (d, 1H), 3.70 (s, 3H), 2.38 (s, 3H).
E.

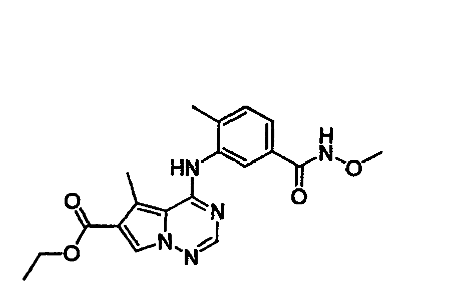

To a solution of the Compound D (41.2 g, 190 mmol) in DMF (230 mL) was added DIPEA (33.1 mL, 180.7 mmol, 0.95 equiv), and the reaction vessel was heated to 55 °C (oil bath temp). Solid Compound C (45.6 g, 190 mmol) was added in several portions over 10 minutes and the flask was rinsed with DMF (150 mL) and added to the reaction. The reaction was heated for 10 hours at 55 °C and cooled to rt. The mixture was then poured into 1.5 L water diluted to 2.2 L with ice slowly over 10 minutes. The pH was adjusted to 6 and the solids were stirred for 1 h. The solids were filtered, washed with water (2x200 mL) and dried on the filter to give 71.9 .g crude ester. The solid was then suspended in acetonitrile (450 mL) and heated with stirring at 50°C for 1 h. The mixture was cooled and filtered to give 64.2 g product (>99% purity). These solids were then dissolved in hot EtOH (2.8 L) and decolorizing carbon (6.4 g) was added followed by heating at reflux for 15 min. The mixture was then filtered through a pad of celite and the reaction flask rinsed with hot EtOH (1 L). The hot filtrate was then concentrated to ~1 L of EtOH by distillation upon which the product started to crystallize out of solution at a volume of 2.5 L. The solution was cooled and placed in a cold room with stirring for 40 h. The solids were filtered and rinsed with 1/1 EtOH/Et₂O (500 mL) to give 58.5 g of Compound E as a white solid (80%).

### F. Example 61

To a solution of ester Compound E (22.5 g, 58.7 mmol) in THF (205 mL) was added 1 N NaOH (205 mL) and the reaction mixture heated to 50°C for 16 h. The THF was removed *in vacuo* and the mixture was acidified to pH 4-5 with 1N aq. HCl to precipitate the product. The heterogeneous mixture was stirred for 1 h, filtered and washed with water (150 mL) and ether (150 mL). The collected solids were partially dried on the filter to give the crude acid intermediate as a moist white solid which was used without further purification.

To a solution of the moist acid in 300 mL of DMF was added HOBt (11.9 g, 88.0 mmol), EDCI (16.9 g, 88.0 mmol) and 1.3 equivalents (117 mmol) of cyclopropyl-amine as the free base or as the hydrochloride salt. The mixture was stirred for 30 min to solubilize the solids, placed in a cold water bath, and DIPEA (20.4 mL, 117 mmol) was added slowly via syringe. The reaction mixture was allowed to stir at rt for 1 h, then poured into rapidly stirred ice water (1.2 L) to precipitate the product. After stirring for 3 h, the solids were collected by suction filtration, washed with water (150 mL) and ether (2x100mL), and allowed to air dry by suction filtration to give Example 61 (92-98%) as a white solid.

### Examples 62-115

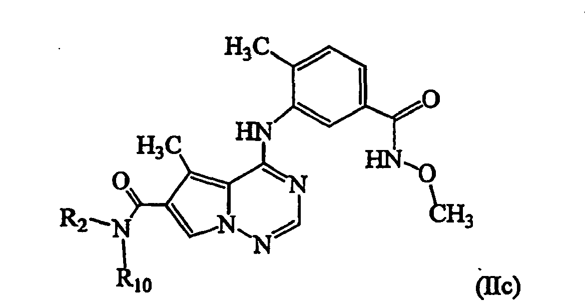

Compounds having the formula (IIc), wherein R₂ and R₁₀ have the values listed in Table 8 were prepared following the same methods set forth above in Scheme 9 and Example 61, using different amines (NR₂R₁₀) in the last step. Additionally, each compound can be recrystallized using a 7 to 1 EtOH/water mixture to afford analytically pure product as a white crystalline solid.

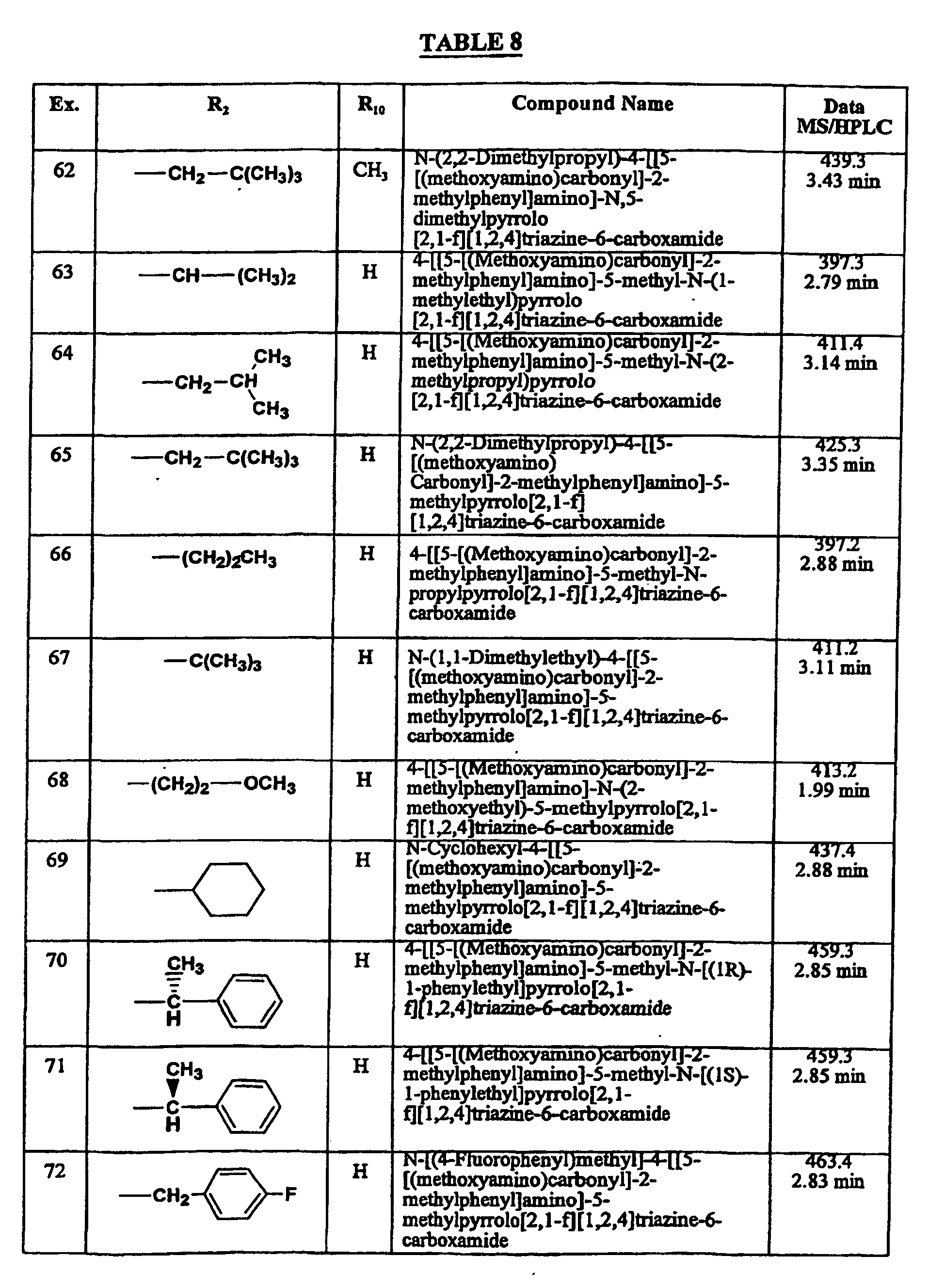

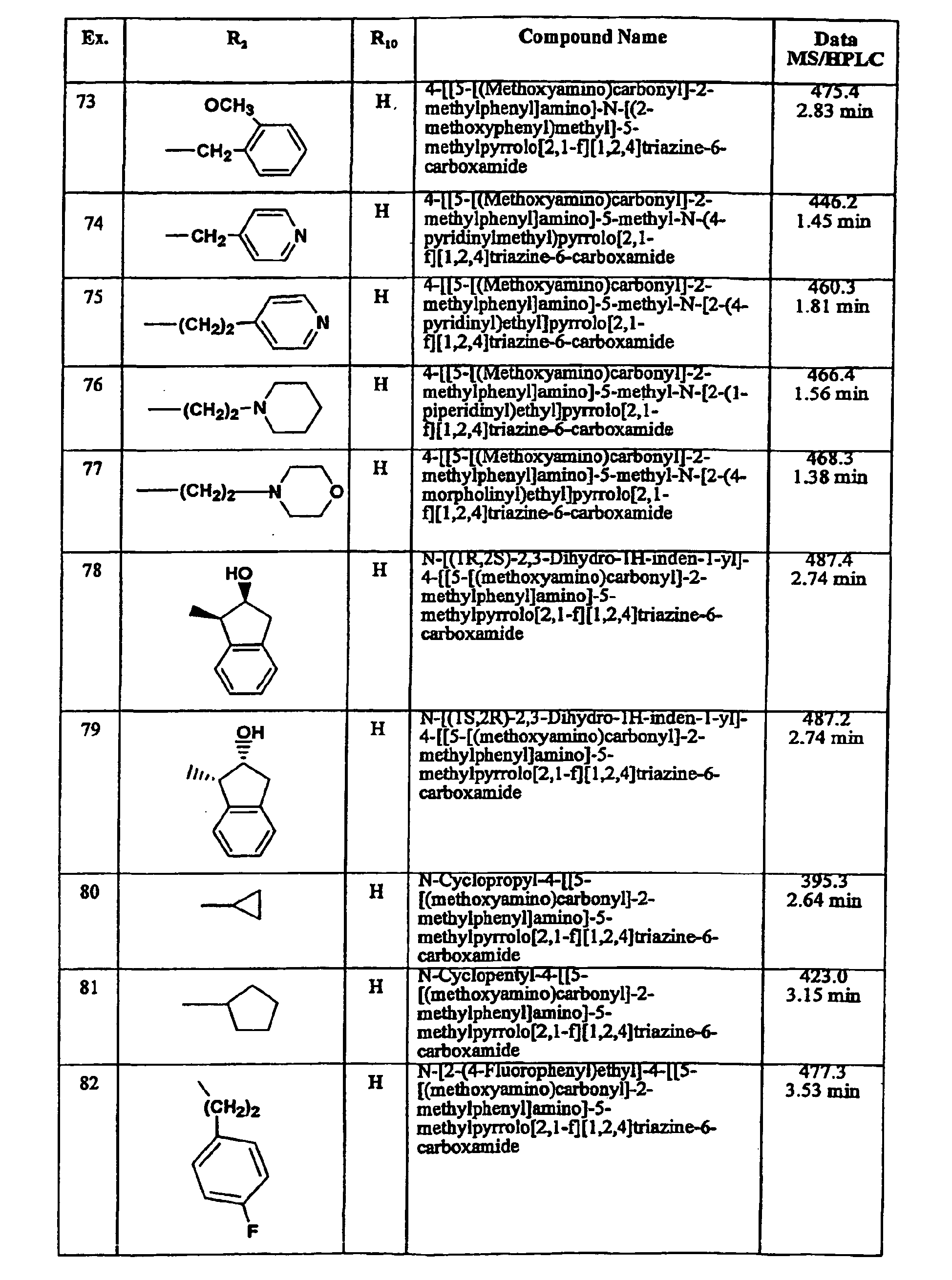

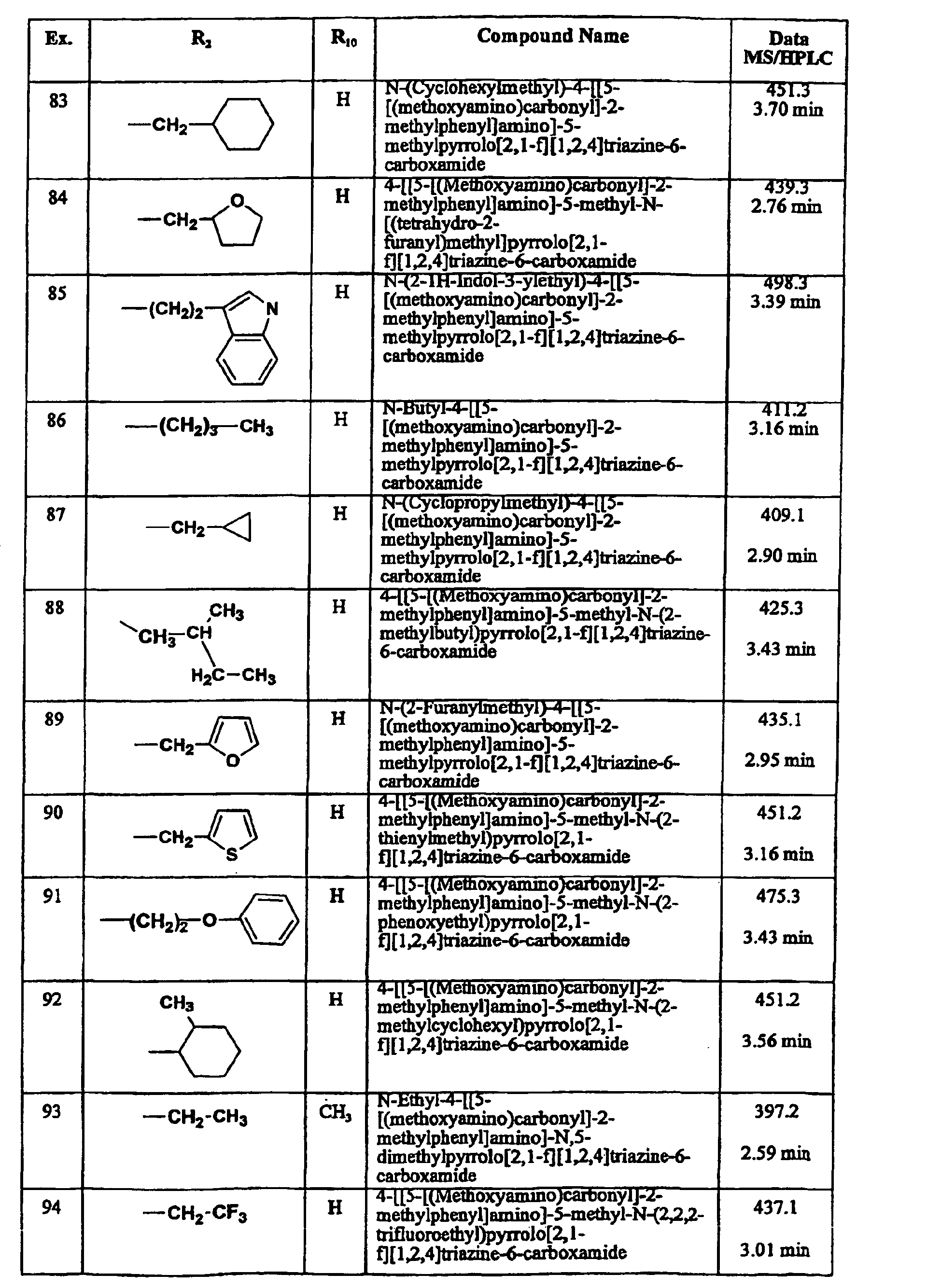

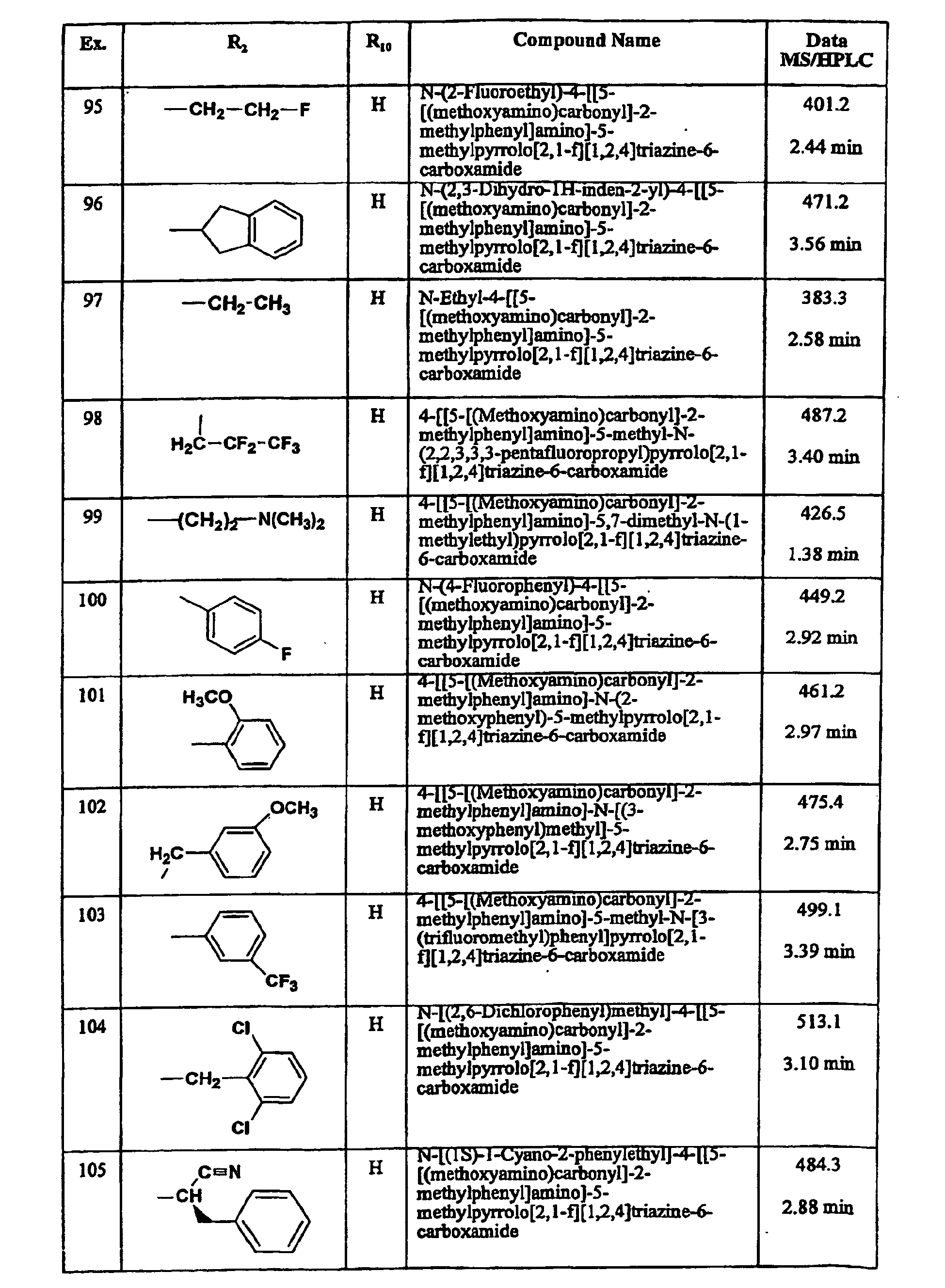

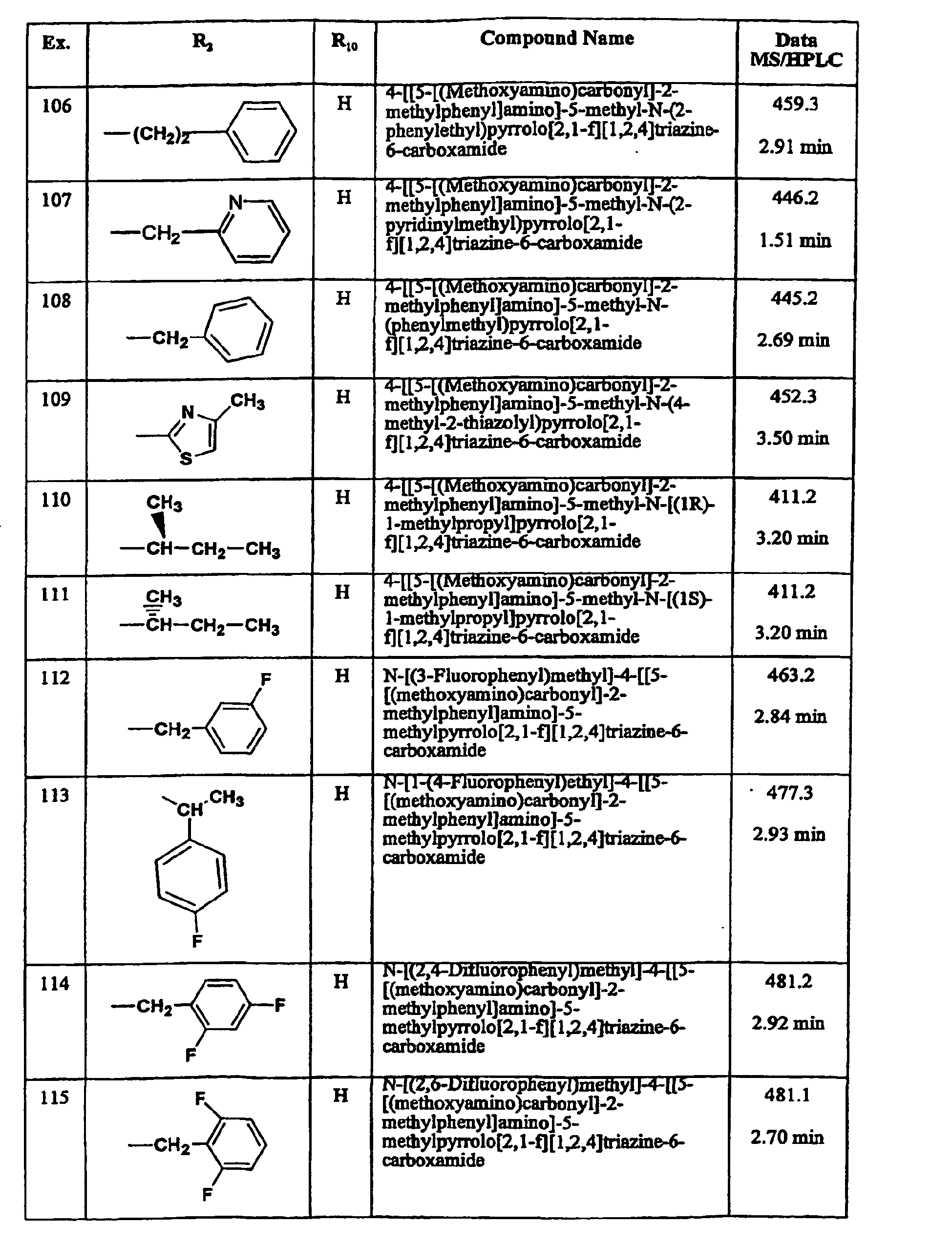

### Examples 116-119

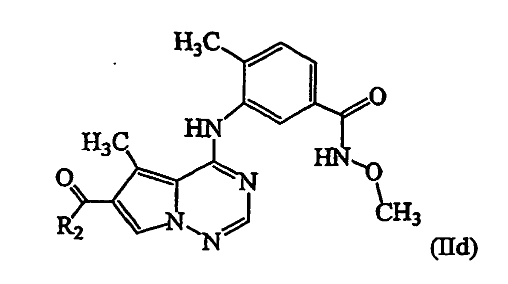

Compounds having the formula (IId), wherein the R₂ groups have the values listed in Table 9, were prepared following the same methods set forth above in Scheme 9 and Examples 62-115.

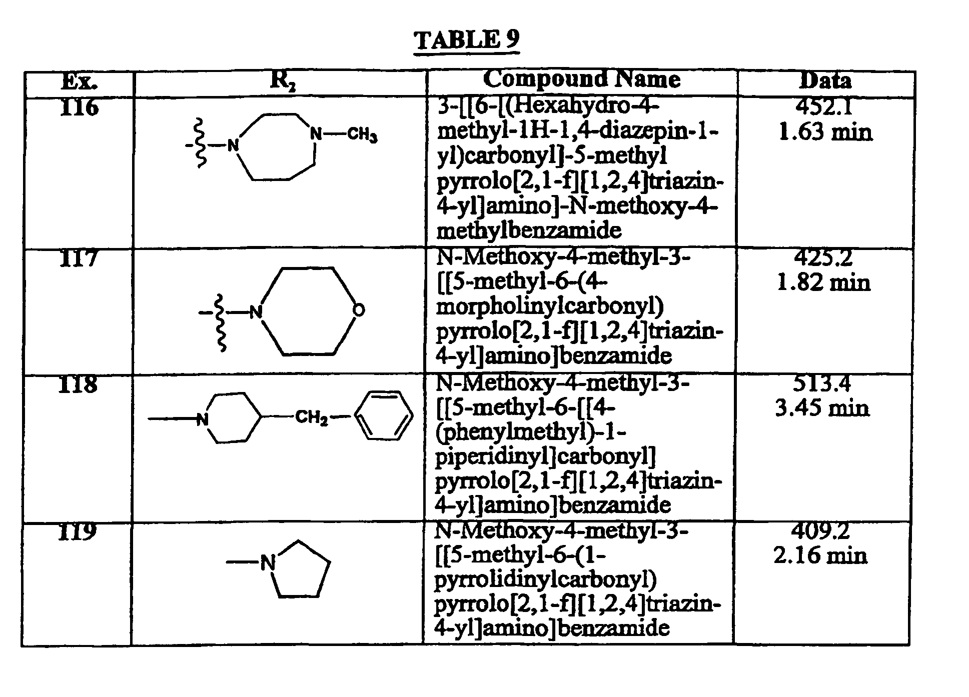

### Examples 120-124

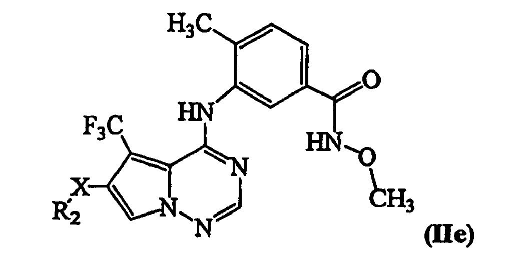

Compounds having the formula (IIe), wherein X and R₂ have the values listed in Table 10, were prepared following the same or similar procedure as in Scheme 9 and Example 61, except in the first step, commercially available 3-trifluoromethyl-1-pyrrole-2,4-diethyl ester was used instead of 3-methyl-1-pyrrole-2,4-diethyl ester.

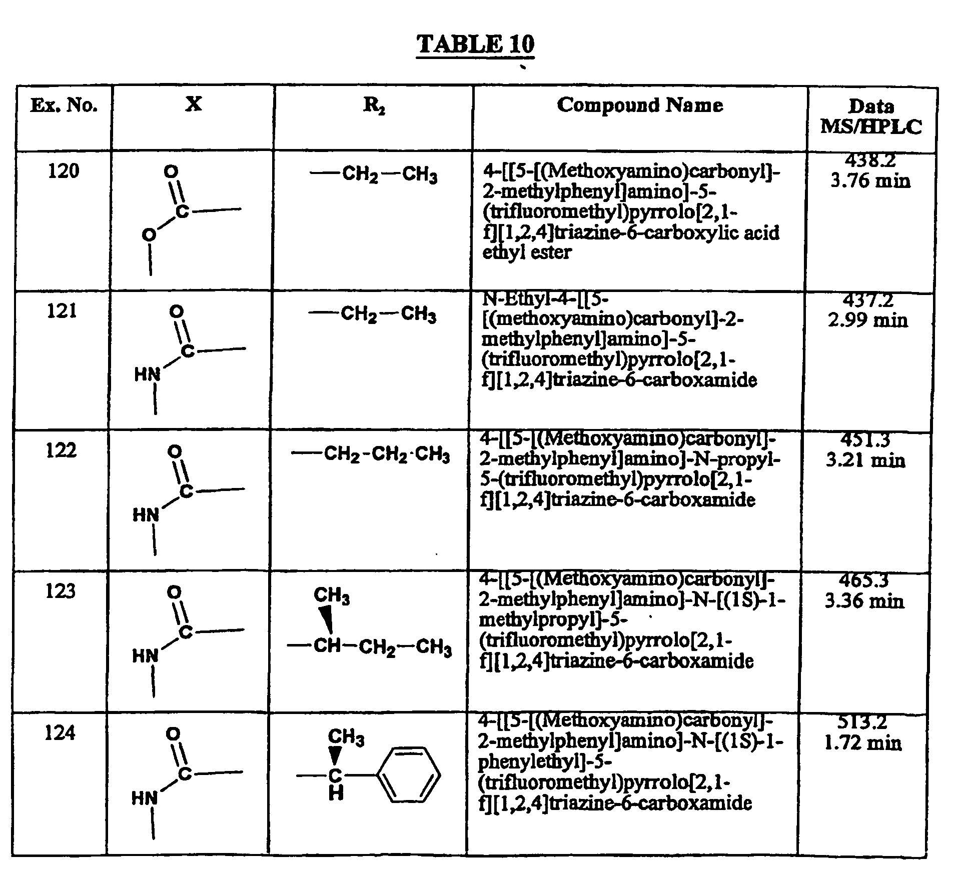

### Example 125

### N-Ethyl-4-[[5-[(methoxyamino)carbonyl]-2-methylphenyl]amino]-5-methylpyrrolo[2,1-f][1,2,4]triazine-6-carboxamide methane sulfonic acid

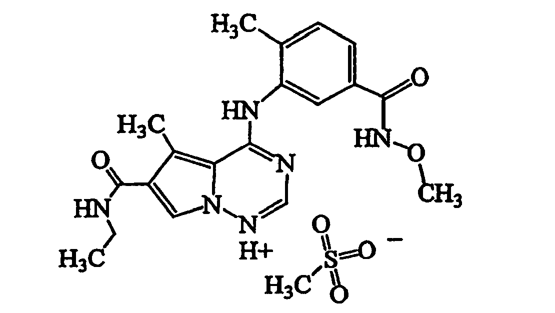

Example 97 as a free base was charged with acetone (10 ml/g Ex. 106), and the jacket was heated to 50-60°C. Reflux was started at 55-57°C, and the mixture was stirred for 30 min. at 50-60°C. Methanesulfonic acid (1.2 eq.) was added, and a slight exotherm was observed. The slurry was stirred at 50-60°C until DSC showed in two consecutive samples the complete conversion of the free base (mp 220-222°C) to the mesylate salt (mp 259-261°C). The slurry was cooled to 20-25°C over about 30 min, and then stirred for at least 30 min. with the temp. kept at 20-25°C. The slurry was then filtered, washed with acetone, and dried *in vacuo* at 40-50°C to an LOD <0.5% to provide Example 125 as a white crystalline solid (yield 90-95%). [M+H]⁺=478.4. The above procedure may be used to prepare mesylate salts of other compounds of Formulae (I) and (II) herein.

### Example 126

### N-Ethyl-5-methyl-4-[[2-methyl-5-[[[3-(trifluoromethyl)phenyl]amino] carbonyl]phenyl]amino]pyrrolo[2,1-f][1,2,4]triazine-6-carboxamide

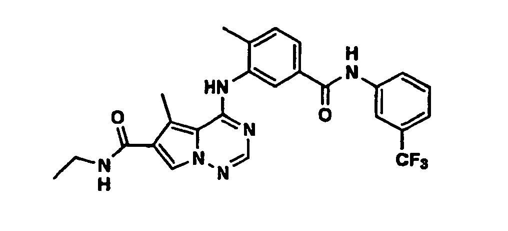

A. To a slurry of 2.0 g (4.2 mmol) of compound (1) from Scheme 10, wherein R₂ is ethyl, in 12 mL of anhydrous MeOH was added 18 mL of a 4 N solution of anhydrous HCl in dioxane at rt. The resulting clear solution was stirred at rt for 16 h and the reaction mixture was concentrated *in vacuo.* The resulting oil was dissolved in 16 mL of 1.5 N aq. KOH solution and heated to 50°C for 3 h. After cooling to rt, the mixture was diluted with 50 mL of water and 10% aq. HCl was added until pH was approximately 3 or 4. The resulting precipitated product was collected by vacuum filtration and washed with 50 mL of water and dried *in vacuo* to afford 1.47 g (99%) of compound (3) from Scheme 10. An analytical sample of this product was prepared by recrystallization from 10% aq. acetonitrile. ¹H NMR (CD₃OD): δ 8.21 (br s, 1H), 8.11 (br s, 1H), 7.89-7.91 (m, 2H), 7.67 (br s, 1H), 7.44 (d, 1H), 3.40 (q, 2H), 2.86 (s, 3H), 2.36 (s, 3H), 1.25 (s, 3H). LCMS (M+H⁺) = 354.2. HPLC (Condition A): 2.24 min.
B. A mixture of Compound A (40 mg, 0.11 mmol), HATU (65 mg, 0.17 mmol), diisopropylamine (20 µL, 0.11 mmol), and 3-trifluoromethylaniline (36 mg, 0.22 mmol) in 0.3 mL of N-methylpyrrolidinone was heated at 80°C for 16 h, and the reaction mixture was purified by RP preparative HPLC to afford 41 mg (74%) of Example 126 as a light tan solid. ¹H NMR (CD₃OD w/ TFA): δ 8.28 (s, 1H), 8.19 (s, 1H), 8.16 (d, 1H), 8.11 (d, 1H), 7.84 (s, 1H), 7.71 (d, 1H), 7.58 (t, 2H), 7.47 (d, 1H), 3.44 (q, 2H), 2.94 (s, 3H), 2.47 (s, 3H), 1.26 (t, 3H). LCMS (M+H⁺) = 497.47. HPLC (Condition A) : 3.30 min.

### Examples 127-129

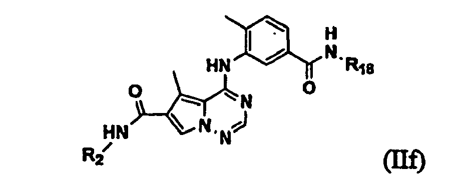

Compounds of Formula (IIf) were prepared as described for Example 126, except appropriate substrates and amines were selected to afford compounds where R₂ and R₁₈ have the values listed in Table 11.

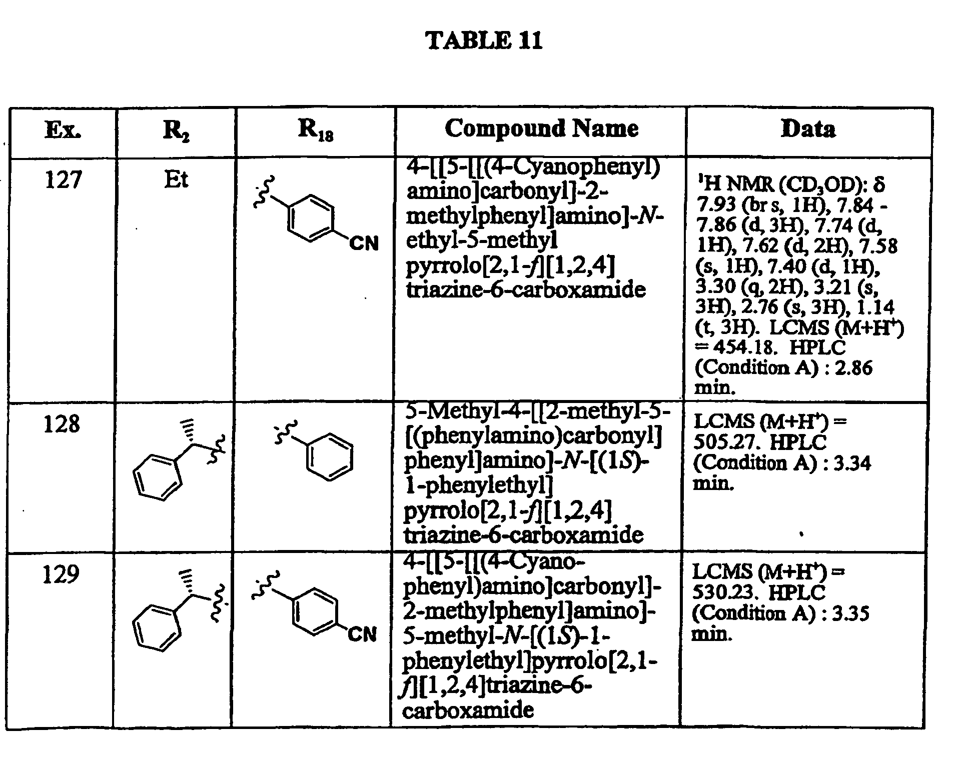

### Example 130

### N-Ethyl-4-[[5-[[(3-fluomphenyl)sulfonyl]amino] 2-methylphenyl)amino]-5-methylpyrrolo[2,1-f][1,2,4]triazine-6-carboxamide

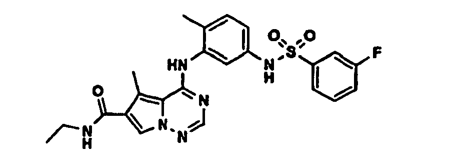

**A.** 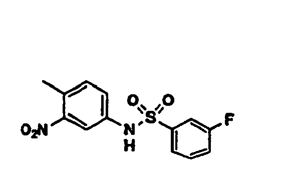
   To a solution of 4-methyl-3-nitroaniline [compound (11) from Scheme 11)(3.72 g, 24.5 mmol)] in 150 ml of DCM at rt was added 3-fluorobenzenesulfonyl chloride (5.00 g, 25.7 mmol), followed by TEA (7.0 ml, 50.2 mmol) via syringe. The resulting mixture was stirred for 20 h and the solvent removed *in vacuo.* The residue was dissolved in DCM (600 ml), washed with sat'd aq. NaHCO₃, dried over sodium sulfate, filtered, and concentrated *in vacuo* to give 8.00 g of dark solid which was triturated with DCM to afford 5.46 g of yellow solid. A portion of this solid (1.63g) was dissolved in 10 ml 1N aq. NaOH and 20 ml THF, and the solution was stirred at rt for 20 h. The solvent was removed *in vacuo* and the residue acidified with 3N HCl to a pH of 2. The resulting precipitated solid was collected by filtration to afford 1.02 g (94%) of a light yellow solid as the desired Compound A. HPLC (Condition A) = 2.99 min. ¹HNMR(CDCl₃) δ 7.67 (d, 1H), 7.59 (dd, 1H), 7.49 (m, 2H), 7.32 (m, 1H), 7.28 (m, 2H), 2.54 (s, 3H).
B. 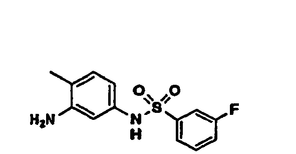
   To 0.20 g (0.64 mmol) of Compound A in MeOH (10 ml) was added 10% Pd/C (20 mg) and the mixture stirred under hydrogen balloon for 6 h at rt. The solution was filtered through a pad of celite and the solvent removed *in vacuo* to give 0.18 g (100%) of Compound B as a colorless, glassy solid. HPLC (Conditions A): 1.77 min. LCMS M+H⁺ (m/z) 281.
C. 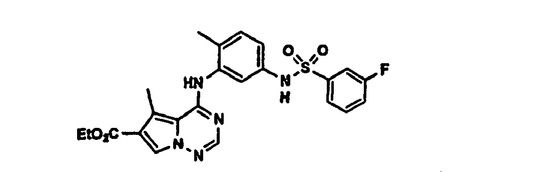
   Compound B (0.18 g, 0.64 mmol) and 0.15 g (0.64 mmol) of 4-chloro-5-methylpyrrolotriazine-6-ethylcarboxylate (compound 8 of Scheme 11) in anhydrous DMF was stirred at rt for 20 h. The reaction was quenched with addition of cold water and sat'd aq. NaHCO₃. The solid was collected, washed with water, and dried *in vacuo* to give 0.27 g (91%) of Compound C as a light yellow solid. HPLC (Condition A: 3.49 min. LCMS M+H⁺ (m/z) 484.
   D. 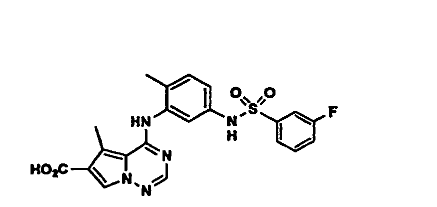
   A solution of 0.27 g (56 mmol) of Compound C in 1 ml of 1N aq. NaOH and 3 ml of MeOH was heated at 60°C for 12 hr. The MeOH was removed *in vacuo* and the aqueous portion acidified with 1N aq. hydrogen chloride to pH ~ 2. The resulting precipitated solid was collected, washed with water, and dried *in vacuo* to afford 0.25 g (98%) of Compound D as a pale yellow solid. HPLC (Condition A): 2.93 min. LCMS M+H⁺ (m/z) 456.

### E. Example 130

A mixture of 30 mg (66 µmol) of Compound D, EDCI (19 mg, 98 µmol), HOBt (13 mg, 98 µmol) and Hunig's base (43 µL, 0.25 mmol) was stirred at rt for 0.5 hr. Ethylamine hydrochloride (10 mg, 0.13 mmol) was added and the mixture stirred for 16 hr. The crude mixture was purified by RP preparative HPLC chromatography to give Example 130. HPLC (Conditions A): 2.83 min. LCMS M+H⁺(m/z) 483.

### Examples 131-132

Examples 131 and 132 as shown in Table 12 were prepared from Compound D of Example 130 and an appropriate amine as described in Example 130, step E.

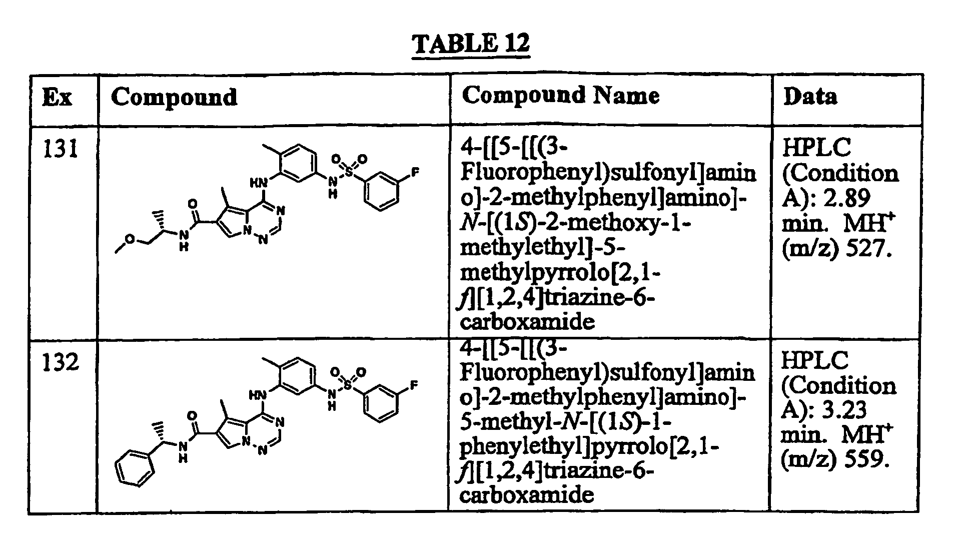

### Examples 133-141

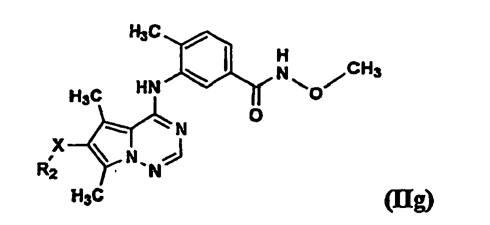

Compounds having the formula (IIg), wherein X and R₂ have the values listed in Table 13 were prepared from commercially-available diethyl-2,4-dimethylpyrrole-3,5-dicarboxylate following the same or similar procedure described above for the preparation of 5-desmethyl pyrrolotriazine.

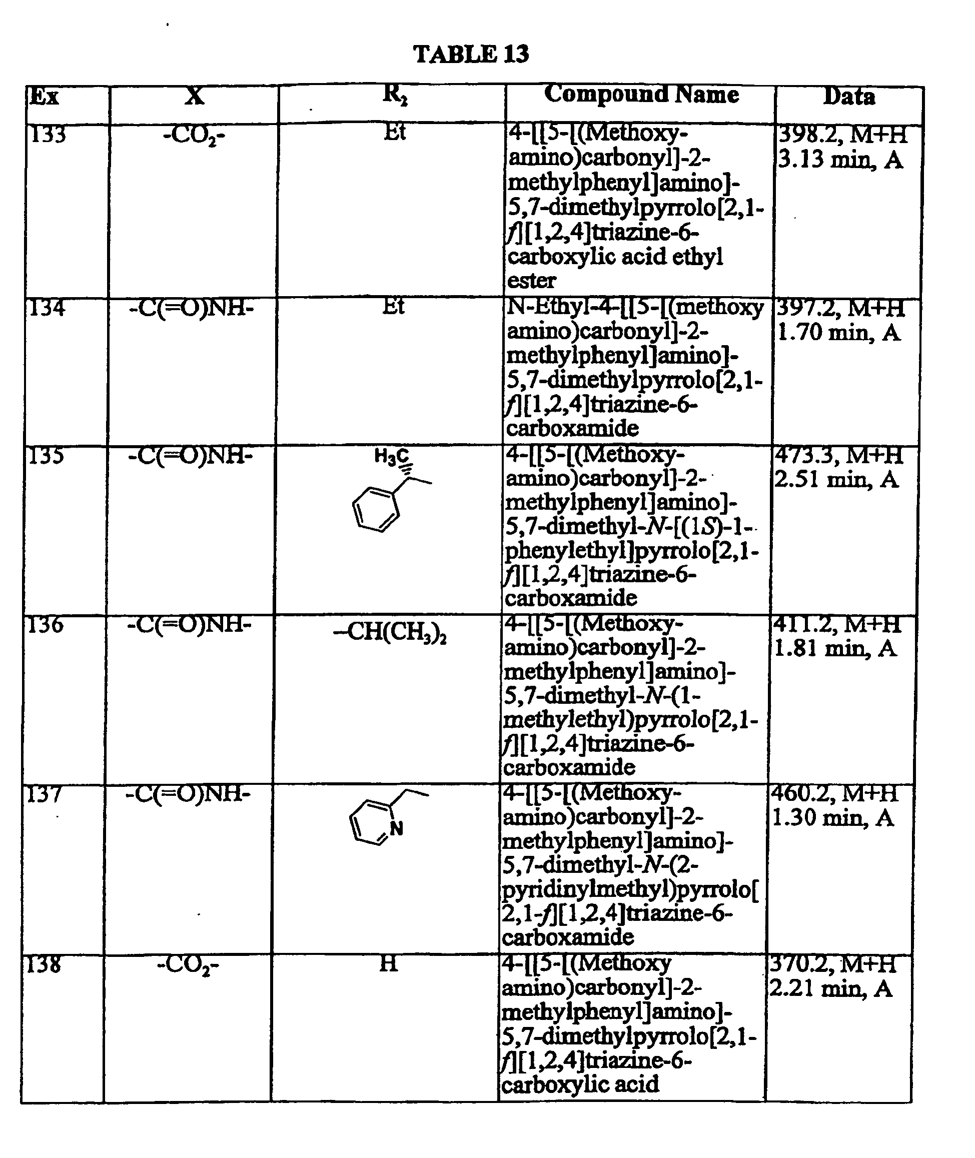

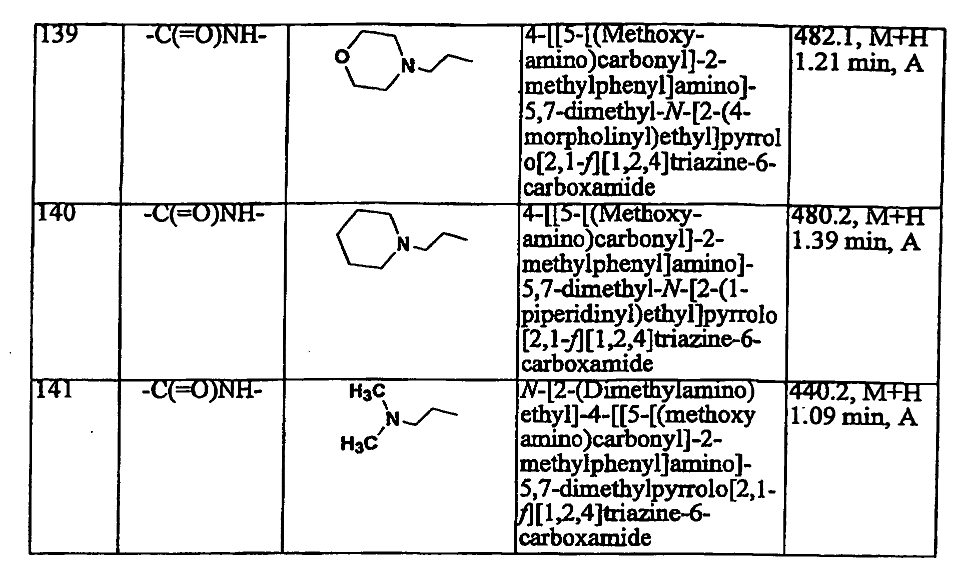

### Example 142

### 4-[(5-[[(Ethylamino)carbonyl]amino] 2-methylphenyl]amino]-5-methyl-N-propyl pyrrolo[2,1-f][1,2,4]triazine-6-carboxamide

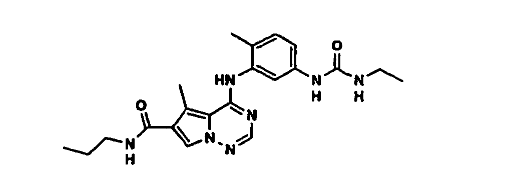

### A. 5-Methyl-4-[(2-methyl-5-nitrophenyl)amino]pyrrolo[2,1-f] [1,2,4]triazine-6-carboxylic acid ethyl ester

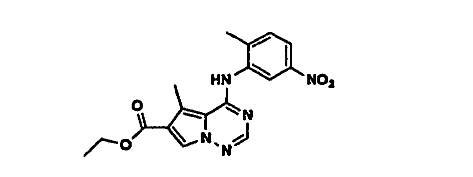

A suspension of chloropyrrolotriazine (compound 1 from Scheme 12) (2.03 g, 8.47 mmol) and 3-nitro-5-methyl aniline (1.41 g, 9.3 mmol) in DMF (25 mL) was stirred at rt for 24 h. Water (125 mL) was added over 30 min and the solution stirred for 1 h upon which the pH was adjusted to neutral with sat. aq. NaHCO₃. The solids were filtered, washed with water, and dried to give compound A (2.589 g, 85% yield) as a pale tan solid.

### B. 5-Methyl-4-[(2-methyl-5-nitrophenyl)amino] pyrrolo [2,1-f] [1,2,4]triazine-6-carboxylic acid

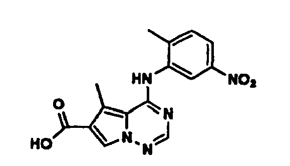

To a solution of Compound A (825 mg, 2.32 mmol) in THF (2 mL) and MeOH (1 mL) was added 1N NaOH (6 mL) and the reaction heated at 60°C for 24 h. The reaction mixture was cooled, concentrated to remove the organic solvents, and the pH was adjusted to neutral with 1 N HCl. The solids were filtered, washed with water, and dried to give compound B. LCMS (M+H⁺) =328.1. HPLC (Condition A): 3.40min.

### C. 5-methyl-4-[(2-methyl-5-nitrophenyl)amino]-N-propylpyrrolo [2,1-f] [1,2,4]triazine-6-carboxamide

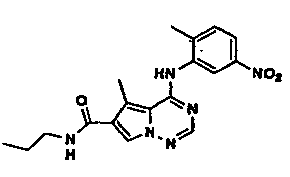

A solution of compound B (2.32 mmol), EDCI (489 mg, 2.55 mmol), and HOBt (345 mg, 2.55 mmol) in DMF (6 mL) was stirred at rt for 1 h, and then n-propyl amine (0.38 mL, 6.4 mmol) was added. The reaction was stirred for 4 h and water was added to precipitate the product. The solids were filtered and purified via column chromatography on silica (33% ethyl acetate\hexanes) to give compound C (0.79 g, 93% yield) as a white solid. ¹H NMR (CDCl₃): δ 9.11 (s, 1H), 7.92 (m, 2H), 7.71 (s, 1H), 7.36 (d, J=8.4 Hz, 1H), 5.82 (br m, 1H), 3.34 (q, J=6.7 Hz, 2H), 2.86 (s, 3H), 2.41 (s, 3H), 1.58 (m, 2H), 1.16 (t, J=7.5 Hz, 3H). LCMS (M+H⁺) = 369.3. HPLC (Condition A): 3.42 min.

### D. 4-[(5-Amino-2-methylphenyl)amino]-5-methyl-N-propylpyrrolo [2,1-f][1,2,4]triazine-6-carbozamide

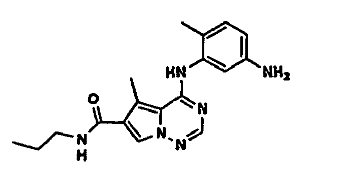

A solution of compound C (794 mg, 2.16 mmol) and 10% Pd/C (250 mg, wet) in MeOH (20 mL) was degassed and backfilled with hydrogen three times and stirred for 2 h. The solution was filtered and concentrated to give compound D (691 mg, 95% yield). ¹H NMR (CDCl₃): δ 7.94 (s, 1H), 7.73 (s, 1H), 7.53 (s, 1H), 7.23 (m, 1H), 7.06 (d, J=8.1 Hz, 1H), 6.53 (dd, J=8.1,2.2 Hz, 1H) 5.86 (br m, 1H), 3.43 (q, J=6.6 Hz, 2H), 2.91 (s, 3H), 2.27 (s, 3H), 1.68 (m, 2H), 1.02 (t, J=7.3 Hz, 3H). LCMS (M+H⁺) = 339.2. HPLC (Condition A): 2.39 min.

### E. Example 142

To a suspension of 25.6 g (0.076 mmol) of compound D in 0.3 mL of DCE was added 22 µL of ethyl isocyanate at rt. The reaction mixture was heated at 50°C for 12 h, then cooled, and isopropanol was added (1 mL). The resulting precipitated product was collected by vacuum filtration and washed with 1 mL of isopropanol and dried *in vacuo* to afford 19.6 mg (63%) of the titled compound as a pure product ¹H NMR (CD₃OD): δ 7.94 (s, 1H), 7.70 (s, 1H), 7.60 (s, 1H), 7.23 (br s, 2H), 7.44 (d, 1H), 3.23 (q, 2H), 2.84 (s, 3H), 2.24 (s, 3H), 1.66 (m, 2H), 1.16 (t, 3H), 1.02 (t, 3H). LCMS (M+H⁺) = 410.2. HPLC (Condition A): 2.82 min.

### Examples 143-148

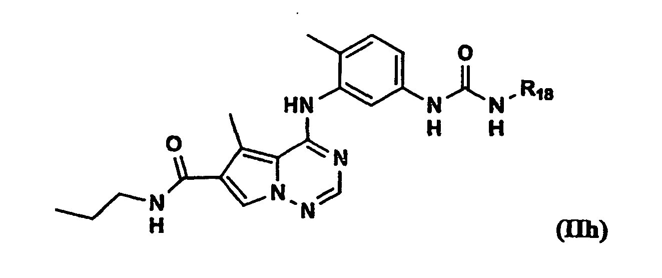

Compound having the formula (IIh), wherein R₁₈ has the values listed in Table 14 were prepared following the procedure outlined for Example 142, using different isocyanates in the last step.

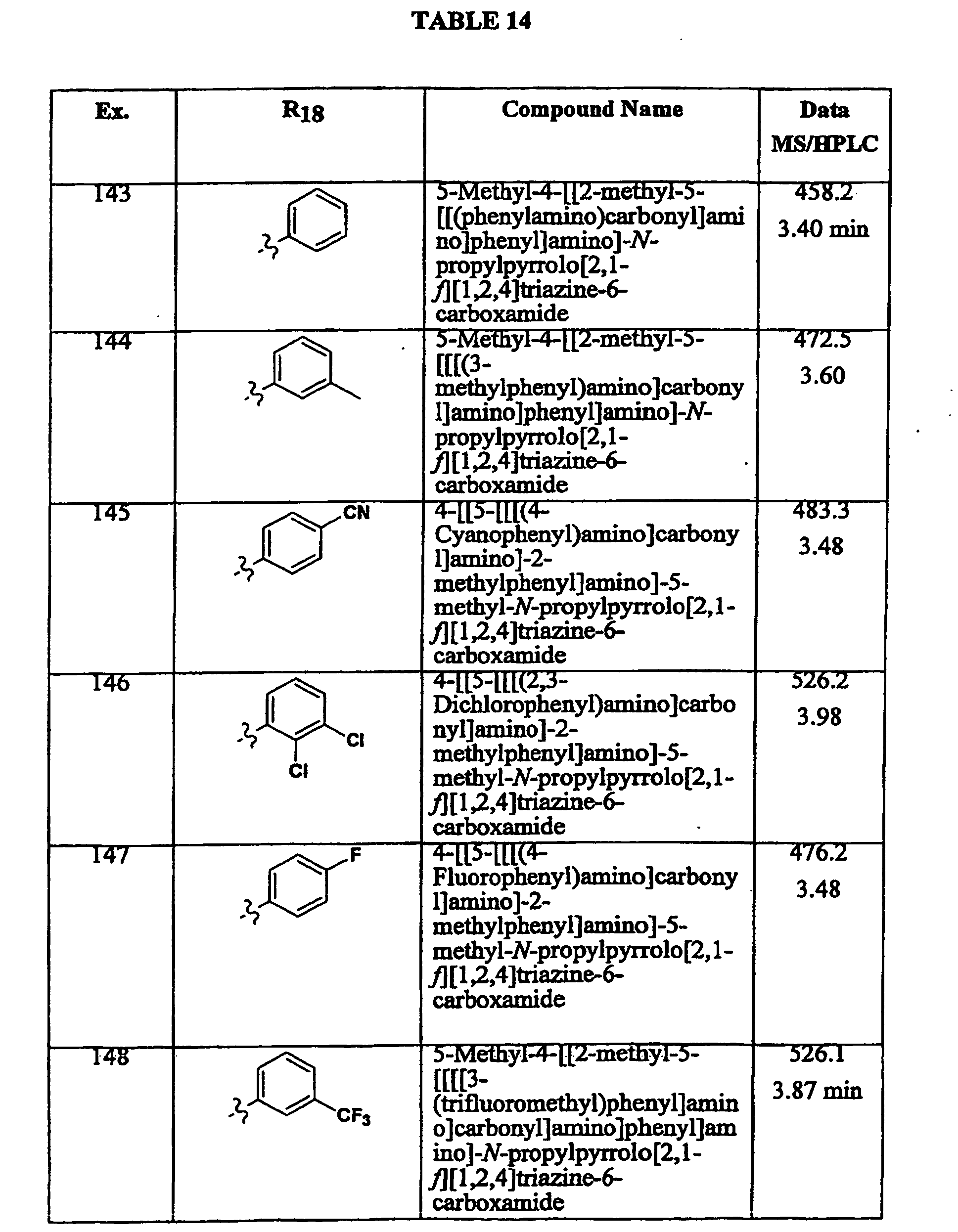

### Examples 149-152

The compounds named below were prepared using methods analogous to the procedures described hereinbefore:
149) 1,3-Dihydro-3-[5-methoxy-6-[[4-(4-methyl-1-piperazinyl)butyl]amino]pyrrolo[2,1-f][1,2,4]triazin-4-yl]-2H-indol-2-one;
150) 1,3-Dihydro-3-[5-methoxy-6-[[4-(4-morpholinyl)butyl]amino]pyrrolo[2,1-f][1,2,4]triazin-4-yl]-2H-indol-2-one;
151) 1-[3-[4-(2,3-Dihydro-2-oxo-1H-indol-3-yl)-5-methylpyrrolo[2,1-f][1,2,4]triazin-6-yl]-1-oxopropyl]-4-methylpiperazine; and
152) 2-Methyl-5-[[5-methyl-6-[3-(2H-1,2,3-triazol-2-yl)propoxy]pyrrolo[2,1-f][1,2,4]triazin-4-yl]amino]phenol.

## Claims

1. Use of at least one compound having the formula (I): 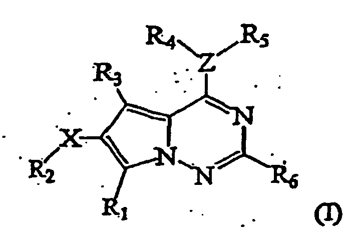 or a pharmaceutically acceptable salt, prodrug, or solvate thereof, wherein:
R₃ is hydrogen, methyl, perfluoromethyl, methoxy, halogen, cyano or NH₂;
X is selected from -O- -OC(=O)-, -S-, -S(=O)-, -SO₂-, -C(=O)-, -CO₂-, -NR₁₀-,-NR₁₀C(=O)-,-NR₁₀C(=C))NR₁₁-,-NR₁₀CO₂-,-NR₁₀SO₂-, -NR₁₀SO₂NR₁₁-, -SO₂NR₁₀-, -C(=O)NR₁₀-, halogen, nitro, and cyano, or X is absent;
Z is selected from O, S, N, and CR₂₀, wherein when Z is CR₂₀, said carbon atom may form an optionally-substituted bicyclic aryl or heteroaryl with R₄ and R₅;
R₁ is hydrogen, -CH₃, -OH, -OCH₃, -SH, -SCH₃, -OC(=O)R₂₁, -S(=O)R₂₂, -SO₂R₂₂, -SO₂NR₂₄R₂₅, -CO₂R₂₁, -C(=O)NR₂₄R₂₅, -NH₂; -NR₂₄R₂₅, -NR₂₁SO₂NR₂₄R₂₅, -NR₂₁SO₂R₂₂, -NR₂₄C(=O)R₂₅, -NR₂₄CO₂R₂₅, -NR₂₁=O)NR₂₄R₂₅, halogen, nitro, or cyano;
R₂ is selected from;
a) hydrogen, provided that R₂ is not hydrogen when X is -S(=O)-, -SO₂-, -NR₁₀CO₂-, or -NR₁₀SO₂-;
b) alkyl, alkenyl, and alkynyl optionally substituted with up to four R₂₆, or pentafluoroalkyl;
c) aryl and heteroaryl optionally substituted with up to three R₂₇; and
d) heterocyclo and cycloalkyl optionally substituted with keto (=O), up to three R₂₇, and/or having a carbon-carbon bridge of 3 to 4 carbon atoms; or
e) R₂ is absent if X is halogen, nitro or cyano;
(i) R₄ is substituted aryl, aryl substituted with NHSO₂alkyl, substituted heteroaryl, or an optionally-substituted bicyclic 7-11 membered saturated or unsaturated carbocyclic or heterocyclic ling, and
R₅ is hydrogen, alkyl, or substituted alkyl, except when Z is O or S, R₅ is absent, or alternatively,
(ii) R₄ and R₅ taken together with Z form an optionally-substituted bicyclic 7-11 membered aryl or heteroaryl;
R₆ is hydrogen, alkyl, substituted alkyl, aryl, substituted aryl, heterocyclo, substituted heterocyclo, -NR₇R₈, -OR₇, or halogen;
R₁₀ and R₁₁ are independently selected from hydrogen, alkyl, substituded alkyl, aryl, substituted aryl, cycloalkyl, substituted cycloalkyl, hetsrocyclo, and substituted heterocyclo;
R₇, R₈, R₂₁, R₂₄, and R₂₅ are independently selected from hydrogen, alkyl, substituted alkyl, aryl, substituted aryl, heterocyclo, and substituted heteipcyclo;
R₂₀ is hydrogen, lower alkyl, or substituted alkyl, or R₂₀ may be absent if the carbon atom to which it is attached together with R₄ and R₅ is part of an unsaturated bicyclic aryl or heteroaryl;
R₂₂ is alkyl, substituted alkyl, aryl, substituted aryl, heterocyclo, or substituted heterocyclo;
R₂₆ is selected from halogen, trifluoromethyl, haloalkoxy, keto (=O), nitro, cyano, SR₂₈, -OR₂₈, -NR₂₈R₂₉, -NR₂₈SO₂, NR₂₈SO₂R₂₉, -SO₂R₂₈, -SO₂NR₂₈R₂₉, -CO₂R₂₈, -C(=O)R₂₈, -C(=O)NR₂₈R₂₉, -OC(=O)R₂₈, -OC(=O)NR₂₈ R₂₉, -NR₂₈C(O)R₂₉, NR₂₈CO₂R₂₉, =N-OH, =N-O-alkyl; aryl optionally substituted with one to three R₂₇; cycloalkyl optionally substituted with keto(=O), one to three R₂₇, or having a carbon-carbon bridge of 3 to 4 carbon atoms; and heterocyclo optionally substituted with keto (=O), one to three R₂₇, or having a carbon-carbon bridge of 3 to 4 carbon atoms; wherein R₂₈ and R₂₉ are each independently selected from hydrogen, alkyl, alkenyl, aryl, aralkyl, C₃₋₇cycloalkyl, and C₃₋₇heterocycle, or may be taken together to form a C₃₋₇-heterocycle; and wherein each R₂₈ and R₂₉ in turn is optionally substituted with up to two of alkyl, alkenyl, halogen, haloalbyl, haloalkoxy, cyano, nitro, amino, hydroxy, alkoxy, alkylthio, phenyl, benzyl, phenyloxy, and benzyloxy; and
R₂₇ is selected from alkyl, R₃₂, and C₁₋₄alkyl substituted with one to three R₃₂, wherein each R₃₂ group is independently selected from halogen, haloalkyl, haloalkoxy, nitro, cyano, -SR₃₀, -OR₃₀, -NR₃₀R₃₁, -NR₃₀SO₂, NR₃₀SO₂R_{31,} -SO₂R₃₀, -SO₂NR₃₀R₃₁, -CO₂R₃₀, -C(=O)R₃₀, -C(=O)NR₃₀R₃₁, -OC(=O)R₃₀, -OC(=O)NR₃₀R₃₁, -NR₃₀C(=O)R₃₁, -NR₃₀CO₂R₃₁, and a 3 to 7 membered carbocyclic or heterocyclic ring optionally substituted with alkyl, halogen, hydroxy, alkoxy, haloalkyl, haloalkoxy, nitro, amino, or cyano, wherein R₃₀ and R₃₁ are each independently selected from hydrogen, alkyl, alkenyl, aryl, aralkyl, C₃₋₇cycloalkyl, and heterocycle, or may be taken together to form a C₃₋₇-heterocycle for the manufacture of a medicament for treating one or more conditions associated with p38 kinase activity.

2. Use according to claim 1, wherein:
R₃ is methyl, -CF₃, or -OCH₃;
X is selected from -C(=O)-, -CO₂-, -NR₁₀-, NR₁₀C(=O)-, -NR₁₀CO₂-, -NR₁₀SO₂-, -SO₂NR₁₀-, and -C(=O)NR₁₀-, or X is absent;
Z is N;
R₂ is hydrogen, C₂₋₆alkyl, C₁₋₄alkyl substituted with up to four R₂₆, pentafluoroalkyl, or aryl or heteroaryl optionally substituted with up to two R₂₇;
R₄ is pheayl substituted with one R₁₂ and zero to three R₁₃;
R₅ and R₁₀ independently are selected from hydrogen and lower alkyl;
R₁₂ is carbamyl, sulfonamido, arylsulfonylamine, or ureido, each of which is optionally substituted with up to two of hydroxy, alkyl, substituted alkyl, allcoxy, aryl, substituted aryl, and aralkyl, or alkylsulfonylamine;
R₁₃ at each occurence is independently selected from alkyl, substituted alkyl, halo, trifluoromethoxy, trifluoromethyl,-OR₁₄, -C(=O)alkyl, -OC(=O)alkyl, -NR₁₅R₁₆, -SR₁₅, -NO₂, -CN, -CO₂R₁₅, -CONH₂, -SO₃H, -S(=O)alkyl, -S(=O)aryl,- NHSO₂-aryl=R₁₇, -NHSO₂-alkyl, -SO₂NHR₁₇, -CONHR₁₇, and -NHC(=O)NHR₁₇;
R₁₄ is hydrogen, alkyl, or aryl;
R₁₅ is hydrogen or alkyl;
R₁₆ is hydrogen, alkyl, aralkyl, or alkanoyl; and
R₁₇ is hydrogen, hydroxy, alkyl, substituted alkyl, alkoxy, aryl, substituted aryl, or aralkyl.

3. Use of at least one compound having the formula (I): 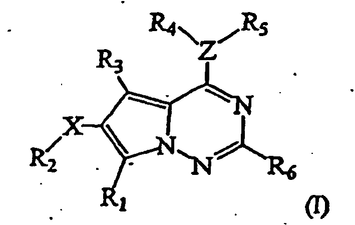 or a pharmaceutically acceptable salt, prodrug or solvate thereof, wherein:
R₃ is hydrogen, methyl, perfluoromethyl methoxy, halogen, cyano or NH₂;
X is selected from -O-, -OC(=O)-, -S-, -S(=O)-, -SO₂-, -C(=O)-, -CO₂-, -NR₁₀-, -NR₁₀C(=O)-, -NR₁₀C(=O)NR₁₁-, -NR₁₀CO₂-, -NR₁₀SO₂-, -NR₁₀SO₂NR₁₁-, -SO₂NR₁₀-, -C(=O)NR₁₀-, halogen, nitro, and cyano, or X is absent;
Z is O, S, N, or CR₂₀;
R₁ is hydrogen, -CH₃, -OH, -OCH₃, -SH, -SCH₃, -OC(=O)R₂₁, -S(=O)R₂₂, -SO₂R₂₂, -SO₂NR₂₄R_{25'} -CO₂R₂₁, -C(=O)NR₂₄R₂₅,-NH₂, -NR₂₁SO₂NR₂₅, -NR₂₁SO₂R₂₂, -NR₂₄C(=O)R₂₅, -NR₂₄CO₂₅, -NR₂₁C(=O)NR₂₄R_{25'} halogen, nitro, or cyano;
R₂ is hydrogen, alkyl, substituted alkyl, alkenyl, substituted alkenyl, alkynyl, substituted alkynyl, aryl, substituted aryl, heterocyclo, substituted heterocyclo, aralkyl, substituted aralkyl, or heterocycloalkyl, or substituted heterocycloalkyl, or when X is halo, nitro or cyano, R₂ is absent, provided that R₂ is not hydrogen when X is -S(=O)-, -SO₂- , -NR₁₀CO₂-, or -NR₁₀SO₂-;
R₄ is substituted aryl, aryl substituted with NHSO₂alkyl, substituted heteroaryl, or an optionally-substituted bicyclic 7-11 membered saturated or unsaturated carbocyclic or heterocyclic ring system;
R₅ is hydrogen, alkyl, or substituted alkyl, except that when Z is O or S, R₅ is absent;
R₆ is hydrogen, alkyl, substituted alkyl, aryl, substituted aryl, heterocyclo, substituted heterocyclo, -NR₇R₈, -OR₇, or halogen;
R₇, R₉, R₁₀, R₁₁, R₂₁, R₂₄, and R₂₅ are independently selected from hydrogen, alkyl, substituted alkyl, aryl, substituted aryl, heterocyclo, and substituted heterocyclo;
R₂₀ is hydrogen, lower alkyl, or substituted alkyl; and
R₂₂ is alkyl, substituted alkyl, aryl, substituted aryl, heterocyclo, or substituted heterocyclo for the manufacture of a medicament for treating one or more conditions associated with p38 kinase activity.

4. Use according to claim 3, wherein R₄ and R₅ taken together with Z form: 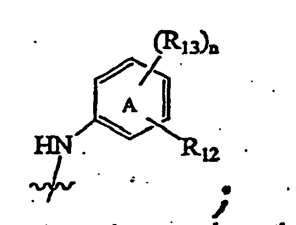
R₁₂ is attached to any available, carbon atom of phenyl ring A and is selected from carbamyl, sulfonamido, arylsulfonylamine, and ureido, each of which is optionally substituted with up to one of hydroxy, alkyl, substituted alkyl, alkoxy, aryl, substituted aryl, and aralkyl, or C₁₋₄alkylsulfonylamine;
R₁₃ is attached to any available carbon atom of phenyl ring A and at each occurrence is independently selected from alkyl, substituted alkyl, halo, trifluoromethoxy, trifluoromethyl, -OR₁₄, -C(=O)alkyl, -OC(=O)alkyl, -NR₁₅R₁₆, -SR₁₅, -NO₂, -CN, -CO₂R₁₅, -CONH₂, -SO₃H, -S(=O)alkyl, -S(=O)aryl, -NHSO₂-aryl- R₁₇, -NHSO₂C₁₋₄alkyl, -SO₂NHR₁₇, -CONHR₁₇, and -NHC(=O)NHR₁₇;
R₁₄ is hydrogen, alkyl, or aryl;
R₁₅ is hydrogen or alkyl;
R₁₆ is hydrogen, alkyl, aralkyl, or alkanoyl; and
R₁₇ is hydrogen, hydroxy, alkyl, substituted alkyl, alkoxy, aryl, substituted aryl, or aralkyl; and
*n* is 0,1,2 or 3.

5. Use according to claim 3, wherein the at least one compound has the formula (II): 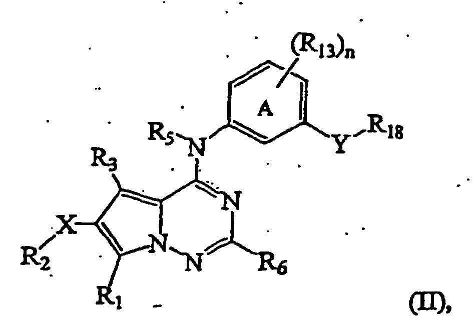 wherein :
R₃ is methyl or CF₃;
X is -C(=O)NR₁₀-, -NR₁₀C(=O)-, -C(=O)-, or -CO₂-;
R₁ is hydrogen, -CH₃, -OH, -OCH₃, halogen, nitro, or cyano;
Y is -C(=O)NH, -NHC(=O)NH-, -NHSO₂-, or -SO₂NH-;
R₁₀ is hydrogen or lower alkyl;
R₁₈ is selected from hydrogen, alkyl, alkoxy, aryl, and aryl substituted with one to three R₁₉, except that when Y is NHSO₂-, R₁₈ is C₁₋₄alkyl, aryl or aryl substituted with R₃₉;
R₁₃ is attached to any available carbon atom of phenyl ring A and at each occurrence is independently selected from alkyl, substituted alkyl, halo, trifluoromethoxy, trifluoromethyl -OR₁₄, -C(=O)alkyl, -OC(=O)alkyl, -NR₁₅R₁₆, -SR₁₅, -NO₂, -CN, -CO₂R₁₅, -CONH₂, -SO₃H, -S(=O)alkyl, -S(=O)aryl, -NHSO₂-aryl- R₁₇, -NHSO₂C₁₋₄alkyl, -SO₂NHR₁₇, -CONHR₁₇, and -NHC(=O)NHR₁₇;
R₁₄, R₁₅, R₁₆ and R₁₇ are hydrogen or alkyl;
R₁₉ at each occurence is selected from alkyl, halo, trifluoromethoxy, trifluoromethyl, hydroxy, alkoxy, alkanoyl, alkanoyloxy, thiol, alkylthio, ureido, nitro, cyano, carboxy, carboxyalkyl, carbamyl, alkoxycarbonyl, alkylthiono, arylthiono, arylsulfonylamine, sulfonic acid, alkylsulfonyl, sulfonamido, and aryloxy, wherein each group R₁₉ may be further substituted by hydroxy, alkyl, alkoxy, aryl or aralkyl; and
*n* is 0, 1 or 2.

6. Use according to claim 3, wherein the at least one componnd has the formula (Ia), (Ib), or (Ic): 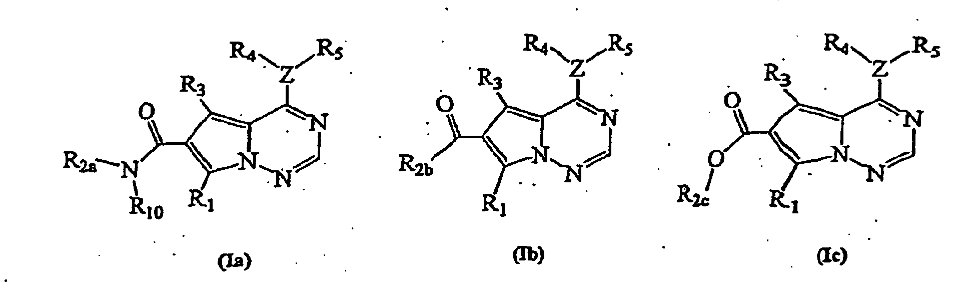
wherein:
R₃ is methyl or CF₃;
R₂ₐ and R_{2c} are independently selected from-hydrogen, C₂₋₆alkyl, substituted C₁₋₄alkyl, aryl, substituted aryl, benzyl, and substituted benzyl;
R_{2b} is heterocyclo or substituted heterocycle; and
R₁₀ is hydrogen or lower alkyl.

7. A compound having the formula (II): 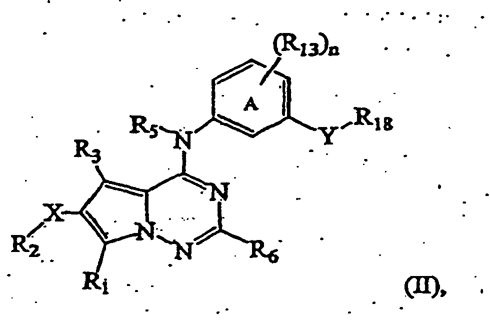 or a pharmaceutically acceptable salt, prodrug or solvate thereof, wherein:
R₃ is methyl-, -CF₃, or -OCH₃; R₅ is hydrogen or alkyl;
Y is -4C(=O)NR₂₃-, -NR₂₃C(=O)NR₂₃-, -SO₂NR₂₃, or -NR₂₃SO₂-;
R₁₈ and R₂₃ are selected from hydrogen, alkyl, alkoxy, aryl, and aryl substituted with one to three R₁₉, except when Y is-NR₂₃SO₂-, R₁₈ C₁₋₄alkyl or aryl optionally substituted with one to three R₁₉;
X is selected from -O-, -OC(=O)-, -S-, -S(=O)-, -SO₂-, -C(=O)-, -CO₂-, -NR₁₀-, -NR₁₀C(=O)-, -NR₁₀C(=O)NR₁₁-, -NR₁₀CO₂-, -NR₁₀SO₂-, -NR₁₀SO₂NR₁₁-, -SO₂NR₁₀-, and -C(=O)NR₁₀-, or X is absent;
R₁ is hydrogen, -CH₃, -OH, -OCH₃, -SH, -SCH₃, -OC(=O)R₂₁, -S(=O)R₂₂, -SO₂R₂₂, -SO₂NR₂₄R₂₅, -CO₂R₂₁, -C(=O)NR₂₄R₂₅, -NH₂, -NR₂₄R₂₅, -NR₂₁SO₂NR₂₄R₂₅, -NR₂₁SO₂R₂₂, -NR₂₄C(=O)R₂₅, -NR₂₄CO₂R₂₅, -NR₂₁C(=O)NR₂₄R₂₅, halogen, nitro, or cyano;
R₂ is selected from
a) hydrogen, provided that R₂ is not hydrogen when X is -S(=O)-, -SO₂-, -NR₁₀CO₂-, or -NR₁₀SO₂-;
b) alkyl, alkenyl, and alkynyl optionally substituted with up to four R₂₆, or pentafluoroalkyl;
c) aryl and heteroaryl optionally substituted with up to three R₂₇; and
d) heterocyclo and cycloalkyl optionally substituted with keto (=O), up to three R₂₇, and/or having a carbon-carbon bridge of 3 to 4 carbon atoms;
R₆ is hydrogen, alkyl, substituted alkyl, aryl, substituted aryl, heterocyclo, substituted heterocyclo, -NR₇R₈, -OR₇, or halogen;
R₁₀ is hydrogen or alkyl;
R₁₃ and R₁₉ at each occurrence are independently selected from alkyl, halo, trifluoromethoxy, trifluoromethyl, hydroxy, alkoxy, alkanoyl, alkanoyloxy, thiol, alkylthio, ureido, nitro, cyano, carboxy, carboxyalkyl, carbamyl, alkoxycarbonyl, alkylthiono, arylthiono, arylsulfonylamine, C₁₋₄-alkylsulfonylamine, sulfonic acid, alkylsulfonyl, sulfonamido, and aryloxy, wherein each R₁₃ and/or R₁₉ group may be further substituted by hydroxy, alkyl, substituted alkyl, alkoxy, aryl, or aralkyl;
R₇, R₈, R₂₁, R₂₄, and R₂₅ are independently selected from hydrogen, alkyl, substituted alkyl, aryl, substituted aryl, heterocyclo, and substituted heterocyclo;
R₂₂ is alkyl, substituted alkyl, aryl, substituted aryl, heterocyclo, or substituted heterocyclo;
R₂₆ is selected from halogen, trifluoromethyl, haloalkoxy, keto (=O), nitro, cyano, -SR₂₈, -OR₂₈, -NR₂₈R₂₉, -NR₂₈SO₂, -NR₂₈SO₂R₂₉, -SO₂R₂₈ -SO₂NR₂₈R₂₉, -CO₂R₂₈, -C(=O)R₂₈, -C(=O)NR₂₈R₂₉, -OC(=O)R₂₆, -OC(=O)NR₂₈R₂₉, -NR₂₈C(=O)R₂₉, -NR₂₈CO₂R₂₉, =N-OH, =N-O-alkyl; aryl optionally substituted with one to three R₂₇; cycloalkyl optionally substituted with keto(=O), one to three R₂₇, or having a carbon-carbon bridge of 3 to 4 carbon atoms; and heterocyc optionally substituted with keto(=O), one to three R₂₇, or having a carbon-carbon bridge of 3 to 4 carbon atoms; wherein R₂₈ and R₂₉ are each independently selected from hydrogen, alkyl, alkenyl, aryl, aralkyl, C₃₋₇cycloalkyl, and C₃₋₇heterocycle, or may be taken together to form a C₃₋₇-heterocycle; and wherein each R₂₈ and R₂₉ in turn is optionally substituted with up to two of alkyl, alkenyl, halogen, haloalkyl, haloalkoxy, cyano, nitro, amino, hydroxy, alkoxy, alkylthio, phenyl, benzyl, phenyloxy, and benzyloxy;
R₂₇ is selected from alkyl, R₃₂, and C₁₋₄alkyl substituted with one to three R₃₂, wherein each R₃₂ group is independently selected from halogen, haloalkyl, haloalkoxy, nitro, cyano, -SR₃₀, -OR₃₀, -NR₃₀R₃₁, -NR₃₀SO₂, -NR₃₀SO₂R₃₁, -SO₂R₃₀, -SO₂NR₃₀R₃₁, -CO₂R₃₀, -C(=O)R₃₀, -C(=O)NR₃₀R₃₁-OC(=O)R₃₀, -OC(=O)NR₃₀R₃₁, -NR₃₀C(=O)R₃₁, -NR₃₀CO₂R₃₁, and a 3 to 7 membered carbocyclic or heterocyclic ring optionally substituted with alkyl, halogen, hydroxy, alkoxy, haloalkyl, haloalkoxy, nitro, amino, or cyano, wherein R₃₀ and R₃₁ are each independently selected from hydrogen, alkyl, alkenyl, aryl, aralkyl, C₃₋₇cycloalkyl, and heterocycle, or may be taken together to form a C₃₋₇-heterocycle; and
*n* is 0,1 or 2; excluding 4-[[3-(aminosulfonyl)-4-methylphenyl]-amino]-5-methylpyrrolo[2,1-f][1,2,4]triazine-6-carboxylic acid methyl ester.

8. A compound according to claim 7, having the formula (IIa) or (IIb), 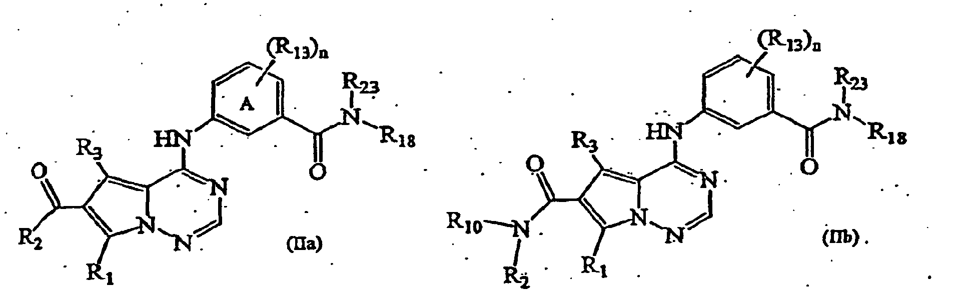 or a pharmaceutically acceptable salt, prodrug, or solvate thereof, wherein:
R₁ and R₁₀ are hydrogen or -CH₃;
R₁₃ is lower alkyl, halogen, trifluoromethoxy, trifluoromethyl, hydroxy, C₁₋₄alkoxy, nitro, or cyano;
R₁₈ is hydroxy, C₁₋₄alkoxy, phenyl, or phenyl substituted with one or two R₁₉;
R₂₃ is hydrogen or lower alkyl; and
*n* is 0, 1 or 2.

9. A compound according to claim 7, or a pharmaceutically acceptable salt, prodrug or solvate thereof, wherein
X is -C(=O)-, -CO₂-, -NR₁₀C(=O)-, or -C(=O)NR₁₀-;
Y is -C(=O)NH-, -NHC(=O)NH-, or -NHSO₂-;
R₅ and R₁₀ are hydrogen or -CH₃;
R₁₃ and R₁₉ at each occurrence are independently selected from lower alkyl, halo, trifluoromethoxy, trifluoromethyl, hydroxy, C₁₋₄alkoxy, nitro, and cyano;
R₇, R₈, R₂₁, R₂₄, and R₂₅ are independently selected from hydrogen and lower alkyl;
R₂₂ is lower alkyl; and
*n* is 0 or 1.

10. A compound according to claim 7 or a pharmaceutically acceptable salt, prodrug or solvate thereof, in which X-R₂ are: 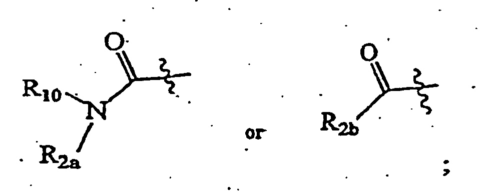
R₂ₐ is selected from:
a) hydrogen;
b) straight or branched C₂₋₆alkyl;
c) cycloalkyl optionally substituted with keto and/or up to two R₂₇;
d) phenyl optionally substituted with up to two R₂₇; and
e) heterocycle optionally substituted with keto and/or up to two R₂₇;
f) pentafluoroalkyl or C₁₋₄alkyl substituted with up to three of halogen, trifruoromethyl, cyano, OR₂₈, NR₂₈R₂₉, CO₂R₂₈, aryl, heterocycle, and/or cycloallcyl, wherein the aryl, heterocycle, and/or cycloalkyl in turn are optionally substituted with up to two of halogen, hydroxy, alkoxy, haloalkyl, haloalkoxy, nitro, cyano and alkyl; and
R_{2b} is a monocyclic or bicyclic heterocycle optionally substituted with up to two R₂₇;
R₂₇ at each occurrence is independently selected from hydrogen, alkyl, trifluoromethyl, trifluoromethoxy, halogen, cyano, nitro, amino, hydroxy, alkoxy, phenyl, benzyl, phenyloxy, and benzyloxy; and
R₂₈ and R₂₉ at each occurrence are independently selected from hydrogen, alkyl, alkenyl, phenyl, and benzyl.

11. A compound according to claim 7 or a pharmaceutically acceptable salt, prodrug or solvate thereof, in which:
R₂ₐ is selected from:
a) straight or branched C₂₋₆alkyl;
b) phenyl optionally substituted with up to two of halogen, C₁₋₄alkoxy, and trifluoromethyl;
c) C₃₋₆cycloalkyl optionally substituted with up to two C₁₋₄alkyl and/or hydroxy;
d) straight or branched C₁₋₄alkyl substituted with up to three of
i) halogen,
ii) trifluoromethyl,
iii) cyano,
iv) C₁₋₄alkoxy,
v) phenyloxy,
vi) benzyloxy,
vii) NH₂, NH(C₁₋₄alkyl), and/or N(C₁₋₄alkyl)₂,
viii) phenyl in turn optionally substituted with up to two of halogen and/or methoxy,
ix) heterocycle selected from pyridinyl, indolyl, thiophenyl, furanyl, thiazolyl, thienyl, morpholinyl, tetrahydrofuranyl, triazinyl, piperazinyl, indenyl, and piperidinyl; said heterocycle optionally substituted with one to two C₁₋₄alkyl,
x) C₃₋₆cycloalkyl; and
R_{2b} is a five to seven membered monocyclic heterocycle selected from diazepinyl, morpholinyl, piperidinyl, and pyrrolidinyl, said heterocycle optionally substituted with C₁₋₄alkyl, phenyl, and/or benzyl.

12. A compound according to claim 7 or a pharmaceutically acceptable salt, prodrug or solvate thereof, in which R₁ and R₁₀ are independently hydrogen or CH₃.

13. A compound according to claim 7 or a pharmaceutically acceptable salt, prodrug or solvate thereof, in which R₆ is hydrogen.

14. A compound according to claim 7 or a pharmaceutically acceptable salt, prodrug or solvate thereof, in which Y is -NHC(=O)NH- or -NHSO₂- ; R₁₈ is aryl or aryl substituted with alkyl, OCH₃, CF₃, cyano, or halogen.

15. A compound according to claim 7 or a pharmaceutically acceptable salt, prodrug or solvate thereof, in which Y is -C(=O)NR₂₃-,R₂₃ is hydrogen or lower alkyl, and R₁₈ is C₁₋₄alkoxy or aryl optionally substituted with alkyl, OCH₃, CF₃, cyano or halogen.

16. A compound according to claim 7, having the formula: 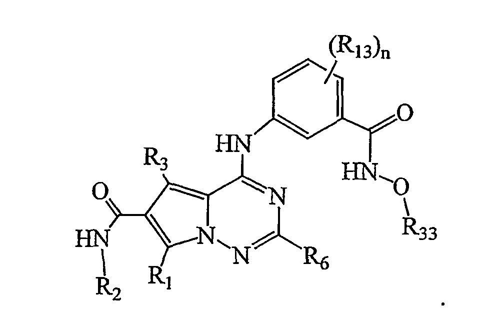 in which R₃₃ is lower alkyl.

17. A compound according to claim 16 or a pharmaceutically acceptable salt, prodrug or solvate thereof, in which R₃ and R₃₃ are methyl, R₁ and R₆ are hydrogen, and R₂ is a straight or branched C₂₋₆alkyl or optionally-substituted benzyl.

18. A compound according to claim 7, which is selected from (i) N-(2,2-Dimethylpropyl)-4-[[5-[(methoxyamino)carbonyl]-2-methylphenyl]amino]-N,5-dimethylpyrrolo[2,1-f] [1,2,4]triazine-6-carboxamide;
3-[[6-[(Hexahydro-4-methyl-1H-1,4-diazepin-1-yl)carbonyl]-5-methylpyrrolo[2,1-f][1,2,4]triazin-4-yl]amino]-N-methoxy-4-methylbenzamide;
4-[[5-[(Methoxyamino)carbonyl]-2-methylphenyl]amino]-5-methyl-N-(1-methylethyl)pyrrolo[2,1-f] [1,2,4]triazine-6-carboxamide;
4-[[5-[(Methoxyamino)carbonyl]-2-methylphenyl]amino]-5-methyl-N-(2-methylpropylpyrrolo[2,1-f][1,2,4]triazine-6-carboxamide;
N-(2,2-Dimethylpropyl)4-[[5-[(methoxyamino)carbonyl]-2-methylphenyl]amino]-5-methylpyrrolo[2,1-f][1,2,4]triazine-6-carboxamide;
4-[[5-[(Methoxyamino)carbonyl]-2-methylphenyl]amino]-5-methyl-N-propylpyrrolo[2,1-f][1,2,4]triazine-6-carboxamide;
N-(1,1-Dimethylethyl)-4-[[5-[(methoxyamino)carbonyl]-2-methylphenyl]amino]-5-methylpyrrolo[2,1-f][1,2,4]triazine-6-carboxamide;
4-[[5-[(Methoxyamino)carbonyl]-2-methylphenyl]amino]-N-(2-methoxyethyl)-5-methylpyrrolo[2,1-f][1,2,4]triazine-6-carboxamide;
N-Methoxy-4-methyl-3-[[5-methyl-6-(4-morpholinylcarbonyl)pyrrolo[2,1-f][1,2,4]triazin-4-yl]amino]benzamide;
N-Cyclohexyl-4-[[5-[methoxyamino)carbonyl]-2-methylphenyl]amino]-5-methylpyrrolo[2,1-f][1,2,4]triazine-6-carboxamide;
4-[[5-[(Methoxyamino)carbonyl]-2-methylphenyl]amino]-5-methyl-N-[(1R)-1-phenylethyl]pyrrolo[2,1-f][1,2,4]triazine-6-carboxamide;
4-[[5-[(Methoxyamino)carbonyl]-2-methylphenyl]amino]-5-methyl-N-(1S)-1-phenylethyl]pyrrolo[2,1-f][1,2,4]triazine-6-carboxamide;
N-[(4-Fluorophenyl)methyl]-4-[[5-[(methoxyamino)carbonyl]-2-methylphenyl]amino]-5-methylpyrrolo[2,1-f][1,2,4]triazine-6-carboxamide;
4-[[5-[(Methoxyamino)carbonyl]-2-methylphenyl]amino]-N[(2-methoxyphenyl)methyl]-5-methylpyrrolo[2,1-f][1,2,4]triazine-6-carboxamide;
4-[[5-[(Methoxyamino)carbonyl]-2-methylphenyl]amino]-5-methyl-N-(4-pyridinylmethyl)pyrrolo[2,1-f][1,2,4]triazine-6-carboxamide;
4-[[5-[(Methoxyamino)carbonyl]-2-methylphenyl]amino]-5-methyl-N-[2-(4-pyridinyl)ethyl]pyrrolo[2,1-f][1,2,4]triazine-6-carboxamide;
4-[[5-[(Methoxyamino)carbonyl]-2-methylphenyl]amino]-5-methyl-N-[2-(1-piperidinyl)ethyl]pyrrolo[2,1-f][1,2,4]triazine-6-carboxamide;
4-[[5-[(Methoxyamino)carbonyl]-2 methylphenyl]amino]-5-methyl-N-[2-(4-morpholinyl)ethyl]pyrrolo[1,2-f][1,2,4]triazine-6-carboxamide;
N-[(1R,2S)-2,3-Dihydro-1H-inden-1-yl]-4-[[5-[(methoxyamino)carbonyl]-2-methylphenyl]amino]-5-methylpyrrolo[2,1-f][1,2,4]triazine-6-carboxamide;
N-[(1S,2R)-2,3-Dihydro-1H-inden-1-yl]-4-[[5-[(methoxyamino)carbonyl]-2-methylphenyl]amino]-5-methylpyrrolo[2,1-f][1,2,4]triazine-6-carboxamide;
N-Methoxy-4-methyl-3-[[5-methyl-6-[[4-(phenylmethyl)-1-piperidinyl]carbonyl]pyrrolo[2,1-f][1,2,4]triazin-4-yl]amino]benzamide;
N-Cyclopropyl-4-[[5-[(methoxyamino)carbonyl]-2-methylphenyl]amino]-5-methylpyrrolo[2,1-f][1,2,4]triazine-6-carboxamide;
N-Cyclopentyl-4-[[5-[(methoxyamino)carbonyl]-2-methylphenyl]amino]-5-methylpyrrolo[2,1-f][1,2,4]triazine-6-carboxamide;
N-[2-(4-Fluorophenyl)ethyl]-4-[[5-[(methoxyamino)carbonyl]-2-methylphenyl]amino]-5-methylpyrrolo[2,1-f][1,2,4]triazine-6-carboxamide;
N-(Cyclohexylmethyl)-4-[[5-[(methoxyamino)carbonyl]-2-methylphenyl]amino]-5-methylpyrrolo[2,1-f][1,2,4]triazine-6-carboxamide;
4-[[5-[(Methoxyamino)carbonyl]-2-methylphenyl]amino]-5-methyl-N-[(tetrahydro-2-furanyl)methyl]pyrrolo[2,1-f][1,2,4]triazine-5-carboxamide;
N-(2-1H-Indol-3-ylethyl)-4-[[5-[(methoxyamino)carbonyl]-2-methylphenyl]amino]-5-methylpyrrolo[2,1-f][1,2,4]triazine-6-carboxamide;
N-Butyl-4-[[5-[(methoxyamino)carbonyl]-2-methylphenyl]amino]-5-methylpyrrolo[2,1-f][1,2,4]triazine-6-carboxamide;
N-(Cyclopropylmethyl)-4-[[5-[(methoxyamino)carbonyl]-2-methylphenyl]amino]-5-methylpyrrolo[2,1-f][1,2,4]triazine-6-carboxamide;
4-[[5-[(Methoxyamino)carbonyl]-2-methylphenyl]amino]-5-methyl-N-(2-methylbutyl)pyrrolo[2,1-f][1,2,4]triazine-6-carboxamide;
N-(2-Furanylmethyl)-4-[[5-[(methoxyamino)carbonyl]-2-methylphenyl]amino]-5-methylpyrrolo[2,1-f][1,2,4]triazine-6-carboxamide;
4-[[5-[(Methoxyamino)carbonyl]-2-methylphenyl]amino]-5-methyl-N-(2-thienylmethyl)pyrrolo[2,1-f][1,2,4]triazine-6-carboxamide;
4-[[5-[(Methoxyamino)carbonyl]-2-methylphenyl]amino]-5-methyl-N-(2-phenoxyethyl)pyrrolo[2,1-f][1,2,4]triazine-6-carboxamide;
4-[[5-[(Methoxyamino)carbonyl]-2-methylphenyl]amino]-5-methyl-N-(2-methylcyclohexyl)pyrrolo[2,1-f][1,2,4]triazine-6-carboxamide;
N-Ethyl-4-[[5-[(methoxyamino)carbonyl]-2-methylphenyl]amino]N,5-dimethylpyrrolo[2,1-f][1,2,4]triazine-6-carboxamide;
4-[[5-[(Methoxyamino)carbonyl]-2-methylphenyl]amino]-5-methyl-N-(2,2,2-trifluoroethyl)pyrrolo[2,1-f][1,2,4]triazine-6-carboxamide;
N-(2-Fluoroethyl)-4-[[5-[(methoxyamino)carbonyl]-2-methylphenyl]amino]-5-methylpyrrolo[2,1-f][1,2,4]triazine-6-carboxamide;
N-(2,3 Diydro-1H-inden-2-yl)-4-[[5-[(methoxyamino)carbonyl]-2-methylphenyl]amino]-5-methylpyrrolo[2,1-1][1,2,4]triazine-6-carboxamide;
N-Ethyl-4-[[5-[(methoxyamino)carbonyl]-2-methylphenyl]amino]-5-methylpyrrolo[2,1-f][1,2,4]triazine-6-carboxamide; 4-[[5-[Methoxyamino)carbonyl]-2-methylphenyl]amino]-5-methyl-N-(2,2,3,3,3-pentafluoropropyl)pyrrolo[2,1-f][1,2,4]triazine-6-carboxamide;
4-[[5-[(Methoxyamino)carbonyl]-2-methylphenyl]amino]-5,7-dimethyl-N-(1-methylethyl)pyrrolo[2,1-f][1,2,4]triazine-6-carboxamide;
N-(4-Fluorophenyl)-4-[[5-[(methoxyamino)carbonyl]-2-methylphenyl]amino]-5-methylpyrrolo[2,1-f][1,2,4]triazine-6-carboxamide;
4-[[5-[(Methoxyamino)carbonyl]-2-methylphenyl]amino]-N-(2-methoxyphenyl)-5-methylpyrrolo[2,1-f][1,2,4]triazine-6-carboxamide;
4-[[5-[(Methoxyamino)carbonyl]-2-methylphenyl]amino]-N-[(3-methoxyphenyl)methyl]-5-methylpyrrolo[2,1-f][1,2,4]triazine-6-carboxamide;
4-[[5-[(Methoxyamino)carbonyl]-2-methylphenyl]amino]-5-methyl-N-[3-(trifluoromethyl)phenyl]pyrrolo[2,1-f][1,2,4]triazine-6-carboxamide;
N-[(2,6-Dichlorophenyl)methyl]-4-[[5-[(methoxyamino)carbonyl]-2-methylphenyl]amino]-5-methylpyrrolo[2,1-f][1,2,4]triazine-6-carboxamide;
N-[(1S)-1-Cyano-2-phenylethyl]-4-[[5-[(methoxyamino)carbonyl]-2-Methylphenyl]amino]-5-methylpyrrolo[2,1-f][1,2,4]triazine-6-carboxamide;
4-[[5-[(Methoxyamino)carbonyl]-2-methylphenyl]amino]-5 methyl-N-(2-phenylethyl)pyrrolo[2,1-f][1,2,4]triazine-6-carboxamide;
N-Methoxy-4-methyl-3-[[5-methyl-6-(1-pyrrolidinylcarbonyl)pyrrolo[2,1-f][1,2,4]triazin-4-yl]amino]benzamide;
4-[[5-[(Methoxyamino)carbonyl]-2 methylphenyl]amino]-5-methyl-N-(2-pyridinylmethyl)pyrrolo[2,1-f][1,2,4]triazine-6-carboxamide;
4-[[5-[(Methoxyamino)carbonyl]-2-methylphenyl]amino]-5-methyl-N-(Phenylmethyl)pyrrolo[2,1-f][1,2,4]triazine-6-carboximide;
4-[[5-[(Methoxyamino)carbonyl]-2-methylphenyl]amino]-5-methyl-N-(4-methyl-2-thiazolyl)pyrrolo[2,1-f][1,2,4]triazine-6-carboxamide;
4-[[5-[(Methoxyamino)carbonyl]-2-methylphenyl]amino]-5-methyl-N-[(1R)-1-methylpropyl]pyrrolo[2,1-f][1,2,4]triazine-6-carboxamide;
4-[[5-[(Methoxyamino)carbonyl]-2-methylphenyl]amino-5-methyl-N-[(1S)-1-methylpropyl]pyrrolo[2,1-f][1,2,4]triazine-6-carboxamide;
N-[(3-Fluorophenyl)methyl]-4-[[5-[(methoxyamino)carbonyl]-2-methylphenyl]amino]-5-methylpyrrolo[2,1-f][1,2,4]triazine-6-carboxamide;
N-[1-(4-Fluorophenyl)ethyl]-4-[[5-[(methoxyamino)carbonyl]-2-methylphenyl]amino]-5-methylpyrrolo[2,1-f][1,2,4]triazine-6-carboxamide;
N-[(2,4-Difluorophenyl)methyl]-4-[[5-[(methoxyamino)carbonyl]-2-methylphenyl]amino]-5-methylpyrrolo[2,1-f][1,2,4]triazine-6-carboxamide; and
N-[(2,6-Difluorophenyl)methyl]-4-[[5-[(methoxyamino)carbonyl]-2-methylphenyl]amino]-5-methylpyrrolo[2,1-f][1,2,4]triazine-6-carboxamide;
4-[[5-[[(4-Cyanophenyl)amino]carbonyl]-2-methylphenyl]amino]-*N*-ethyl-5-methylpyrrolo[2,1-*f*][1,2,4]triazine-6-carboxamide; 4-[[5-[[(4-Cyanophenyl)amino]carbonyl]-2-methylphenyl]amino]-5-methyl-N-[(1S)-1-phenylethyl]pyrrolo[2,1-*f*][1,2,4]triazine-6-carboxamide; 4-[[5-[[[(4-Cyanophenyl)amino]carbonyl]amino]-2-methylphenyl]amino]-5-methyl-*N-*propylpyrrolo[2,1-f][1,2,4]triazine-6-carboxamide;
or is (ii) a pharmaceutically-acceptable salt, hydrate, or prodrug thereof.

19. A compound according to claim 7 which is selected from N-Ethyl-4-[[5-[(methoxyamino)carbonyl]-2-methylphenyl]amino]-5 methylpyrrolo[2,1-f][1,2,4]triazine-6-carboxamide; 4-[[5-[(Methoxyamino)carbonyl]-2-methylphenyl]amino]-5-methyl-N-(1-methylethyl)pyrrolo[2,1-f][1,2,4]triazine-6-carboxamide; 4-[[5-[(Methoxyamino)carbonyl]-2-methylphenyl]amino]-5-methyl-N-propylpyrrolo[2,1-f][1,2,4]triazine-6-carboxamide; 4-[[5-[(Methoxyamino)carbonyl]-2-methylphenyl]amino]-5-methyl-N-[1-phenylethyl]pyrollo[2,1-f][1,2,4]triazine-6-carboxamide; and 4-[[5-[(Methoxyamino)carbonyl]-2-methylphenyl]amino]-5-methyl-N-[1-methylpropyl[2,1-f][1,2,4]triazine-6-carboxamide; and a pbarmaceutically-acceptable salt thereof.

20. A compound having the formula (II): 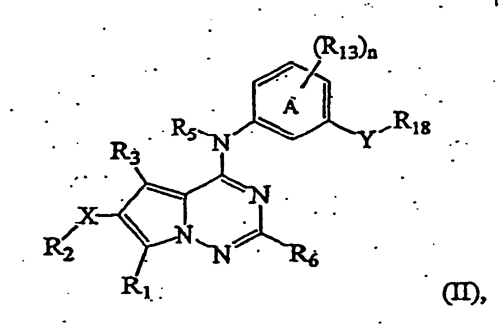 or a pharmaceutically acceptable salt, prodrug or solvate thereof, wherein
R₃ is methyl or CF₃;
R₅ is hydrogen or lower alkyl;
Y is -C(=O)NR₂₃-,-NR₂₃C(=O)NR₂₃-, -NR₂₃SO₂-, or -SO₂NH₂-;
R₁₈ and R₂₃ are selected from hydrogen, alkyl, alkoxy, aryl, and aryl substituted with one to three R₁₉, except when Y is -NR₂₃SO₂-, R₁₈ is C₁₋₄alkyl or aryl optionally substituted with one to three R₁₉;
X is selected from -O-, -OC(=O)-, -S-, -S(=O)-, -SO₂-, -C(=O)-, -CO₂-, -NR₁₀-, -NR₁₀C(=O)-, -NR₁₀C(=O)-NR₁₁-, -NR₁₀CO₂-, -NR₁₀SO₂-, -NR₁₀SO₂NR₁₁-, -SO₂NR₁₀-, and -C(=O)NR₁₀-, or X is absent;
R₁ is hydrogen, -CH₃-, OH, -OCH₃, -SH, -SCH₃, -OC(=O)R₂₁, -S(=O)R₂₂, -SO₂R₂₂, -SO₂NR₂₄R₂₅, -CO₂R₂₁, -C(=O)NR₂₄R₂₅, -NH₂, -NR₂₁SO₂NR₂₄R₂₅, -NR₂₁SO₂R₂₂, -NR₂₄C(=O)R₂₅, -NR₂₄CO₂R₂₅, -NR₂₁C(=O)NR₂₄R₂₅, halogen, nitro, or cyano;
R₂ is alkyl, substituted alkyl alkenyl, substituted alkenyl, alkynyl, substituted alkynyl, aryl, substituted aryl, heterocyclo, substituted heterocyclo, aralkyl, substituted aralkyl, heterocycloalkyl, or substituted heterocycloalkyl;
R₆ is hydrogen, alkyl, substituted alkyl, aryl, substituted aryl, heterocydo, substituted heterocyclo, -NR₇R₈,-OR₇, or halogen;
R₁₃ and R₁₉ at each occurrence are independently selected from alkyl, halo, trifluoromethoxy, trifluoromethyl, hydroxy, alkoxy, alkanoyl, alkanoyloxy, thiol, alkylthio, ureido, nitro, cyano, carboxy, carboxyalkyl, carbamyl; alkoxycarbonyl, alkylthiono, arylthion arylsulfonylamine, C₁₋₄-alkylsulfonylamine; sulfonic acid, alkylsulfonyl, sulfonamido, and aryloxy, wherein each group R₁₃ and R₁₉ may be further substituted by hydroxy, alkyl, alkoxy, aryl, or aralkyl;
R₇, R₈, R₂₁, R₂₄, and R₂₅ are independently selected from hydrogen, alkyl, substituted alkyl, aryl, substituted aryl, heterocyclo, and substituted heterocyclo;
R₂₂ is alkyl, substituted alkyl, aryl, substituted aryl, heterocyclo, or substituted heterocyclo; and
*n* is 0, 1 or 2.

21. A compound according to claim 20, having the formula: 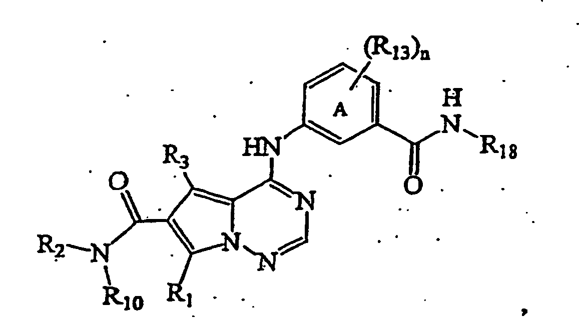 or a pharmaceutically acceptable salt, prodrug or solvate thereof; wherein.
R₁₈ is alkoxy, aryl or aryl substituted with R₁₉;
R₁ and R₁₀ are hydrogen or -CH₃; and
R₁₃ is lower alkyl, halogen, trifluoromethoxy, trifluoromethyl, hydroxy, C₁₋₄alkoxy, nitro, or cyano.

22. A compound according to claim 20 having the formula: 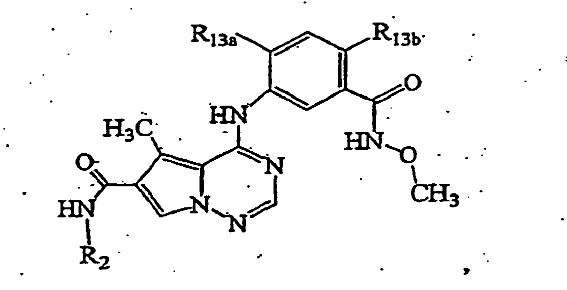 or a pharmaceutically-acceptable salt, hydrate or prodrug thereof, in which R₂ is a straight or branched C₂₋₆alkyl or optionally-substituted benzyl, and R₁₃ₐ and R_{13b} are selected from hydrogen, C₁₋₄alkyl, hydroxy, halogen, cyano, and trifluoromethyl.

23. A compound according to claim 20 which is N-Ethyl-4-[[5-[(methoxyamino)carbonyl]-2-methylphenyl]amino]-5-methylpyrrolo[2,1-f][1,2,4]triazine-6-carboxamide; or a pharmaceutically acceptable salt comprising a methanesulfonic salt thereof.

24. A compound according to claim 20 which is 4-[[5-[(Methoxyamino)carbonyl]-2-methylphenyl]amino]-5-methyl-N-(1-methylethyl)pyrrolo [2,1-f][1,2,4]triazine-6-carboxamide; or a pharmaceutically acceptale salt thereof.

25. A compound according to claim 20 which is 4-[[5-[(Methoxyamino)carbonyl]-2-methylphenyl]amino]-5-methyl-N-propylpyrrolo[2,1-f][1,2,4]triazine-6-carboxamide; or a pharmaceutically acceptable salt thereof.

26. A compound according to claim 20 which is 4-[[6-[(Methoxyamino)carbonyl]-2-methylphenyl]amino]-5-methyl-N-[1-phenylethyl]pyrrolo[2,1-f][1,2,4]triazine-6-carboxamide; or a pharmaceutically acceptable salt thereof.

27. A compound according to claim 20 which is 4-[[5-[(Methoxyamino)carbonyl]-2-methylphenyl]amino]-5-methyl-N-[1-methylpropyl]pyrrolo[2,1-f][1,2,4]triazine-6-carboxamide; or a pharmaceutically-acceptable salt thereof.

28. A pharmaceutical composition comprising at least one compound according to any of claims 7 to claim 27 and a pharmareutically-acceptable carrier or diluent.

29. Use of a pharmaceutical composition according to claim 28 for the manufacture of a medicament for treating an inflammatory disorder.

30. Use according to claim 29 in which the inflammatory disorder is selected from asthma, adult respiratory distress syndrome, chronic obstructive pulmonary disease, chronic pulmonary inflammatory disease, diabetes, inflammatory bowel disease, osteoporosis, psoriasis, graft vs. host rejection, atherosclerosis, and arthritis including rheumatoid arthritis, psoriatic arthritis, traumatic arthritis, rubella arthritis, gouty arthritis and osteoarthritis.

## Patentansprüche

1. Verwendung mindestens einer Verbindung der Formel (I): 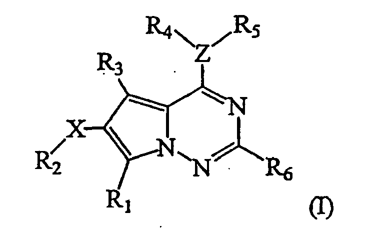 oder eines pharmazeutisch verträglichen Salzes, Prodrugs oder Solvats davon, wobei:
R₃ Wasserstoff, Methyl, Perfluormethyl, Methoxy, Halogen, Cyano oder NH₂ ist;
X ausgewählt ist aus -O-, -OC(=O)-, -S-, -S(=O)-, -SO₂-, -C(=O)-, -CO₂-, -NR₁₀-, -NR₁₀C(=O)-, -NR₁₀C(=O)NR₁₁-, -NR₁₀CO₂-, -NR₁₀SO₂-, -NR₁₀SO₂NR₁₁-, -SO₂NR₁₀-, -C(=O)NR₁₀-, Halogen, Nitro und Cyano oder X abwesend ist;
Z ausgewählt ist aus O, S, N und CR₂₀, wobei wenn Z gleich CR₂₀ ist, das Kohlenstoffatom ein gegebenenfalls substituiertes bicyclisches Aryl oder Heteroaryl mit R₄ und R₅ bilden kann;
R₁ Wasserstoff, -CH₃, -OH, -OCH₃, -SH, -SCH₃, -OC(=O)R₂₁, -S(=O)R₂₂, -SO₂R₂₂, -SO₂NR₂₄R₂₅, -CCO₂R₂₁, -C(=O)NR₂₄R₂₅, -NH₂, -NR₂₄R₂₅, -NR₂₁SO₂NR₂₄R₂₅, -NR₂₁SO₂R₂₂, -NR₂₄C(=O)R₂₅, -NR₂₄CO₂R₂₅, -NR₂₁C(=O)NR₂₄R₂₅, Halogen, Nitro oder Cyano ist;
R₂ ausgewählt ist aus:
a) Wasserstoff, mit der Maßgabe, dass R₂ nicht Wasserstoff ist, wenn X gleich -S(=O)--, -SO₂-, -NR₁₀CO₂- oder -NR₁₀SO₂-;
b) Alkyl, Alkenyl und Alkinyl, gegebenenfalls substituiert mit bis zu vier R₂₆, oder Pentafluoralkyl,
c) Aryl und Heteroaryl, gegebenenfalls substituiert mit bis zu drei R₂₇; und
d) einem Heterocyclus und Cycloalkyl, gegebenenfalls substituiert mit Keto (=O), bis zu drei R₂₇ und/oder mit einer Kohlenstoff-Kohlenstoff-Brücke mit 3 bis 4 Kohlenstoffatomen; oder
e) R₂ abwesend ist, wenn X Halogen, Nitro oder Cyano ist;
(i) R₄ substituiertes Aryl, Aryl, substituiert mit NHSO₂-Alkyl, substituiertes Heteroaryl oder ein gegebenenfalls substituierter bicyclischer 7- bis 11-gliedriger gesättigter oder ungesättigter carbocyclischer oder heterocyclischer Ring ist; und
R₅ Wasserstoff, Alkyl oder substituiertes Alkyl ist, mit der Ausnahme, dass wenn Z gleich O oder S ist, dann R₅ abwesend ist;
oder alternativ
(ii) R₄ und R₅ zusammengenommen mit Z ein gegebenenfalls substituiertes bicyclisches 7- bis 11-gliedriges Aryl oder Heteroaryl bilden;
R₆ Wasserstoff, Alkyl, substituiertes Alkyl, Aryl, substituiertes Aryl, ein Heterocyclus, substituierter Heterocyclus, -NR₇R₈, -OR₇ oder Halogen ist;
R₁₀ und R₁₁ unabhängig voneinander ausgewählt sind aus Wasserstoff, Alkyl, substituiertem Alkyl, Aryl, substituiertem Aryl, Cycloalkyl, substituiertem Cycloalkyl, einem Heterocyclus und substituiertem Heterocyclus;
R₇, R₈, R₂₁, R₂₄ und R₂₅ unabhängig voneinander ausgewählt sind aus Wasserstoff, Alkyl, substituiertem Alkyl, Aryl, substituiertem Aryl, einem Heterocyclus und substituiertem Heterocyclus;
R₂₀ Wasserstoff, Niederalkyl oder substituiertes Alkyl ist oder R₂₀ abwesend sein kann, wenn das Kohlenstoffatom, an das es zusammen mit R₄ und R₅ gebunden ist, Teil eines ungesättigten bicyclischen Aryls oder Heteroaryls ist;
R₂₂ Alkyl, substituiertes Alkyl, Aryl, substituiertes Aryl, ein Heterocyclus oder substituierter Heterocyclus ist;
R₂₆ ausgewählt ist aus Halogen, Trifluormethyl, Halogenalkoxy, Keto (=O), Nitro, Cyano, -SR₂₈, -OR₂₈, -NR₂₈R₂₉, -NR₂₈SO₂, -NR₂₈SO₂R₂₉, -SO₂R₂₈, -SO₂NR₂₈R₂₉, -CO₂R₂₈, -C(=O)R₂₈, -C(=O)NR₂₈R₂₉, -OC(=O)R₂₈, -OC(=O)NR₂₈R₂₉, -NR₂₈C(=O)R₂₉, -NR₂₈CO₂R₂₉, =N-OH, =N-O-Alkyl; Aryl, gegebenenfalls substituiert mit ein bis drei R₂₇; Cycloalkyl, gegebenenfalls substituiert mit Keto (=O), ein bis drei R₂₇, oder mit einer Kohlenstoff-Kohlenstoff-Brücke mit 3 bis 4 Kohlenstoffatomen; und einem Heterocyclus, gegebenenfalls substituiert mit Keto (=O), ein bis drei R₂₇, oder mit einer Kohlenstoff-Kohlenstoff-Brücke mit 3 bis 4 Kohlenstoffatomen; wobei R₂₈ und R₂₉ jeweils unabhängig voneinander ausgewählt sind aus Wasserstoff, Alkyl Alkenyl, Aryl, Aralkyl, C₃₋₇-Cycloalkyl und einem C₃₋₇-Heterocyclus, oder zusammengenommen einen C₃₋₇-Heterocyclus bilden können; und wobei jeder R₂₈ und R₂₉ wiederum gegebenenfalls mit bis zu zwei aus Alkyl, Alkenyl, Halogen, Halogenalkyl, Halogenalkoxy, Cyano, Nitro, Amino, Hydroxy, Alkoxy, Alkylthio, Phenyl, Benzyl, Phenyloxy und Benzyloxy substituiert ist; und
R₂₇ ausgewählt ist aus Alkyl, R₃₂ und C₁₋₄-Alkyl, substituiert mit ein bis drei R₃₂, wobei jeder Rest R₃₂ unabhängig voneinander ausgewählt ist aus Halogen, Halogenalkyl, Halogenalkoxy, Nitro, Cyano, -SR₃₀, -OR₃₀, -NR₃₀R₃₁, -NR₃₀SO₂, -NR₃₀SO₂R₃₁, -SO₂R₃₀, -SO₂NR₃₀R₃₁, -CO₂R₃₀, -C(=O)R₃₀, -C(=O)NR₃₀R₃₁, -OC(=O)R₃₀, -OC(=O)NR₃₀R₃₁, -NR₃₀C(=O)R₃₁, -NR₃₀CO₂R₃₁ und einem 3- bis 7-gliedrigen carbocyclischen oder heterocyclischen Ring, gegebenenfalls substituiert mit Alkyl, Halogen, Hydroxy, Alkoxy, Halogenalkyl, Halogenalkoxy, Nitro, Amino oder Cyano, wobei R₃₀ und R₃₁ jeweils unabhängig voneinander ausgewählt sind aus Wasserstoff, Alkyl, Alkenyl, Aryl, Aralkyl, C₃₋₇-Cycloalkyl und einem Heterocyclus, oder zusammengenommen einen C₃₋₇-Heterocyclus bilden können;
zur Herstellung eines Medikaments zur Behandlung eines oder mehrerer Zustände, die mit p38-Kinase-Aktivität in Zusammenhang stehen.

2. Verwendung nach Anspruch 1, wobei:
R₃ Methyl, -CF₃ oder-OCH₃ ist;
X ausgewählt ist aus -C(=O)-, -CO₂-, -NR₁₀-, -NR₁₀C(=O)-, -NR₁₀CO₂-, -NR₁₀SO₂-, -SO₂NR₁₀- und -C(=O)NR₁₀-, oder X abwesend ist;
Z gleich N ist;
R₂ Wasserstoff, C₂₋₆-Alkyl, C₁₋₄-Alkyl, substituiert mit bis zu vier R₂₆, Pentafluoralkyl oder Aryl oder Heteroaryl, gegebenenfalls substituiert mit bis zu zwei R₂₇, ist;
R₄ Phenyl, substituiert mit einem R₁₂ und null bis drei R₁₃, ist;
R₅ und R₁₀ unabhängig voneinander ausgewählt sind aus Wasserstoff und Niederalkyl;
R₁₂ Carbamyl, Sulfonamido, Arylsulfonylamin oder Ureido ist, von denen jedes gegebenenfalls mit bis zu zwei aus Hydroxy, Alkyl, substituiertem Alkyl, Alkoxy, Aryl, substituiertem Aryl und Aralkyl oder Alkylsulfonylamin substituiert ist;
R₁₃ bei jedem Vorkommen unabhängig voneinander ausgewählt ist aus Alkyl, substituiertem Alkyl, Halogen, Trifluormethoxy, Trifluormethyl, -OR₁₄, -C(=O)-Alkyl, -OC(=O)-Alkyl, -NR₁₅R₁₆, -SR₁₅, -NO₂, -CN, -CO₂R₁₅, -CONH₂, -SO₃H, -S(=O)-Alkyl, -S(=O)-Aryl, -NHSO₂-Aryl-R₁₇, -NHSO₂-Alkyl, -SO₂NHR₁₇, -CONHR₁₇ und -NHC(=O)NHR₁₇;
R₁₄ Wasserstoff, Alkyl oder Aryl ist;
R₁₅ Wasserstoff oder Alkyl ist;
R₁₆ Wasserstoff, Alkyl, Aralkyl oder Alkanoyl ist; und
R₁₇ Wasserstoff, Hydroxy, Alkyl, substituiertes Alkyl, Alkoxy, Aryl, substituiertes Aryl oder Aralkyl ist.

3. Verwendung mindestens einer Verbindung der Formel (I): 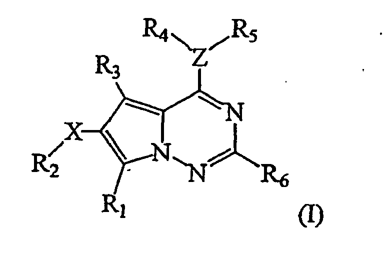 oder eines pharmazeutisch verträglichen Salzes, Prodrugs oder Solvats davon, wobei:
R₃ Wasserstoff, Methyl, Perfluormethyl, Methoxy, Halogen, Cyano oder NH₂ ist;
X ausgewählt ist aus -O-, -OC(=O)-, -S-, -S(=O)-, -SO₂-, -C(=O)-, -CO₂-, -NR₁₀-, -NR₁₀C(=O)-, -NR₁₀C(=4)NR₁₁-, -NR₁₀CO₂-, -NR₁₀SO₂-, -NR₁₀SO₂NR₁₁-, -SO₂NR₁₀-, -C(=O)NR₁₀-, Halogen, Nitro und Cyano, oder X abwesend ist;
Z gleich O, S, N oder CR₂₀ ist;
R₁ Wasserstoff, -CH₃, -OH, -OCH₃, -SH, -SCH₃, -OC(=O)R₂₁, -S(=O)R₂₂, -SO₂R₂₂, -SO₂NR₂₄R₂₅, -CO₂R₂₁, -C(=O)NR₂₄R₂₅, -NH₂, -NR₂₁SO₂NR₂₄R₂₅, -NR₂₁SO₂R₂₂, -NR₂₄C(=O)R₂₅, -NR₂₄CO₂R₂₅, -NR₂₁C(=O)NR-₂₄R₂₅, Halogen, Nitro oder Cyano ist;
R₂ Wasserstoff, Alkyl, substituiertes Alkyl, Alkenyl, substituiertes Alkenyl, Alkinyl, substituiertes Alkinyl, Aryl, substituiertes Aryl, ein Heterocyclus, substituierter Heterocyclus, Aralkyl, substituiertes Aralkyl oder Heterocycloalkyl oder substituiertes Heterocycloalkyl ist, oder wenn X Halogen, Nitro oder Cyano ist, R₂ abwesend ist, mit der Maßgabe, dass R₂ nicht Wasserstoff ist, wenn X gleich -S(=O)-, -SO₂-, -NR₁₀CO₂₋oder -NR₁₀SO₂- ist,
R₄ substituiertes Aryl, Aryl, substituiert mit NHSO₂-Alkyl, substituiertes Heteroaryl oder ein gegebenenfalls substituiertes bicyclisches 7- bis 11-gliedriges gesättigtes oder ungesättigtes carbocyclisches oder heterocyclisches Ringsystem ist;
R₅ Wasserstoff, Alkyl oder substituiertes Alkyl ist, mit der Ausnahme, dass wenn Z gleich O oder S ist, R₅ abwesend ist;
R₆ Wasserstoff, Alkyl, substituiertes Alkyl, Aryl, substituiertes Aryl, ein Heterocyclus, substituierter Heterocyclus, -NR₇R₈, -OR₇ oder Halogen ist;
R₇, R₈, R₁₀, R₁₁, R₂₁, R₂₄ und R₂₅ unabhängig voneinander ausgewählt sind aus Wasserstoff, Alkyl, substituiertem Alkyl, Aryl, substituiertem Aryl, einem Heterocyclus und substituiertem Heterocyclus;
R₂₀ Wasserstoff, Niederalkyl oder substituiertes Alkyl ist; und
R₂₂ Alkyl, substituiertes Alkyl, Aryl, substituiertes Aryl, ein Heterocyclus oder substituierter Heterocyclus ist;
zur Herstellung eines Medikaments zur Behandlung eines oder mehrerer Zustände, die mit p38-Kinase-Aktivität in Zusammenhang stehen.

4. Verwendung nach Anspruch 3, wobei R₄ und R₅ zusammengenommen mit Z 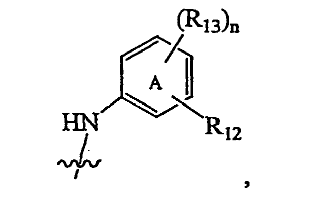 bilden;
R₁₂ an ein beliebiges verfügbares Kohlenstoffatom des Phenylrings A gebunden ist und ausgewählt ist aus Carbamyl, Sulfonamido, Arylsulfonylamin und Ureido, von denen jeder gegebenenfalls mit bis zu einem aus Hydroxy, Alkyl, substituiertem Alkyl, Alkoxy, Aryl, substituiertem Aryl und Aralkyl oder C₁₋₄-Alkylsulfonylamin substituiert ist;
R₁₃ an jedes beliebige verfügbare Kohlenstoffatom des Phenylrings A gebunden ist und bei jedem Vorkommen unabhängig voneinander ausgewählt ist aus Alkyl, substituiertem Alkyl, Halogen, Trifluormethoxy, Trifluormethyl, -OR₁₄, -C(=O)-Alkyl, -OC(=O)-Alkyl, -NR₁₅R₁₆, -SR₁₅, -NO₂, -CN, -CO₂R₁₅, -CONH₂, -SO₃H, -S(=O)-Alkyl, -S(=O)-Aryl, -NHSO₂-Aryl-R₁₇, -NHSO₂-C₁₋₄-Alkyl, -SO₂NHR₁₇, -CONHR₁₇ und -NHC(=O)NHR₁₇;
R₁₄ Wasserstoff, Alkyl oder Aryl ist;
R₁₅ Wasserstoff oder Alkyl ist;
R₁₆ Wasserstoff, Alkyl, Aralkyl oder Alkanoyl ist; und
R₁₇ Wasserstoff, Hydroxy, Alkyl, substituiertes Alkyl, Alkoxy, Aryl, substituiertes Aryl oder Aralkyl ist; und
n gleich 0, 1, 2 oder 3 ist.

5. Verwendung nach Anspruch 3, wobei die mindestens eine Verbindung die Formel (II) aufweist: 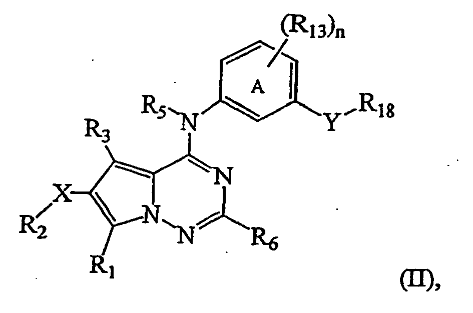
wobei:
R₃ Methyl oder CF₃ ist;
X gleich -C(=O)NR₁₀-, -NR₁₀C(=O)-, -C(=O)- oder -CO₂- ist;
R₁ Wasserstoff, -CH₃, -OH, -OCH₃, Halogen, Nitro oder Cyano ist;
Y gleich -C(=O)NH-, -NHC(=O)NH-, -NHSO₂- oder -SO₂NH- ist;
R₁₀ Wasserstoff oder Niederalkyl ist;
R₁₈ ausgewählt ist aus Wasserstoff, Alkyl, Alkoxy, Aryl und Aryl, substituiert mit ein bis drei R₁₉, mit der Ausnahme, dass wenn Y gleich -NHSO₂- ist, R₁₈ C₁₋₄-Alkyl, Aryl oder Aryl, substituiert mit R₁₉, ist;
R₁₃ an jedes beliebige verfügbare Kohlenstoffatom des Phenylrings A gebunden ist und bei jedem Vorkommen unabhängig voneinander ausgewählt ist aus Alkyl, substituiertem Alkyl, Halogen, Trifluormethoxy, Trifluormethyl, -OR₁₄, -C(=O)-Alkyl, -OC(=O)-Alkyl, -NR₁₅R₁₆, -SR₁₅, -NO₂, -CN, -CO₂R₁₅, -CONH₂, -SO₃H, -S(=O)-Alkyl, -S(=O)-Aryl, -NHSO₂-Aryl-R₁₇, -NHSO₂-C₁₋₄-Alkyl, -SO₂NHR₁₇, -CONHR₁₇ und -NHC(=O)NHR₁₇;
R₁₄, R₁₅, R₁₆ und R₁₇ Wasserstoff oder Alkyl sind;
R₁₉ bei jedem Vorkommen ausgewählt ist aus Alkyl, Halogen, Trifluormethoxy, Trifluormethyl, Hydroxy, Alkoxy, Alkanoyl, Alkanoyloxy, Thiol, Alkylthio, Ureido, Nitro, Cyano, Carboxy, Carboxyalkyl, Carbamyl, Alkoxycarbonyl, Alkylthiono, Arylthiono, Arylsulfonylamin, Sulfonsäure, Alkylsulfonyl, Sulfonamido und Aryloxy, wobei jeder Rest R₁₉ weiter mit Hydroxy, Alkyl, Alkoxy, Aryl oder Aralkyl substituiert sein kann; und
n 0, 1 oder 2 ist.

6. Verwendung nach Anspruch 3, wobei die mindestens eine Verbindung die Formel (Ia), (Ib) oder (Ic) aufweist: 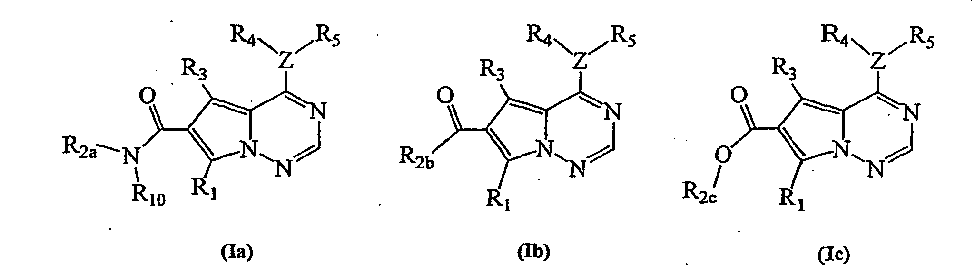
wobei:
R₃ Methyl oder CF₃ ist;
R₂ₐ und R_{2c} unabhängig voneinander ausgewählt sind aus Wasserstoff, C₂₋₆-Alkyl, substituiertem C₁₄-Alkyl, Aryl, substituiertem Aryl, Benzyl und substituiertem Benzyl;
R_{2b} ein Heterocyclus oder substituierter Heterocyclus ist; und
R₁₀ Wasserstoff oder Niederalkyl ist.

7. Verbindung der Formel (II): 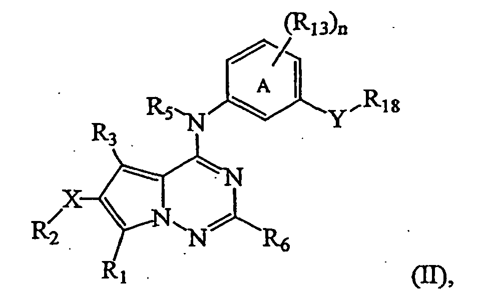 oder ein pharmazeutisch verträgliches Salz, Prodrug oder Solvat davon, wobei:
R₃ Methyl, -CF₃ oder -OCH₃ ist; R₅ Wasserstoff oder Alkyl ist;
Y gleich -C(=O)NR₂₃-, -NR₂₃C(=O)NR₂₃-, -SO₂NR₂₃ oder -NR₂₃SO₂- ist;
R₁₈ und R₂₃ ausgewählt sind aus Wasserstoff, Alkyl, Alkoxy, Aryl und Aryl, substituiert mit ein bis drei R₁₉, mit der Ausnahme, dass wenn Y gleich -NR₂₃SO₂- ist, R₁₈ C₁₋₄₋Alkyl oder Aryl, gegebenenfalls substituiert mit ein bis drei R₁₉, ist;
X ausgewählt ist aus -O-, -OC(=O)-, -S-, -S(=O)-, -SO₂-, -C(=O)-, -CO₂-, -NR₁₀-, -NR₁₀C(=O)-, -NR₁₀C(=O)NR₁₁-, -NR₁₀CO₂-, -NR₁₀SO₂-, -NR₁₀SO₂NR₁₁-, -SO₂NR₁₀- und -C(=O)NR₁₀-, oder X abwesend ist;
R₁ Wasserstoff, -CH₃, -OH, -OCH₃, -SH, -SCH₃, -OC(=O)R₂₁, -S(=O)R₂₂, -SO₂R₂₂, -SO₂NR₂₄R₂₅, -CO₂R₂₁, -C(=O)NR₂₄R₂₅, -NH₂, -NR₂₄R₂₅, -NR₂₁SO₂NR₂₄R₂₅, -NR₂₁SO₂R₂₂, -NR₂₄C(=O)R₂₅, -NR₂₄CO₂R₂₅, -NR₂₁C(=O)NR₂₄R₂₅, Halogen, Nitro oder Cyano ist;
R₂ ausgewählt ist aus
a) Wasserstoff, mit der Maßgabe, dass R₂ nicht Wasserstoff ist, wenn X gleich -S(=O)-, -SO₂-, -NR₁₀CO₂- oder -NR₁₀SO₂- ist;
b) Alkyl, Alkenyl und Alkinyl, gegebenenfalls substituiert mit bis zu vier R₂₆, oder Pentafluoralkyl;
c) Aryl und Heteroaryl, gegebenenfalls substituiert mit bis zu drei R₂₇; und
d) einem Heterocyclus und Cycloalkyl, gegebenenfalls substituiert mit Keto (=O), bis zu drei R₂₇ und/oder mit einer Kohlenstoff-Kohlenstoff-Brücke mit 3 bis 4 Kohlenstoffatomen;
R₆ Wasserstoff, Alkyl, substituiertes Alkyl, Aryl, substituiertes Aryl, ein Heterocyclus, substituierter Heterocyclus, -NR₇R₈, -OR₇ oder Halogen ist;
R₁₀ Wasserstoff oder Alkyl ist;
R₁₃ und R₁₉ bei jedem Vorkommen unabhängig voneinander ausgewählt sind aus Alkyl, Halogen, Trifluormethoxy, Trifluormethyl, Hydroxy, Alkoxy, Alkanoyl, Alkanoyloxy, Thiol, Alkylthio, Ureido, Nitro, Cyano, Carboxy, Carboxyalkyl, Carbamyl, Alkoxycarbonyl, Alkylthiono, Arylthiono, Arylsulfonylamin, C₁₋₄-Alkylsulfonylamin, Sulfonsäure, Alkylsulfonyl, Sulfonamido und Aryloxy, wobei jeder Rest R₁₃ und/oder R₁₉ weiter mit Hydroxy, Alkyl, substituiertem Alkyl, Alkoxy, Aryl oder Aralkyl substituiert sein kann;
R₇, R₈, R₂₁, R₂₄ und R₂₅ unabhängig voneinander ausgewählt sind aus Wasserstoff, Alkyl, substituiertem Alkyl, Aryl, substituiertem Aryl, einem Heterocyclus und einem substituierten Heterocyclus;
R₂₂ Alkyl, substituiertes Alkyl, Aryl, substituiertes Aryl, ein Heterocyclus oder substituierter Heterocyclus ist;
R₂₆ ausgewählt ist aus Halogen, Trifluormethyl, Halogenalkoxy, Keto (=O), Nitro, Cyano, -SR₂₈, -OR₂₈, -NR₂₈R₂₉, -NR₂₈SO₂, -NR₂₈SO₂R₂₉, -SO₂R₂₈, -SO₂NR₂₈R₂₉, -CO₂R₂₈, -C(=O)R₂₈, -C(=O)NR₂₈R₂₉, -OC(=O)R₂₈, -OC(=O)NR₂₈R₂₉, -NR₂₈C(=O)R₂₉, -NR₂₈CO₂R₂₉, =N-OH, =N-O-Alkyl; Aryl, gegebenenfalls substituiert mit ein bis drei R₂₇; Cycloalkyl, gegebenenfalls substituiert mit Keto (=O), ein bis drei R₂₇, oder mit einer Kohlenstoff-Kohlenstoff-Brücke mit 3 bis 4 Kohlenstoffatomen; und einem Heterocyclus, gegebenenfalls substituiert mit Keto (=O), ein bis drei R₂₇ oder mit einer Kohlenstoff-Kohlenstoff-Brücke mit 3 bis 4 Kohlenstoffatomen; wobei R₂₈ und R₂₉ jeweils unabhängig voneinander ausgewählt sind aus Wasserstoff, Alkyl, Alkenyl, Aryl, Aralkyl, C₃₋₇-Cycloalkyl und einem C₃₋₇-Heterocyclus oder zusammengenommen einen C₃₋₇-Heterocyclus bilden können; und wobei jeder Rest R₂₈ und R₂₉ wiederum gegebenenfalls substituiert ist mit bis zu zwei aus Alkyl, Alkenyl, Halogen, Halogenalkyl, Halogenalkoxy, Cyano, Nitro, Amino, Hydroxy, Alkoxy, Alkylthio, Phenyl, Benzyl, Phenyloxy und Benzyloxy;
R₂₇ ausgewählt ist aus Alkyl, R₃₂ und C₁₋₄-Alkyl, substituiert mit ein bis drei R₃₂, wobei jeder Rest R₃₂ unabhängig voneinander ausgewählt ist aus Halogen, Halogenalkyl, Halogenalkoxy, Nitro, Cyano, -SR₃₀, -OR₃₀, -NR₃₀R₃₁, -NR₃₀SO₂, -NR₃₀SO₂R₃₁, -SO₂R₃₀, -SO₂NR₃₀R₃₁, -CO₂R₃₀, -C(=O)R₃₀, -C(=O)NR₃₀R₃₁, -OC(=O)R₃₀, -OC(=O)NR₃₀R₃₁, -NR₃₀C(=O)R₃₁, -NR₃₀CO₂R₃₁ und einem 3- bis 7-gliedrigen carbocyclischen oder heterocyclischen Ring, gegebenenfalls substituiert mit Alkyl, Halogen, Hydroxy, Alkoxy, Halogenalkyl, Halogenalkoxy, Nitro, Amino oder Cyano, wobei R₃₀ und R₃₁ jeweils unabhängig voneinander ausgewählt sind aus Wasserstoff, Alkyl, Alkenyl, Aryl, Aralkyl, C₃₋₇-Cycloalkyl und einem Heterocyclus oder zusammengenommen einen C₃₋₇-Heterocyclus bilden können; und
n 0, 1 oder 2 ist; unter Ausschluss von 4-[[3-(Aminosulfonyl)-4-methylphenyl]amino]-5-methylpyrrolo[2,1-f][1,2,4]triazin-6-carbonsäuremethylester.

8. Verbindung nach Anspruch 7 der Formel (IIa) oder (IIb) 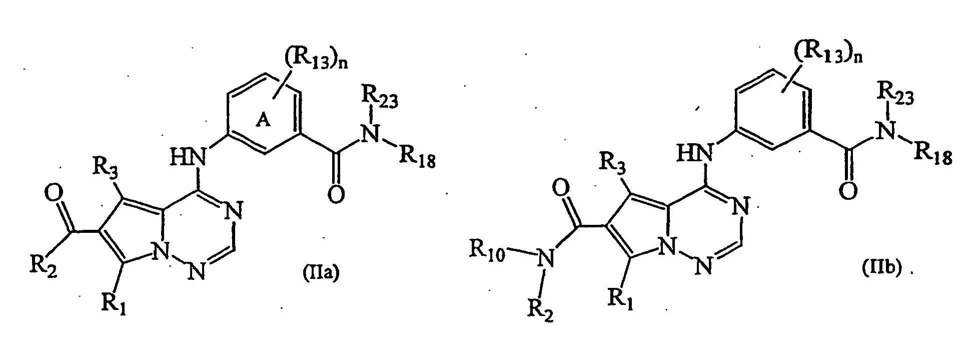 oder ein pharmazeutisch verträgliches Salz, Prodrug oder Solvat davon, wobei:
R₁ und R₁₀ Wasserstoff oder -CH₃ sind;
R₁₃ Niederalkyl, Halogen, Trifluormethoxy, Trifluormethyl, Hydroxy, C₁₋₄-Alkoxy, Nitro oder Cyano ist;
R₁₈ Hydroxy, C₁₋₄-Alkoxy, Phenyl oder Phenyl, substituiert mit ein oder zwei R₁₉, ist;
R₂₃ Wasserstoff oder Niederalkyl ist; und
n 0, 1 oder 2 ist.

9. Verbindung nach Anspruch 7 oder ein pharmazeutisch verträgliches Salz, Prodrug oder Solvat davon, wobei:
X gleich -C(=O)-, -CO₂-, -NR₁₀C(=O)- oder -C(=O)N-R₁₀- ist;
Y gleich -C(=O)NH-, -NHC(=O)NH- oder -NHSO₂- ist;
R₅ und R₁₀ Wasserstoff oder -CH₃ sind;
R₁₃ und R₁₉ bei jedem Vorkommen unabhängig voneinander ausgewählt sind aus Niederalkyl, Halogen, Trifluormethoxy, Trifluormethyl, Hydroxy, C₁₋₄-Alkoxy, Nitro und Cyano;
R₇, R₈, R₂₁, R₂₄ und R₂₅ unabhängig voneinander ausgewählt sind aus Wasserstoff und Niederalkyl;
R₂₂ Niederalkyl ist; und
n 0 oder 1 ist.

10. Verbindung nach Anspruch 7 oder ein pharmazeutisch verträgliches Salz, Prodrug oder Solvat davon, wobei X-R₂ für folgende steht: 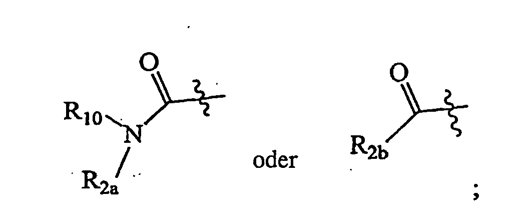
R₂ₐ ausgewählt ist aus:
a) Wasserstoff;
b) geradkettigem oder verzweigtem C₂₋₆-Alkyl;
c) Cycloalkyl, gegebenenfalls substituiert mit Keto und/oder bis zu zwei R₂₇;
d) Phenyl, gegebenenfalls substituiert mit bis zu zwei R₂₇; und
e) einem Heterocyclus, gegebenenfalls substituiert mit Keto und/oder bis zu zwei R₂₇;
f) Pentafluoralkyl oder C₁₋₄-Akyl, substituiert mit bis zu drei aus Halogen, Trifluormethyl, Cyano, OR₂₈, NR₂₈R₂₉, CO₂R₂₈, Aryl, einem Heterocyclus und/oder Cycloalkyl, wobei das Aryl, der Heterocyclus und/oder das Cycloalkyl wiederum gegebenenfalls mit bis zu zwei aus Halogen, Hydroxy, Alkoxy, Halogenalkyl, Halogenalkoxy, Nitro, Cyano und Alkyl substituiert sind; und
R_{2b} ein monocyclischer oder bicyclischer Heterocyclus ist, der gegebenenfalls mit bis zu zwei R₂₇ substituiert ist;
R₂₇ bei jedem Vorkommen unabhängig voneinander ausgewählt ist aus Wasserstoff, Alkyl, Trifluormethyl, Trifluormethoxy, Halogen, Cyano, Nitro, Amino, Hydroxy, Alkoxy, Phenyl, Benzyl, Phenyloxy und Benzyloxy; und
R₂₈ und R₂₉ bei jedem Vorkommen unabhängig voneinander ausgewählt sind aus Wasserstoff, Alkyl, Alkenyl, Phenyl und Benzyl.

11. Verbindung nach Anspruch 7 oder ein pharmazeutisch verträgliches Salz, Prodrug oder Solvat davon, wobei:
R₂ₐ ausgewählt ist aus:
a) geradkettigem oder verzweigtem C₂₋₆-Alkyl;
b) Phenyl, gegebenenfalls substituiert mit bis zu zwei aus Halogen, C₁₋₄-Alkoxy und Trifluormethyl;
c) C₃₋₆-Cycloalkyl, gegebenenfalls substituiert mit bis zu zwei C₁₋₄-Alkyl und/oder Hydroxy;
d) geradkettigem oder verzweigtem C₁₋₄-Alkyl, substituiert mit bis zu drei aus
i) Halogen,
ii) Trifluormethyl,
iii) Cyano,
iv) C₁₋₄-Alkoxy,
v) Phenyloxy,
vi) Benzyloxy,
vii) NH₂, NH(C₁₋₄-Alkyl) und/oder N(C₁₋₄-Alkyl)₂,
viii) Phenyl, wiederum gegebenenfalls substituiert mit bis zu zwei aus Halogen und/oder Methoxy,
ix) einem Heterocyclus, ausgewählt aus Pyridinyl, Indolyl, Thiophenyl, Furanyl, Thiazolyl, Thienyl, Morpholinyl, Tetrahydrofuranyl, Triazinyl, Piperazinyl, Indenyl und Piperidinyl; wobei der Heterocyclus gegebenenfalls mit ein bis zwei C₁₋₄-Alkyl substituiert ist;
x) C₃₋₆-Cycloalkyl; und
R_{2b} ein 5- bis 7-gliedriger monocyclischer Heterocyclus ist, der ausgewählt ist aus Diazepinyl, Morpholinyl, Piperidinyl und Pyrrolidinyl, wobei der Heterocyclus gegebenenfalls mit C₁₋₄-Alkyl, Phenyl und/oder Benzyl substituiert ist.

12. Verbindung nach Anspruch 7 oder ein pharmazeutisch verträgliches Salz, Prodrug oder Solvat davon, wobei R₁ und R₁₀ unabhängig Wasserstoff oder CH₃ sind.

13. Verbindung nach Anspruch 7 oder ein pharmazeutisch verträgliches Salz, Prodrug oder Solvat davon, wobei R₆ Wasserstoff ist.

14. Verbindung nach Anspruch 7 oder ein pharmazeutisch verträgliches Salz, Prodrug oder Solvat davon, wobei Y gleich -NHC(=O)NH- oder -NHSO₂- ist; R₁₈ Aryl oder Aryl, substituiert mit Alkyl, OCH₃, CF₃, Cyano oder Halogen, ist.

15. Verbindung nach Anspruch 7 oder ein pharmazeutisch verträgliches Salz, Prodrug oder Solvat davon, wobei Y gleich -C(=O)NR₂₃- ist, R₂₃ Wassserstoff oder Niederalkyl ist und R₁₈ C₁₋₄-Alkoxy oder Aryl, gegebenenfalls substituiert mit Alkyl, OCH₃, CF₃, Cyano oder Halogen, ist.

16. Verbindung nach Anspruch 7 der Formel: 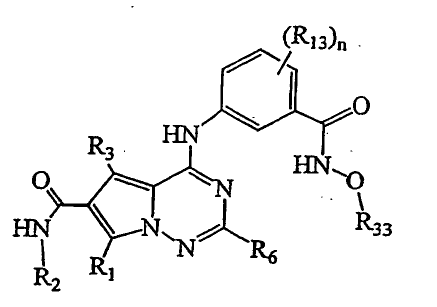 wobei R₃₃ Niederalkyl ist.

17. Verbindung nach Anspruch 16 oder ein pharmazeutisch verträgliches Salz, Prodrug oder Solvat davon, wobei R₃ und R₃₃ Methyl sind, R₁ und R₆ Wasserstoff sind und R₂ geradkettiges oder verzweigtes C₂₋₆-Alkyl oder gegebenenfalls substituiertes Benzyl ist.

18. Verbindung nach Anspruch 7, welche ausgewählt ist aus (i) N-(2,2-Dimethylpropyl)-4-[[5-[(methoxyamino)carbonyl]-2-methylphenyl]amino]-N,5-dimethylpyrrolo[2,1-f][1,2,4]triazin-6-carboxamid;
3-[[6-[(Hexahydro-4-methyl-1H-1,4-diazepin-1-yl)carbonyl]-5-methylpyrrolo[2,1 - f][1,2,4]triazin-4-yl]amino]-N-methoxy-4-methylbenzamid;
4-[[5-[(Methoxyamino)carbonyl]-2-methylphenyl]amino]-5-methyl-N-(1-methylethyl)pyrrolo[2,1-f] [1,2,4]triazin-6-carboxamid;
4-[[5-[(Methoxyamino)carbonyl]-2-methylphenyl]amino]-5-methyl-N-(2-methylpropyl)pyrrolo[2,1-f] [1,2,4]triazin-6-carboxamid;
N-(2,2-Dimethylpropyl)-4-[[5-[(methoxyamino)carbonyl]-2-methylphenyl]amino]-S-methylpyrrolo[2,1-f][1,2,4]triazin-6-carboxamid;
4-[[5-[(Methoxyamino)carbonyl]-2-methylphenyl]amino]-5-methyl-N-propylpyrrolo[2,1-f][1,2,4]triazin-6-carboxamid;
N-(1,1-Dimethylethyl)-4-[[5-[(methoxyamino)carbonyl]-2-methylphenyl]amino]-5-methylpyrrolo[2,1-f][1,2,4] triazin-6-carboxamid;
4-[[5-[(Methoxyamino)carbonyl]-2-methylphenyl]amino]-N-(2-methoxyethyl)-5-methylpyrrolo[2,1-f][1,2,4]triazin-6-carboxamid;
N-Methoxy-4-methyl-3-[[5-methyl-6-(4-morpholinylcarbonyl)pyrrolo[2,1-f] [1,2,4]triazin-4-yl]amino]benzamid;
N-Cyclohexyl-4-[[5-[(methoxyamino)carbonyl]-2-methylphenyl]amino]-5-methylpyrrolo[2,1-f] [1,2,4]triazin-6-carboxamid;
4-[[5-[(Methoxyamino)carbonyl]-2-methylphenyl]amino)-5-methyl-N-[(1R)-1-phenylethyl]pyrrolo[2,1-f] [1,2,4]triazin-6-carboxamid;
4-[[5-[(Methoxyamino)carbonyl]-2-methylphenyl]amino]-5-methyl-N-[(1S)-1-phenylethyl]pyrrolo[2,1-f] [1,2,4]triazin-6-carboxamid;
N-[(4-Fluorphenyl)methyl]-4-[[5-[(methoxyamino)carbonyl]-2-methylphenyl]amino]-5-methylpyrrolo[2,1-f][1,2,4]triazin-6-carboxamid;
4-[[5-[(Methoxyamino)carbonyl]-2-methylphenyl]amino]-N-[(2-methoxyphenyl)methyl]-5-methylpyrrolo[2,1-f][1,2,4]triazin-6-carboxamid;
4-[[5-[(Methoxyamino)carbonyl]-2-methylphenyl]amino]-5-methyl-N-(4-pyridinylmethyl)pyrrolo[2,1-f][1,2,4]triazin-6-carboxamid;
4-[[5-[(Methoxyamino)carbonyl]-2-methylphenyl]amino]-5-methyl-N-[2-(4-pyridinyl)ethyl]pyrrolo[2,1-f][1,2,4]triazin-6-carboxamid;
4-[[5-[(Methoxyamino)carbonyl]-2-methylphenyl]amino]-5-methyl-N-[2-(1-piperidinyl)ethyl]pyrrolo[2,1-f][1,2,4]triazin-6-carboxamid;
4-[[5-[(Methoxyamino)carbonyl]-2-methylphenyl]amino]-5-methyl-N-[2-(4-morpholinyl)ethyl]pyrrolo[2,1-f][1,2,4]triazin-6-carboxamid;
N-[(1R,2S)-2,3-Dihydro-1H-inden-1-yl]-4-[[5-[(methoxyamino)carbonyl]-2-methylphenyl]amino]-5-methylpyrrolo[2,1-f][1,2,4]triazin-6-carboxamid;
N-[(1S,2R)-2,3-Dihydro-1H-inden-1-yl]-4-[[5-[(methoxyamino)carbonyl]-2-methylphenyl]amino]-5-methylpyrrolo[2,1-f][1,2,4]triazin-6-carboxamid;
N-Methoxy-4-methyl-3-[[5-methyl-6-[[4-(phenylmethyl)-1-piperidinyl]carbonyl]pyrrolo[2,1-f][1,2,4]triazin-4-yl]amino]benzamid;
N-Cyclopropyl-4-[[5-[(methoxyamino)carbonyl]-2-methylphenyl]amino]-5-methylpyrrolo[2,1-f][1,2,4]triazin-6-carboxamid;
N-Cyclopentyl-4-[[5-[(methoxyamino)carbonyl]-2-methylphenyl]amino]-5-methylpyrrolo[2,1-f][1,2,4]triazin-6-carboxamid;
N-[2-(4-Fluorphenyl)ethyl]-4-([5-[(methoxyamino)carbonyl]-2-methylphenyl]amino]-5-methylpyrrolo[2,1-f][1,2,4]triazin-6-carboxamid;
N-(Cyclohexylmethyl)-4-[[5-[(methoxyamino)carbonyl]-2-methylphenyl]amino]-5-methylpyrrolo[2,1-f][1,2,4]triazin-6-carboxamid;
4-[[5-[(Methoxyamino)carbonyl]-2-methylphenyl]amino]-5-methyl-N-[(tetrahydro-2-furanyl)methyl]pyrrolo[2,1-f][1,2,4]triazin-6-carboxamid;
N-(2-1H-Indol-3-ylethyl)-4-[[5-[(methoxyamino)carbonyl]-2-methylphenyl]amino]-5-methylpyrrolo[2,1-f][1,2,4]triazin-6-carboxamid;
N-Butyl-4-[[5-[(methoxyamino)carbonyl]-2-methylphenyl]amino]-5-methylpyrrolo[2,1-f][1,2,4]triazin-6-carboxamid;
N-(Cyclopropylmethyl)-4-[[5-[(methoxyamino)carbonyl]-2-methylphenyl]amino]-5-methylpyrrolo[2,1-f][1,2,4]triazin-6-carboxamid;
4-[[5-[(Methoxyamino)carbonyl]-2-methylphenyl]amino]-5-methyl-N-(2-methylbutyl)pyrrolo[2,1-f][1,2,4]triazin-6-carboxamid;
N-(2-Furanylmethyl)-4-[[5-[(methoxyamino)carbonyl]-2-methylphenyl]amino]-5-methylpyrrolo[2,1-t][1,2,4]triazin-6-carboxamid;
4-[[5-[(Methoxyamino)carbonyl]-2-methylphenyl]amino]-5-methyl-N-(2-thienylmethyl)pyrrolo[2,1-f] [1,2,4] triazin-6-carboxamid;
4-[[5-[(Methoxyamino)carbonyl]-2-methylphenyl]amino]-5-methyl-N-(2-phenoxyethyl)pyrrolo[2,1-f][1,2,4]triazin-6-carboxamid;
4-[[5-[(Methoxyamino)carbonyl]-2-methylphenyl]amino]-5-methyl-N-(2-methylcyclohexyl)pyrrolo[2,1-f][1,2,4]triazin-6-carboxamid;
N-Ethyl-4-[[5-[(methoxyamino)carbonyl]-2-methylphenyl]amino]-N,5-dimethylpyrrolo[2,1-f][1,2,4]triazin-6-carboxamid;
4-[[5-[(Methoxyamino)carbonyl]-2-methylphenyl]amino]-5-methyl-N-(2,2,2-trifluorethyl)pyrrolo[2,1-f] [1,2,4]triazin-6-carboxamid;
N-(2-Fluorethyl)-4-[[5-[(methoxyamino)carbonyl]-2-methylphenyl]amino]-5-methylpyrrolo[2,1-f][1,2,4] triazin-6-carboxamid;
N-(2,3-Dihydro-1H-inden-2-yl)-4-[[5-[(methoxyamino)carbonyl]-2-methylphenyllaminol-5-methylpyrrolo[2,1-f][1,2,4]triazin-6-carboxamid;
N-Ethyl-4-[[5-[(methoxyamino)carbonyl]-2-methylphenyl]amino]-5-methylpyrrolo[2,1-f][1,2,4]triazin-6-carboxamid;
4-[[5-[(Methoxyamino)carbonyl]-2-methylphenyl]amino]-5-methyl-N-(2,2,3,3,3-pentafluorpropyl)pyrrolo[2,1-f] [1,2,4]triazin-6-carboxamid;
4-[[5-[(Methoxyamino)carbonyl]-2-methylphenyl]amino]-5,7-dimethyl-N-(1-methylethyl)pyrrolo[2,1-f] [1,2,4]triazin-6-carboxamid;
N-(4-Fluorphenyl)-4-[[5-[(methoxyamino)carbonyl]-2-methylphenyl]amino]-5-methylpyrrolo[2,1-f][1,2,4]triazin-6-carboxamid;
4-[[5-[(Methoxyamino)carbonyl]-2-methylphenyl]amino]-N-(2-methoxyphenyl)-5-methylpyrrolo[2,1-f][1,2,4]triazin-6-carboxamid;
4-[[5-[(Methoxyamino)carbonyl]-2-methylphenyl]amino]-N-[(3-methoxyphenyl)methyl]-5-methylpyrrolo[2,1-f][1,2,4]triazin-6-carboxamid;
4-[[5-[(Methoxyamino)carbonyl]-2-methylphenyl]amino]-5-methyl-N-[3-(trifluormethyl)phenyl]pyrrolo[2,1-f][1,2,4]triazin-6-carboxamid;
N-[(2,6-Dichlorphenyl)methyl]-4-[[5-[(methoxyamino)carbonyl]-2-methylphenyl]amino]-5-methylpyrrolo[2,1-f][1,2,4]triazin-6-carboxamid;
N-[(1S)-1-Cyano-2-phenylethyl]-4-[[5-[(methoxyamino)carbonyl]-2-methylphenyl]amino]-5-methylpyrrolo[2,1-f][1,2,4]triazin-6-carboxamid;
4-[[5-[(Methoxyamino)carbonyl]-2-methylphenyl]amino]-5-methyl-N-(2-phenylethyl)pyrrolo[2,1-f][1,2,4]triazin-6-carboxamid;
N-Methoxy-4-methyl-3-[[5-methyl-6-(1-pyrrolidinylcarbonyl)pyrrolo[2,1-f][1,2,4]triazin-4-yl]amino]benzamid;
4-[[5-[(Methoxyamino)carbonyl]-2-methylphenyl]amino]-5-methyl-N-(2-pyridinylmethyl)pyrrolo[2,1-f] [1,2,4]triazin-6-carboxamid;
4-[[5-[(Methoxyamino)carbonyl]-2-methylphenyl]amino]-5-methyl-N-(phenylmethyl)pyrrolo[2,1-f] [1,2,4]triazin-6-carboxamid;
4-[[5-[(Methoxyamino)carbonyl]-2-methylphenyl]amino]-5-methyl-N-(4-methyl-2-thiazolyl)pyrrolo[2,1-f][1,2,4]triazin-6-carboxamid;
4-[[5-[(Methoxyamino)carbonyl]-2-methylphenyl]amino]-5-methyl-N-[(1R)-1-methylpropyl]pyrrolo[2,1-f][1,2,4]triazin-6-carboxamid;
4-[[5-[(Methoxyamino)carbonyl]-2-methylphenyl]amino]-5-methyl-N-[(1S)-1-methylpropyl]pyrrolo[2,1-f][1,2,4]triazin-6-carboxamid;
N-[(3-Fluorphenyl)methyl]-4-[[5-[(methoxyamino)carbonyl]-2-methylphenyl]amino]-5-methylpyrrolo[2,1-f][1,2,4]triazin-6-carboxamid;
N-[1-(4-Fluorphenyl)ethyl]-4-[[5-[(methoxyamino)carbonyl]-2-methylphenyl]amino]-5-methylpyrrolo[2,1-f][1,2,4]triazin-6-carboxamid;
N-[(2,4-Difluorphenyl)methyl]-4-[[5-[(methoxyamino)carbonyl]-2-methylphenyl]amino]-5-methylpyrrolo[2,1-f][1,2,4]triazin-6-carboxamid; und
N-[(2,6-Difluorphenyl)methyl]-4-[[5-[(methoxyamino)carbonyl]-2-methylphenyl]amino]-5-methylpyrrolo[2,1-f] [1,2,4]triazin-6-carboxamid;
4-[[5-[[(4-Cyanophenyl)amino]carbonyl]-2-methylphenyl]amino]-N-ethyl-5-methylpyrrolo[2,1-f] [1,2,4]triazin-6-carboxamid;
4-[[5-[[(4-Cyanophenyl)amino]carbonyl]-2-methylphenyl]amino]-5-methyl-N-[(1 S)-1-phenylethyl]pyrrolo[2,1-f] [1,2,4]triazin-6-carboxamid;
4-[[5-[[[(4-Cyanophenyl)amino]carbonyl]amino]-2-methylphenyl]amino]-5-methyl-N-propylpyrrolo[2,1-f] [1,2,4] triazin-6-carboxamid;
oder (ii) ein pharmazeutisch verträgliches Salz, Hydrat oder Prodrug davon ist.

19. Verbindung nach Anspruch 7, welche ausgewählt ist aus
N-Ethyl-4-[[5-[(methoxyamino)carbonyl]-2-methylphenyl]amino]-5-methylpyrrolo[2,1-f][1,2,4]triazin-6-carboxamid;
4-[[5-[(Methoxyamino)carbonyl]-2-methylphenyl]amino]-5-methyl-N-(1-methylethyl)pyrrolo[2,1-f][1,2,4]triazin-6-carboxamid;
4-[[5-[(Methoxyamino)carbonyl]-2-methylphenyl]amino]-5-methyl-N-propylpyrrolo[2,1-f][1,2,4]triazin-6-carboxamid;
4-[[5-[(Methoxyamino)carbonyl]-2-methylphenyl]amino]-5-methyl-N-[1-phenylethyl]pyrrolo[2,1-f][1,2,4]triazin-6-carboxamid; und
4-[[5-[(Methoxyamino)carbonyl]-2-methylphenyl]amino]-5-methyl-N-[1-methylpropyl]pyrrolo[2,1-f][1,2,4]triazin-6-carboxamid; und ein pharmazeutisch verträgliches Salz davon.

20. Verbindung der Formel (II): 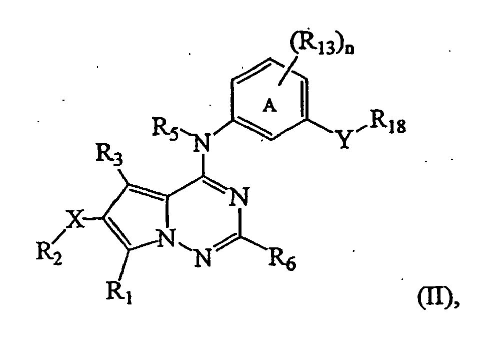 oder ein pharmazeutisch verträgliches Salz, Prodrug oder Solvat davon, wobei R₃ Methyl oder CF₃ ist;
R₅ Wasserstoff oder Niederalkyl ist;
Y gleich -C(=O)NR₂₃-, -NR₂₃C(=O)NR₂₃-, -NR₂₃SO₂- oder -SO₂NH₂- ist;
R₁₈ und R₂₃ ausgewählt sind aus Wasserstoff, Alkyl, Alkoxy, Aryl und Aryl, sustituiert mit ein bis drei R₁₉, mit der Ausnahme, dass wenn Y gleich -NR₂₃SO₂- ist, R₁₈ C₁₋₄₋Alkyl oder Aryl, gegebenenfalls substituiert mit ein bis drei R₁₉, ist;
X ausgewählt ist aus -O-, -OC(=O)-, -S-, -S(=O)-, -SO₂-, -C(=O)-, -CO₂-, -NR₁₀-, -NR₁₀C(=O)-, -NR₁₀C(=O)NR₁₁-, -NR₁₀CO₂-, -NR₁₀SO₂-, -NR₁₀SO₂NR₁₁-, -SO₂NR₁₀- und -C(=O)NR₁₀- oder X abwesend ist;
R₁ Wasserstoff, -CH₃, -OH, -OCH₃, -SH, -SCH₃, -OC(=O)R₂₁, -S(=O)R₂₂, -SO₂R₂₂, -SO₂N-R₂₄R_{25,} -CO₂R₂₁, -C(=O)NR₂₄R₂₅, -NH₂, -NR₂₁SO₂NR₂₄R₂₅, -NR₂₁SO₂R₂₂, -NR₂₄C(=O)R₂₅, -NR₂₄CO₂R₂₅, -NR₂₁C(=O)NR₂₄R₂₅, Halogen, Nitro oder Cyano ist;
R₂ Alkyl, substituiertes Alkyl, Alkenyl, substituiertes Alkenyl, Alkinyl, substituiertes Alkinyl, Aryl, substituiertes Aryl, ein Heterocyclus, substituierter Heterocyclus, Aralkyl, substituiertes Aralkyl, Heterocycloalkyl oder substituiertes Heterocycloalkyl ist;
R₆ Wasserstoff, Alkyl, substituiertes Alkyl, Aryl, substituiertes Aryl, ein Heterocyclus, substituierter Heterocyclus, -NR₇R₈, -OR₇ oder Halogen ist;
R₁₃ und R₁₉ bei jedem Vorkommen unabhängig voneinander ausgewählt sind aus Alkyl, Halogen, Trifluormethoxy, Trifluormethyl, Hydroxy, Alkoxy, Alkanoyl, Alkanoyloxy, Thiol, Alkylthio, Ureido, Nitro, Cyano, Carboxy, Carboxyalkyl, Carbamyl, Alkoxycarbonyl, Alkylthiono, Arylthiono, Arylsulfonylamin, C₁₋₄-Alkylsulfonylamin, Sulfonsäure, Alkylsulfonyl, Sulfonamido und Aryloxy, wobei jeder Rest R₁₃ und R₁₉ weiter mit Hydroxy, Alkyl, Alkoxy, Aryl oder Aralkyl substituiert sein kann;
R₇, R₈, R₂₁, R₂₄ und R₂₅ unabhängig voneinander ausgewählt sind aus Wasserstoff, Alkyl, substituiertem Alkyl, Aryl, substituiertem Aryl, einem Heterocyclus und einem substituierten Heterocyclus;
R₂₂ Alkyl, substituiertes Alkyl, Aryl, substituiertes Aryl, ein Heterocyclus oder ein substituierter Heterocyclus ist; und
n 0, 1 oder 2 ist.

21. Verbindung nach Anspruch 20 der Formel: 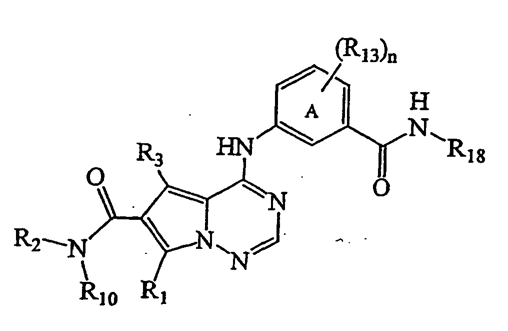 oder ein pharmazeutisch verträgliches Salz, Prodrug oder Solvat davon, wobei:
R₁₈ Alkoxy, Aryl oder Aryl, substituiert mit R₁₉, ist;
R₁ und R₁₀ Wasserstoff oder -CH₃ sind; und
R₁₃ Niederalkyl, Halogen, Trifluormethoxy, Trifluormethyl, Hydroxy, C₁₋₄-Alkoxy, Nitro oder Cyano ist.

22. Verbindung nach Anspruch 20 der Formel: 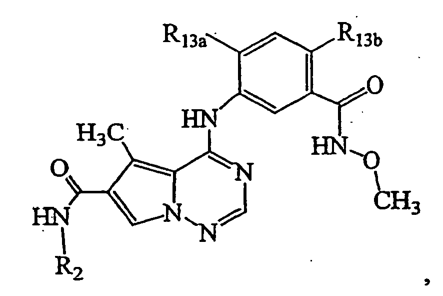 oder ein pharmazeutisch verträgliches Salz, Hydrat oder Prodrug davon, wobei R₂ ein geradkettiges oder verzweigtes C₂₋₆-Alkyl oder gegebenenfalls substituiertes Benzyl ist und R₁₃ₐ und R_{13b} ausgewählt sind aus Wasserstoff, C₁₋₄-Alkyl, Hydroxy, Halogen, Cyano und Trifluormethyl.

23. Verbindung nach Anspruch 20, bei welcher es sich um N-Ethyl-4-[[5-[(methoxyamino)carbonyl]-2-methylphenyl]amino]-5-methylpyrrolo[2,1-f][1,2,4]triazin-6-carboxamid handelt; oder ein Methansulfonsäuresalz umfassendes pharmazeutisch verträgliches Salz davon.

24. Verbindung nach Anspruch 20, bei welcher es sich um 4-[[5-[(Methoxyamino)carbonyl]-2-methylphenyl]amino]-5-methyl-N-(1-methylethyl)-pyrrolo[2,1-f][1,2,4]triazin-6-carboxamid handelt; oder ein pharmazeutisch verträgliches Salz davon.

25. Verbindung nach Anspruch 20, bei welcher es sich um 4-[[5-[(Methoxyamino)carbonyl]-2-methylphenyl]amino]-5-methyl-N-propylpyrrolo[2,1-f][1,2,4]triazin-6-carboxamid handelt; oder ein pharmazeutisch verträgliches Salz davon.

26. Verbindung nach Anspruch 20, bei welcher es sich um 4-[[5-[(Methoxyamino)carbonyl]-2-methylphenyl]amino]-5-methyl-N-[1-phenylethyl]-pyrrolo(2,1-f][1,2,4]triazin-6-carboxamid handelt; oder ein pharmazeutisch verträgliches Salz davon.

27. Verbindung nach Anspruch 20, bei welcher es sich um 4-[[5-[(Methoxyamino)carbonyl]-2-methylphenyl]amino]-5-methyl-N-[1-methylpropyl]-pyrrolo[2,1-f] [1,2,4]triazin-6-carboxamid handelt; oder ein pharmazeutisch verträgliches Salz davon.

28. Arzneimittel umfassend mindestens eine Verbindung nach einem der Ansprüche 7 bis 27 und einen pharmazeutisch verträglichen Träger oder ein pharmazeutisch verträgliches Verdünnungsmittel.

29. Verwendung eines Arzneimittels nach Anspruch 28 zur Herstellung eines Medikaments zur Behandlung einer entzündlichen Störung.

30. Verwendung nach Anspruch 29, wobei die entzündliche Störung ausgewählt ist aus Asthma, Schocklunge, chronisch-obstruktiver Lungenerkrankung, chronischer Lungenentzündung, Diabetes, entzündlicher Darmerkrankung, Osteoporose, Psoriasis, Transplantat-Wirt-Abstoßung, Atherosklerose und Arthritis einschließlich rheumatoider Arthritis, psoriatischer Arthritis, traumatischer Arthritis, Rötelarthritis, Gichtarthritis und Osteoarthritis.

## Revendications

1. Utilisation d'au moins un composé répondant à la formule (I) : 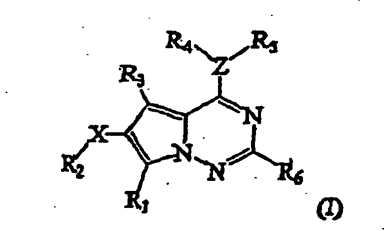 ou d'un sel, promédicament ou solvate pharmaceutiquement acceptable de celui-ci, dans laquelle:
R₃ est un atome d'hydrogène, un groupe méthyle, perfluorométhyle, méthoxy, un atome d'halogène, un groupe cyano ou NH₂;
X est choisi parmi -O-, -OC (=O) -S-, -S(=O)-, -SO₂-, -C (=O) -, -CO₂-, -NR₁₀-, -NR₁₀C (=O) -, -NR₁₀C (=O) NR₁₁-, -NR₁₀CO₂-, -NR₁₀SO₂-, -NR₁₀SO₂NR₁₁-, -SO₂NR₁₀-, -C (=O) NR₁₀-, un atome d'halogène, un groupe nitro et cyano, ou X est absent;
Z est choisi' parmi O, S, N et CR₂₀, dans lequel lorsque Z est CR₂₀, ledit atome de carbone peut former un groupe aryle ou hétéroaryle bicyclique facultativement substitué avec R₄ et R₅;
R₁ est un atome d'hydrogène, -CH₃, -OH, -OCH₃, -SH, -SCH₃, -OC (=O) R₂₁, -S(=O)R₂₂, -SO₂R₂₂, -SO₂NR₂₄R₂₅, -CO₂R₂₁, C (=O) NR₂₄R₂₅, -NH₂, -NR₂₄R₂₅, NR₂₁SO₂NR₂₄R₂₅, -NR₂₁SO₂R₂₂, -NR₂₄C (=O) R₂₅, -NR₂₄CO₂R₂₅, -NR₂₁C (=O) NR₂₄R₂₅, un atome d' halogène, un groupe nitro ou cyano;
R₂ est choisi parmi:
a) un atome d'hydrogène, à condition que R₂ ne soit pas un atome d'hydrogène lorsque X est -S (=O) -, -SO₂-, -NR₁₀CO₂-, ou -NR₁₀SO₂-;
b) un groupe alkyle, alcényle et alcynyle facultativement substitué par jusqu'à quatre groupes R₂₆ ou pentafluoroalkyle;
c) un groupe aryle et hétéroaryle facultativement substitué par jusqu'à trois groupes R₂₇; et
d) un groupe hétérocyclo et cycloalkyle facultativement substitué par un groupe céto (=O), jusqu'à trois groupes R₂₇, et/ou ayant un pont carbone-carbone de 3 à 4 atomes de carbone; ou
e) R₂ est absent si X est un atome d'halogène, un groupe nitro ou cyano;
(i) R₄ est un groupe aryle substitué, aryle substitué par un groupe NHSO₂alkyle, hétéroaryle substitué, ou un cycle carbocyclique ou hétérocyclique saturé ou insaturé bicyclique facultativement substitué à 7 à 11 chaînons, et
R₅ est un atome d'hydrogène, un groupe alkyle ou alkyle substitué, excepté lorsque Z est O ou S, R₅ est absent, ou en variante,
(ii) R₄ et R₅ pris conjointement avec Z forment un groupe aryle ou hétéroaryle bicyclique facultativement substitué à 7 à 11 chaînons;
R₆ est un atome d'hydrogène, un groupe alkyle, alkyle substitué, aryle, aryle substitué, hétérocyclo, hétérocyclo substitué, -NR₇R₈, -OR₇ ou un atome d'halogène;
R₁₀ et R₁₁ sont indépendamment choisis parmi un atome d'hydrogène, un groupe alkyle, alkyle substitué, aryle, aryle substitué, cycloalkyle, cycloalkyle substitué, hétérocyclo et hétérocyclo substitué;
R₇, R₈, R₂₁, R₂₄ et R₂₅ sont indépendamment choisis parmi un atome d'hydrogène, un groupe alkyle, alkyle substitué, aryle, aryle substitué, hétérocyclo et hétérocyclo substitué;
R₂₀ est un atome d'hydrogène, un groupe alkyle inférieur ou alkyle substitué, ou R₂₀ peut être absent si l'atome de carbone auquel il est attaché conjointement avec R₄ et R₅ fait partie d'un groupe aryle ou hétéroaryle bicyclique insaturé;
R₂₂ est un groupe alkyle, alkyle substitué, aryle, aryle substitué, hétérocyclo ou hétérocyclo substitué;
R₂₆ est choisi parmi un atome d'halogène, un groupe trifluorométhyle, halogénoalcoxy, céto (=O), nitro, cyano, -SR₂₈, -OR₂₈, -NR₂₈R₂₉, -NR₂₈SO₂-, -NR₂₈SO₂R₂₉, -SO₂R₂₈, -SO₂NR₂₈R₂₉, -CO₂R₂₈, -C(=O)R₂₈, -C (=O) NR₂₈R₂₉, -OC (=O) R₂₈, -OC (=O) NR₂₈R₂₉, -NR₂₈C (=O) R₂₉, -NR₂₈CO₂R₂₉, =N-OH, =N-O-alkyle; aryle facultativement substitué par un à trois groupe(s) R₂₇; cycloalkyle facultativement substitué par un groupe céto(=O), un à trois groupe(s) R₂₇, ou ayant un pont carbone-carbone de 3 à 4 atomes de carbone; et hétérocyclo facultativement substitué par un groupe céto (=O), un à trois groupe (s) R₂₇, ou ayant un pont carbone-carbone de 3 à 4 atomes de carbone; dans lequel R₂₈ et R₂₉ sont chacun indépendamment choisis parmi un atome d'hydrogène, un groupe alkyle, alcényle, aryle, aralkyle, cycloalkyle en C_{3 à 7}, et hétérocycle en C_{3 à 7}, ou peuvent être pris ensemble pour former un hétérocycle en C_{3 à 7;} et dans lequel chaque R₂₈ et R₂₉ est à son tour facultativement substitué par jusqu'à deux parmi les groupes alkyle, alcényle, un atome d'halogène, les groupes halogénoalkyle, halogénoalcoxy, cyano, nitro, amino, hydroxy, alcoxy, alkylthio, phényle, benzyle, phényloxy et benzyloxy; et
R₂₇ est choisi parmi un groupe alkyle, R₃₂, et alkyle en C_{1 à 4} substitué par un à trois groupe (s) R₃₂, dans lequel chaque groupe R₃₂ est indépendamment choisi parmi un atome d'halogène, un groupe halogénoalkyle, halogénoalcoxy, nitro, cyano, -SR₃₀, -OR₃₀, -NR₃₀R₃₁, -NR₃₀SO₂, -NR₃₀SO₂R_{31,} -SO₂R₃₀, -SO₂NR₃₀R₃₁, -CO₂R₃₀, -C (=O) R₃₀, -C (=O) NR₃₀R₃₁, -OC (=O) R₃₀, -OC (=O) NR₃₀R₃₁, -NR₃₀C (=O) R₃₁, -NR₃₀CO₂R₃₁, et un cycle carbocyclique ou hétérocyclique à 3 à 7 chaînons facultativement substitué par un groupe alkyle, un atome d'halogène, un groupe hydroxy, alcoxy, halogénoalkyle, halogénoalcoxy, nitro, amino ou cyano, dans lequel R₃₀ et R₃₁ sont chacun indépendamment choisis parmi un atome d'hydrogène, un groupe alkyle, alcényle, aryle, aralkyle, cycloalkyle en C_{3 à 7}, et hétérocycle, ou peuvent être pris ensemble pour former un hétérocycle en C_{3 à 7}, pour la fabrication d'un médicament destiné à traiter une ou plusieurs conditions associées à l'activité de la kinase p38.

2. Utilisation selon la revendication 1, dans laquelle:
R₃ est un groupe méthyle, -CF₃ ou -OCH₃;
X est choisi parmi -C (=O) -, -CO₂-, -NR₁₀-, -NR₁₀C(=O)-, -NR₁₀CO₂-, -NR₁₀SO₂-, -SO₂NR₁₀- et -C (=O) NR₁₀-, ou X est absent;
Z est N;
R₂ est un atome d'hydrogène, un groupe alkyle en C _{2 à 6}, alkyle en C_{1 à 4} substitué par jusqu'à quatre groupes R₂₆, pentafluoroalkyle, ou aryle ou hétéroaryle facultativement substitué par jusqu'à deux groupes R₂₇;
R₄ est un groupe phényle substitué par un groupe R₁₂ et zéro à trois groupe(s) R₁₃;
R₅ et R₁₀ sont indépendamment choisis parmi un atome d'hydrogène et un groupe alkyle inférieur;
R₁₂ est un groupe carbamyle, sulfonamido, arylsulfonylamine ou uréido, dont chacun est facultativement substitué par jusqu'à deux parmi les groupes hydroxy, alkyle, alkyle substitué, alcoxy, aryle, aryle substitué et aralkyle, ou alkylsulfonylamine;
R₁₃, à chaque occurrence, est indépendamment choisi parmi un groupe alkyle, alkyle substitué, halogéno, trifluorométhoxy, trifluorométhyle, -OR₁₄, -C(=O)alkyle, -OC (=O) alkyle, -NR₁₅R₁₆, -SR₁₅, -NO₂, -CN, -CO₂R₁₅, -CONH₂, -SO₃H, -S (=O) alkyle, -S (=O) aryle, -NHSO₂-aryl-R₁₇, -NHSO₂₋alkyle, -SO₂NHR₁₇, -CONHR₁₇ et -NHC (=O) NHR₁₇;
R₁₄ est un atome d'hydrogène, un groupe alkyle ou aryle;
R₁₅ est un atome d'hydrogène ou un groupe alkyle;
R₁₆ est un atome d'hydrogène, un groupe alkyle, aralkyle ou alcanoyle; et
R₁₇ est un atome d'hydrogène, un groupe hydroxy, alkyle, alkyle substitué, alcoxy, aryle, aryle substitué ou aralkyle.

3. Utilisation d'au moins composé répondant à la formule (I) : 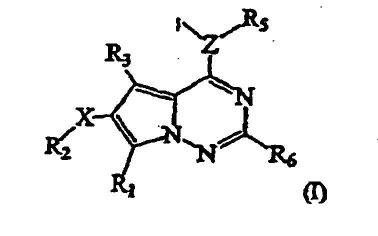 ou d'un sel, promédicament ou solvate pharmaceutiquement acceptable de celui-ci, dans laquelle:
R₃ est un atome d'hydrogène, un groupe méthyle, perfluorométhyle, méthoxy, un atome d'halogène, un groupe cyano ou NH₂;
X est choisi parmi -O-, -OC(=O)-, -S-, -S(=O), -SO₂-, -C (=O) -, -CO₂-, -NR₁₀-, -NR₁₀C (=O) -, -NR₁₀C (=O) NR₁₁-, -NR₁₀CO₂-, -NR₁₀SO₂-, -NR₁₀SO₂NR₁₁-, -SO₂NR₁₀-, -C (=O) NR₁₀-, un atome d'halogène, un groupe nitro et cyano, ou X est absent;
Z est O, S, N ou CR_{20;}
R₁ est un atome d'hydrogène, -CH₃-, -OH, -OCH₃, -SH, - SCH₃ , -OC (=O) R₂₁, -S (=O) R₂₂, -SO₂R₂₂, -SO₂NR₂₄R₂₅, -CO₂R₂₁, -C (=O) NR₂₄R₂₅, -NH₂, -NR₂₁SO₂NR₂₉R₂₅, -NR₂₁SO₂R₂₂, -NR₂₄C (=O) R₂₅, -NR₂₄CO₂R₂₅,
-NR₂₁C(=O)NR₂₄R₂₅, un atome d'halogène, un groupe nitro ou cyano;
R₂ est un atome d'hydrogène, un groupe alkyle, alkyle substitué, alcényle, alcényle substitué, alcynyle, alcynyle substitué, aryle, aryle substitué, hétérocyclo, hétérocyclo substitué, aralkyle, aralkyle substitué ou hétérocycloalkyle, ou hétérocycloalkyle substitué, ou lorsque X est un groupe halogéno, nitro ou cyano, R₂ est absent, à condition que R₂ ne soit pas un atome d'hydrogène lorsque X est -S(=O)-, -SO₂-, -NR₁₀CO₂-, ou -NR₁₀SO₂-;
R₄ est un groupe aryle substitué, aryle substitué par un groupe NHSO₂alkyle, hétéroaryle substitué, ou un cycle carbocyclique ou hétérocyclique saturé ou insaturé bicyclique facultativement substitué à 7 à 11 chaînons;
R₅ est un atome d'hydrogène, un groupe alkyle ou alkyle substitué, excepté lorsque Z est O ou S, R₅ est absent;
R₆ est un atome d'hydrogène, un groupe alkyle, alkyle substitué, aryle, aryle substitué, hétérocyclo, hétérocyclo substitué, -NR₇R₈, -OR₇ ou un atome d'halogène;
R₇, R₈, R₁₀, R₁₁, R₂₁, R₂₄ et R₂₅ sont indépendamment choisis parmi un atome d'hydrogène, un groupe alkyle, alkyle substitué, aryle, aryle substitué, hétérocyclo et hétérocyclo substitué;
R₂₀ est un atome d'hydrogène, un groupe alkyle inférieur ou alkyle substitué; et
R₂₂ est un groupe alkyle, alkyle substitué, aryle, aryle substitué, hétérocyclo ou hétérocyclo substitué, pour la fabrication d'un médicament destiné à traiter une ou plusieurs conditions associées à l'activité de la kinase p38.

4. Utilisation selon la revendication 3, dans laquelle R₄ et R₅ pris conjointement avec Z forment: 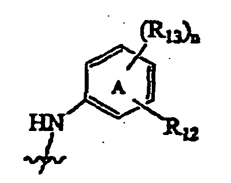
R₁₂ est attaché à tout atome de carbone disponible du cycle phényle A et est choisi parmi les groupes carbamyle, sulfonamido, arylsulfonylamine et uréido, dont chacun est facultativement substitué par jusqu'à l'un parmi les groupes hydroxy, alkyle, alkyle substitué, alcoxy, aryle, aryle substitué et aralkyle ou (alkyl en C_{1 à 4}) sulfonylamine;
R₁₃ est attaché à tout atome de carbone disponible du cycle phényle A et, à chaque occurrence est indépendamment choisi parmi un groupe alkyle, alkyle substitué, halogéno, trifluorométhoxy, trifluorométhyle, -OR₁₄, -C (=O) alkyle, -OC (=O) alkyle, -NR₁₅R₁₆, -SR₁₅, -NO₂, -CN, -CO₂R₁₅, -CONH₂, -SO₃H, -S (=O) alkyle, -S (=O) aryle, -NHSO₂-aryl-R₁₇, -NHSO₂ (alkyle en C_{1 à 4}), -SO₂NHR₁₇, -CONHR₁₇ et -NHC (=O) NHR₁₇;
R₁₄ est un atome d'hydrogène, un groupe alkyle ou aryle;
R₁₅ est un atome d'hydrogène ou un groupe alkyle;
R₁₆ est un atome d'hydrogène, un groupe alkyle, aralkyle ou alcanoyle; et
R₁₇ est un atome d'hydrogène, un groupe hydroxy, alkyle, alkyle substitué, alcoxy, aryle, aryle substitué ou aralkyle; et
n vaut 0, 1, 2 ou 3.

5. Utilisation selon la revendication 3, dans laquelle le au moins un composé répond à la formule (II): 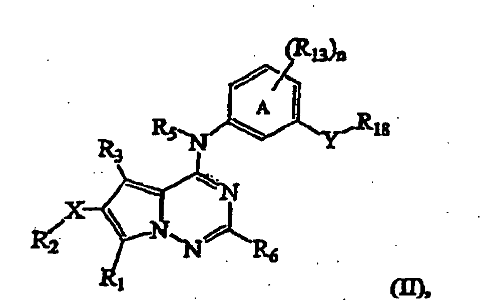 dans laquelle:
R₃ est un groupe méthyle ou CF₃;
X est -C (=O) NR₁₀-, -NR₁₀C (=O) -, -C (=O) - ou -CO₂-;
R₁ est un atome d'hydrogène, -CH₃-, -OH, -OCH₃, un atome d'halogène, un groupe nitro ou cyano;
Y est -C (=O) NH-, -NHC (=O) NH-, -NHSO₂-, ou -SO₂NH-;
R₁₀ est un atome d'hydrogène ou un groupe alkyle inférieur;
R₁₈ est choisi parmi un atome d'hydrogène, un groupe alkyle, alcoxy, aryle et aryle substitué par un à trois groupe (s) R₁₉, excepté que lorsque Y est -NHSO₂-, R₁₈ est un groupe alkyle en C₁ à ₄, aryle ou aryle substitué par un groupe R₁₉;
R₁₃ est attaché à tout atome de carbone disponible du cycle phényle A et, à chaque occurrence, est indépendamment choisi parmi les groupes alkyle, alkyle substitué, halogéno, trifluorométhoxy, trifluorométhyle, -OR₁₄, -C(=O)alkyle, -OC (=O) alkyle, -NR₁₅R₁₆, -SR₁₅, -NO₂, -CN, -CO₂R₁₅, -CONH₂, -SO₃H, -S (=O) alkyle, -S (=O) aryle, -NHSO₂-aryl-R₁₇, -NHSO₂ (alkyle en C_{1 à 4}), -SO₂NHR₁₇, -CONHR₁₇ et -NHC(=O)NHR₁₇;
R₁₄, R₁₅, R₁₆ et R₁₇ sont un atome d'hydrogène ou un groupe alkyle;
R₁₉, à chaque occurrence, est choisi parmi un groupe alkyle, halogéno, trifluorométhoxy, trifluorométhyle, hydroxy, alcoxy, alcanoyle, alcanoyloxy, thiol, alkylthio, uréido, nitro, cyano, carboxy, carboxyalkyle, carbamyle, alcoxycarbonyle, alkylthiono, arylthiono, arylsulfonylamine, acide sulfonique, alkysulfonyle, sulfonamido et aryloxy, dans lequel chaque groupe R₁₉ peut être en outre substitué par un groupe hydroxy, alkyle, alcoxy, aryle ou aralkyle et
n vaut 0, 1 ou 2.

6. Utilisation selon la revendication 3, dans laquelle le au moins un composé répond à la formule (Ia), (Ib) ou (Ic): 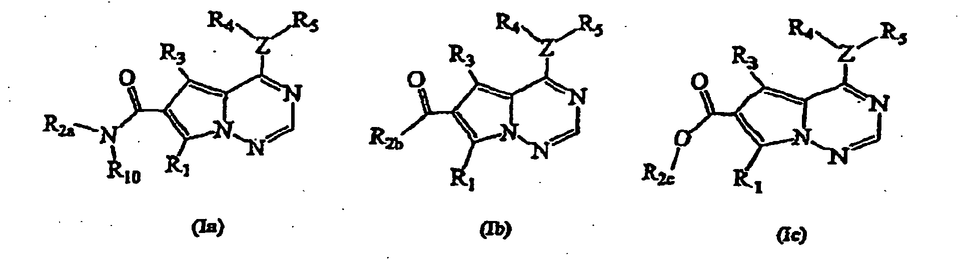 dans lesquelles:
R₃ est un groupe méthyle ou CF₃;
R₂ₐ et R_{2c} sont indépendamment choisis parmi un atome hydrogène, un groupe alkyle en C_{2 à 6}, alkyle substitué en C_{1 à 4}, aryle, aryle substitué, benzyle et benzyle substitué;
R_{2b} est un groupe hétérocyclo ou hétérocyclo substitué; et
R₁₀ est un atome d'hydrogène ou un groupe alkyle inférieur.

7. Composé répondant à la formule (II): 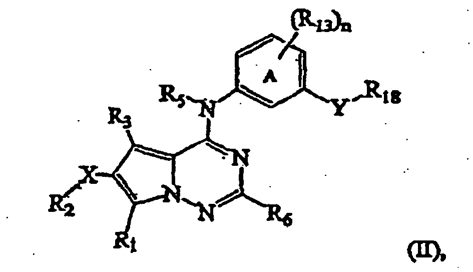 ou un sel, promédicament ou solvate pharmaceutiquement acceptable de celui-ci, dans laquelle:
R₃ est un groupe méthyle, -CF₃ ou -CH₃; R₅ est un atome d'hydrogène ou un groupe alkyle;
Y est -C (=O) NR₂₃-, -NR₂₃C(=O)NR₂₃-, -SO₂NR₂₃ ou -NR₂₃SO₂-;
R₁₈ et R₂₃ sont choisis parmi un atome d'hydrogène, un groupe alkyle, alcoxy, aryle et aryle substitué par un à trois groupe (s) R₁₉, excepté que lorsque Y est -NR₂₃SO₂-, R₁₈ est un groupe alkyle en C_{1 à 4} ou aryle facultativement substitué par un à trois groupe(s) R₁₉;
X est choisi parmi -O-, -OC(=O)-, -S-, -S(=O)-, -SO₂-, -C (=O) -, -CO₂-, -NR₁₀-, -NR₁₀C (=O) -, -NR₁₀C(=O)NR₁₁-, -NR₁₀CO₂-, -NR₁₀SO₂-, -NR₁₀SO₂NR₁₁-, -SO₂NR₁₀-, et -C (=O) NR₁₀-, ou X est absent;
R₁ est un atome d'hydrogène, -CH₃-, -OH, -OCH₃, -SH, -SCH₃, -OC (=O) R₂₁, -S (=O) R₂₂, -SO₂R₂₂, -SO₂NR₂₄R₂₅, -CO₂R₂₁, -C (=O) NR₂₄R₂₅, -NH₂, NR₂₄R₂₅, -NR₂₁SO₂NR₂₄R₂₅, -NR₂₁SO₂R₂₂, -NR₂₄(C=O)R₂₅, -NR₂₄CO₂R₂₅, -NR₂₁C(=O)NR₂₄R₂₅, un atome d'halogène, un groupe nitro ou cyano;
R₂ est choisi parmi
a) un atome d'hydrogène, à condition que R₂ ne soit pas un atome d'hydrogène lorsque X est -S (=O) -, -SO₂-, -NR₁₀CO₂-, ou NR₁₀SO₂-;
b) un grope alkyle, alcényle et alcynyle facultativement substitué par jusqu'à quatre groupes R₂₆, ou pentafluokroalkyle;
c) un groupe aryle et hétéroaryle facultativement substitué par jusqu'à trois groupes R₂₇; et
d) un groupe hétérocyclo et cycloalkyle facultativement substitué par un groupe céto (=O), jusqu'à trois groupes R₂₇, et/ou ayant un pont carbone-carbone de 3 à 4 atomes de carbone;
R₆ est un atome d'hydrogène, un groupe alkyle, alkyle substitué, aryle, aryle substitué, hétérocyclo, hétérocyclo substitué, -NR₇R₈, -OR₇, ou un atome d'halogène;
R₁₀ est un atome d'hydrogène ou un groupe alkyle;
R₁₃ et R₁₉ sont, à chaque occurrence, indépendamment choisis parmi un groupe alkyle, halogéno, trifluorométhoxy, trifluorométhyle, hydroxy, alcoxy, alcanoyle, alcanoyloxy, thiol, alkylthio, uréido, nitro, cyano, carboxy, carboxyalkyle, carbamyle, alcoxycarbonyle, alkylthiono, arylthiono, arylsulfonylamine, (alkyl en C_{1 à 4})sulfonylamine, acide sulfonique, alkylsulfonyle, sulfonamido et aryloxy, dans lequel chaque groupe R₁₃ et/ou R₁₉ peut être en outre substitué par un groupe hydroxy, alkyle, alkyle substitué, alcoxy, aryle ou aralkyle;
R₇, R₈, R₂₁, R₂₄ et R₂₅ sont indépendamment choisis parmi un atome d'hydrogène, un groupe alkyle, alkyle substitué, aryle, aryle substitué, hétérocyclo et hétérocyclo substitué;
R₂₂ est un groupe alkyle, alkyle substitué, aryle, aryle substitué, hétérocyclo ou hétérocyclo substitué;
R₂₆ est choisi parmi un atome d'halogène, un groupe trifluorométhyle, halogénoalcoxy, céto (=O), nitro, cyano, -SR₂₈, -OR₂₈, -NR₂₈R₂₉, -NR₂₈SO₂-, -NR₂₈SO₂R₂₉, -SO₂R₂₈, -SO₂NR₂₈R₂₉, -CO₂R₂₈, -C (=O) R₂₈, -C (=O)NR₂₈R₂₉, -OC (=O) R₂₈, -OC (=O)NR₂₈R₂₉, -NR₂₈C(=O)R₂₉, -NR₂₈CO₂R₂₉, =N-OH, =N-O-alkyle; aryle facultativement substitué par un à trois groupe(s) R₂₇; cycloalkyle facultativement substitué par un groupe céto(=O), un à trois R₂₇, ou ayant un pont carbone-carbone de 3 à 4 atomes de carbone;et hétérocyclo facultativement substitué par un groupe céto (=O), un à trois groupe(s) R₂₇, ou ayant un pont carbone-carbone de 3 à 4 atomes de carbone; dans lequel les groupes R₂₈ et R₂₉ sont chacun indépendamment choisis parmi un atome d'hydrogène, un groupe alkyle, alcényle, aryle, aralkyle, cycloalkyle en C_{3 à 7}, et hétérocycle en C_{3 à 7}, ou peuvent être pris ensemble pour former un hétérocycle en C_{3 à 7}; et dans lequel chaque groupe R₂₈ et R₂₉ est à son tour facultativement substitué par jusqu'à deux parmi les groupes alkyle, alcényle, un atome d'halogène, les groupes halogénoalkyle, halogénoalcoxy, cyano, nitro, amino, hydroxy, alcoxy, alkylthio, phényle, benzyle, phényloxy et benzyloxy;
R₂₇ est choisi parmi un groupe alkyle, R₃₂, et alkyle en C₁₋₄ substitué par un à trois groupe (s) R₃₂, dans lequel chaque groupe R₃₂ est indépendamment choisi parmi un atome d'halogène, un groupe halogénoalkyle, un halogénoalcoxy, nitro, cyano, -SR₃₀, -OR₃₀, -NR₃₀R₃₁, -NR₃₀SO₂, -NR₃₀SO₂R₃₁, -SO₂R₃₀, -SO₂NR₃₀R₃₁, -CO₂R₃₀, -C (=O) R₃₀, -C (=O) NR₃₀R₃₁, -OC (=O) R₃₀, -OC (=O) NR₃₀R₃₁, -NR₃₀C (=O) R₃₁, -NR₃₀CO₂R₃₁, et un cycle carbocyclique ou hétérocyclique à 3 à 7 chaînons facultativement substitué par un groupe alkyle, un atome d'halogène, un groupe hydroxy, alcoxy, halogénoalkyle, halogénoalcoxy, nitro, amino ou cyano, dans lequel R₃₀ et R₃₁ sont chacun indépendamment choisis parmi un atome dhydrogène, un groupe alkyle, alcényle, aryle, aralkyle, cycloalkyle en C_{3 à 7}, et hétérocycle ou peuvent être pris ensemble pour former un hétérocycle en C_{3 à 7}; et
n vaut 0, 1 ou 2; à l'exclusion de l'ester de méthyle d'acide 4-[[3-(aminosulfonyl)-4-méthylphényl]-amino]-5-méthylpyrrolo[2,1-f][1,2,4]triazine-6-carboxylique.

8. Composé selon la revendication 7, répondant à la formule (IIa) ou (IIb), 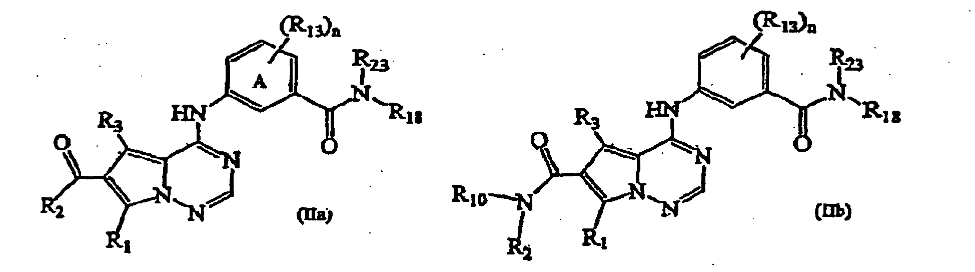
ou un sel, promédicament ou solvate pharmaceutiquement acceptable de celui-ci, dans laquelle:
R₁ et R₁₀ sont un atome d'hydrogène ou -CH₃;
R₁₃ est un groupe alkyle inférieur, un atome d' halogène, un groupe trifluorométhoxy, trifluorométhyle, hydroxy, alcoxy en C_{1 à 4}, nitro ou cyano;
R₁₈ est un groupe hydroxy, alcoxy en C_{1 à 4}, phényle ou phényle substitué par un ou deux groupe(s) R₁₉;
R₂₃ est un atome d'hydrogène ou un groupe alkyle inférieur; et
n vaut 0, 1 ou 2.

9. Composé selon la revendication 7, ou un sel, promédicament ou solvate pharmaceutiquement acceptable de celui-ci, dans lequel
X est -C (=O) -, -CO₂-, -NR₁₀C (=O) -, ou -C (=O) NR₁₀-;
Y est -C(=O)NH-, -NHC(=O)NH-, ou -NHSO₂-;
R₅ et R₁₀ sont un atome d'hydrogène ou -CH_{3;}
R₁₃ et R₁₉, à chaque occurrence, sont indépendamment choisis parmi un groupe alkyle inférieur, halogéno, trifluorométhoxy, trifluorométhyle, hydroxy, alcoxy en C_{1 à 4}, nitro et cyano;
R₇, R₈, R₂₁, R₂₄, et R₂₅ sont indépendamment choisis parmi un atome d'hydrogène et un groupe alkyle inférieur;
R₂₂ est un groupe alkyle inférieur; et
n vaut 0 ou 1.

10. Composé selon la revendication 7 ou sel, promédicament ou solvate pharmaceutiquement acceptable de celui-ci, dans lequel X-R₂ est: 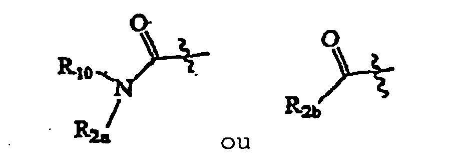 R₂ₐ est choisi parmi:
a) un atome d'hydrogène;
b) un groupe alkyle en C₂ à ₆ linéaire ou ramifié;
c) un groupe cycloalkyle facultativement substitué par un groupe céto et/ou jusqu'à deux groupes R₂₇;
d) un groupe phényle facultativement substitué par jusqu'à deux groupes R₂₇; et
e) un hétérocycle facultativement substitué par un groupe céto et/ou jusqu'à deux groupes R₂₇;
f) un groupe pentafluoroalkyle ou alkyle en C_{1 à 4} substitué par jusqu'à trois parmi un atome d'halogène, un groupe trifluorométhyle, cyano, OR₂₈, NR₂₈R₂₉, CO₂R₂₈, aryle, hétérocycle et/ou cycloalkyle, dans lequel le groupe aryle, hétérocycle et/ou cycloalkyle est à son tour facultativement substitué par jusqu'à deux parmi un atome d'halogène, un groupe hydroxy, alcoxy, halogénoalkyle, halogénoalcoxy, nitro, cyano et alkyle; et
R_{2b} est un hétérocycle monocyclique ou bicyclique facultativement substitué par jusqu'à deux groupes R₂₇;
R₂₇, à chaque occurrence, est indépendamment choisi parmi un atome d'hydrogène, un groupe alkyle, trifluorométhyle, trifluorométhoxy, un atome d'halogène, un groupe cyano, nitro, amino, hydroxy, alcoxy, phényle, benzyle, phényloxy et benzyloxy; et
R₂₈ et R₂₉, à chaque occurrence, sont indépendamment choisis parmi un atome d'hydrogène, un groupe alkyle, alcényle, phényle et benzyle.

11. Composé selon la revendication 7 ou un sel, promédicament ou solvate pharmaceutiquement acceptable de celui-ci, dans lequel:
R₂ₐ est choisi parmi:
a) un groupe alkyle en C_{2 à 6} linéaire ou ramifié;
b) un groupe phényle facultativement substitué par jusqu'à deux parmi un atome d'halogène, un groupe alcoxy en C_{1 à 4} et trifluorométhyle;
c) un groupe cycloalkyle en C_{3 à 6} facultativement substitué par jusqu'à deux groupes alkyle en C_{1 à 4} et/ou hydroxy;
d) un groupe alkyle en C_{1 à 4} linéaire ou ramifié substitué par jusqu'à trois parmi
i) un atome d'halogène,
ii) un groupe fluorométhyle,
iii) un groupe cyano,
iv) un groupe alcoxy en C_{1 à 4},
v) un groupe phényloxy,
vi) un groupe benzyloxy,
vii) NH₂, NH (alkyle en C_{1 à 4}) et/ou N(alkyle en C_{1 à 4})₂,
viii) un groupe phényle à son tour facultativement substitué par jusqu'à deux parmi un atome d'halogène et/ou un groupe méthoxy,
ix) un hétérocycle choisi parmi les groupes pyridinyle, indolyle, thiophényle, furanyle, thiazolyle, thiényle, morpholinyle, tétrahydrofuranyle, triazinyle, pipérazinyle, indényle, et pipéradinyle, ledit hétérocycle étant facultativement substitué par un à deux groupes alkyle en C_{1 à 4},
x) un groupe cycloalkyle en C_{3 à 6}; et
R_{2b} est un hétérocycle monocyclique à cinq à sept chaînons choisi parmi les groupes diazépinyle, morpholinyle, pipéridinyle et pyrrolidinyle, ledit hétérocycle étant facultativement substitué par un groupe alkyle en C_{1 à 4}, phényle et/ou benzyle.

12. Composé selon la revendication 7 ou sel, promédicament ou solvate pharmaceutiquement acceptable de celui-ci, dans lequel R₁ et R₁₀ sont indépendamment un atome d'hydrogène ou CH₃.

13. Composé selon la revendication 7 ou sel, promédicament ou solvate pharmaceutiquement acceptable de celui-ci, dans lequel R₆ est un atome d'hydrogène.

14. Composé selon la revendication 7 ou sel, promédicament ou solvate pharmaceutiquement acceptable de celui-ci, dans lequel Y est -NHC(=O)NH- ou -NHSO₂-; R₁₈ est un groupe aryle ou aryle substitué par un alkyle, OCH₃, CF₃, cyano ou un atome d'halogène.

15. Un composé selon la revendication 7 ou un sel, promédicament ou solvate pharmaceutiquement acceptable de celui-ci, dans lequel Y est -C(=O)NR₂₃-, R₂₃ est un atome d'hydrogène ou un groupe alkyle inférieur, et R₁₈ est un groupe alcoxy en C_{1 à 4} ou aryle facultativement substitué par un groupe alkyle, OCH₃, CF₃, cyano ou un atome d'halogène.

16. Composé selon la revendication 7, répondant à la formule: 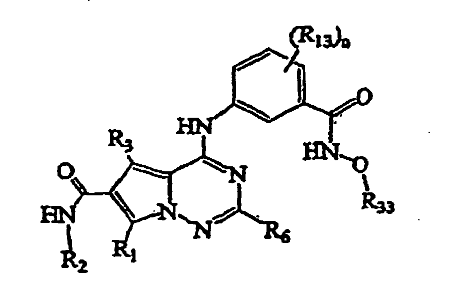 dans laquelle R₃₃ est un groupe alkyle inférieur.

17. Composé selon la revendication 16 ou sel, promédicament ou solvate pharmaceutiquement acceptable de celui-ci, dans lequel R₃ et R₃₃ sont un groupe méthyle, R₁ et R₆ sont un atome d'hydrogène, et R₂ est un groupe alkyle en C_{2 à 6} linéaire ou ramifié ou un groupe benzyle facultativement substitué.

18. Composé selon la revendication 7, qui est choisi parmi (i) le N-(2,2-diméthylpropyl)-4-[[5-[(méthoxyamino)carbonyl]-2-méthylphényl]amino]-N,5-diméthylpyrrolo[2,1-f][1,2,4]triazine-6-carboxamide; le 3-[[6-[(hexahydro-4-méthyl-1H-1,4-diazépin-1-yl) carbonyl]-5-méthylpyrrolo[2,1-f][1,2,4]triazin-4-yl] amino]-N-méthoxy-4-méthylbenzamide;
le 4-[[5-[(méthoxyamino)carbonyl]-2-méthylphényl]amino]-5-méthyl-N-(1-méthyléthyl)pyrrolo[2,1-f][1,2,4]triazine-6-carboxamide;
le 4-[[5-[(méthoxyamino)carbonyl]-2-méthylphényl]amino]-5-méthyl-N-(2-méthylpropyl)pyrrolo[2,1-f][1,2,4]triazine-6-carboxamide;
le N-(2,2-diméthylpropyl)-4-[[5-((méthoxyamino)carbonyl]-2-méthylphényl]amino]-5-méthylpyrrolo[2,1-f][1,2,4]triazine-6-carboxamide;
le 4-[[5-[(méthoxyamino)carbonyl]-2-méthylphényl]amino]-5-méthyl-N-propylpyrrolo[2,1-f][1,2,4]triazine-6-carboxamide;
le N-(1,1-diméthyléthyl)-4-[[5-[(méthoxyamino)carbonyl]-2-méthyl phényl]amino]-5-méthylpyrrolo[2,1-f][1,2,4]triazine-6-carboxamide;
le 4-[[5-[(méthoxyamino)carbonyl]-2-méthylphényl]amino]-N-(2-méthoxyéthyl)-5-méthylpyrrolo[2,1-f][1,2,4]triazine-6-carboxamide;
le N-méthoxy-4-méthyl-3-[[5-méthyl-6-(4-morpholinylcarbonyl) pyrrolo[2,1-f][1,2,4]triazin-4-yl]amino]benzamide;
le N-cyclohexyl-4-[[5-[(méthoxyamino)carbonyl]-2-méthylphényl] amino]-5-méthylpyrrolo[2,1-f][1,2,4]triazine-6-carboxamide;
le 4-[[5-[(méthoxyamino)carbonyl]-2-méthylphényl]amino]-5-méthyl-N-[(1R)-1-phényléthyl]pyrrolo[2,1-f][1,2,4]triazine-6-carboxamide;
le 4-[[5-[(méthoxyamino)carbonyl]-2-méthylphényl]amino]-5-méthyl-N-[(1S)-1-phényléthyl]pyrrolo[2,1-f][1,2,4]triazine-6-carboxamide;
le N-[(4-fluorophényl)méthyl]-4-[[5-[(méthoxyamino)carbonyl]-2-méthylphényl]amino]-5-méthylpyrrolo[2,1-f][1,2,4]triazine-6-carboxamide;
le 4-[[5-[(méthoxyamino)carbonyl)-2-méthylphényl)amino]-N-[(2-méthoxyphényl)méthyl]-5-méthylpyrrolo[2,1-f][1,2,4]triazine-6-carboxamide;
le 4-[[5-[(méthoxyamino)carbonyl]-2-méthylphényl]amino]-5-méthyl-N-(4-pyridinylméthyl)pyrrolo[2,1-f][1,2,4]triazine-6-carboxamide;
le 4-[[5-[(méthoxyamino)carbonyl]-2-méthylphényl)amino]-5-méthyl-N-[2-(4-pyridinyl)éthyl]pyrrolo[2,1-f][1,2,4]triazine-6-carboxamide;
le 4-[[5-[(méthoxyamino)carbonyl]-2-méthylphényl]amino]-5-méthyl-N-[2-(1-pipéridinyl)éthyl]pyrrolo[2,1-f][1,2,4]triazine-6-carboxamide;
le 4-[[5-[(méthoxyamino)carbonyl)-2-méthylphényl)amino]-5-méthyl-N-[2-(4-morpholinyl)éthyl]pyrrolo[2,1-f][1,2,4]triazine-6-carboxamide;
le N-[(1R,2S)-2,3-dihydro-1H-indèn-1-yl]-4-[[5-[(méthoxyamino) carbonyl]-2-méthylphényl]amino]-5-méthylpyrrolo[2,1-f][1,2,4] triazine-6-carboxamide;
le N-[(1S,2R)-2,3-dihydro-1H-indèn-1-yl]-4-[[5-[(méthoxyamino) carbonyl]-2-méthylphényl]amino]-5-méthylpyrrolo[2,1-f][1,2,4] triazine-6-carboxamide;
le N-méthoxy-4-méthyl-3-[[5-méthyl-6-[[4-(phénylméthyl)-1-pipéridinyl]carbonyl]pyrrolo[2,1-f][1,2,4]triazin-4=yl] amino] benzamide;
le N-cyclopropyl-4-[[5-[(méthoxyamino)carbonyl]-2-méthylphényl] amino]-5-méthylpyrrolo[2,1-f][1,2,4]triazine-6-carboxamide;
le N-cyclopentyl-4-[[5-((méthoxyamino)carbonyl]-2-méthylphényl]amino]-5-méthylpyrrolo[2,1-f][1,2,4]triazine-6-carboxamide;
le N-[2-(4-fluorophényl)éthyl]-4-[[5-[(méthoxyamino)carbonyl)-2-méthylphényl]amino]-5-méthylpyrrolo[2,1-f][1,2,4]triazine-6-carboxamide;
le N-(cyclohexylméthyl)-4-[[5-[(méthoxyamino)carbonyl]-2-méthylphényl]amino]-5-méthylpyrrolo[2,1-f][1,2,4]triazine-6-carboxamide;
le -4-[[5-[(méthoxyamino)carbonyl]-2-méthylphényl]amino]-5 méthyl-N-[(tétrahydro-2-furanyl)méthyl]pyrrolo[2,1-f][1,2,4]triazine-6-carboxamide;
le N-(2-1H-indol-3-yléthyl)-4-[[5-[(méthoxyamino)carbonyl]-2-méthylphényl]amino]-5-méthylpyrrolo[2,1-f][1,2,4]triazine-6-carboximide;
le N-butyl-4-[[5-[(méthoxyamino)carbonyl]-2-méthylphényl]amino] -5-méthylpyrrolo[2,1-f][1,2,4]triazine-6-carboxamide;
le N-(cyclopropylméthyl)-4-[[5-[(méthoxyamino)carbonyl]-2-méthylphényl]amino]-5-méthylpyrrolo[2,1-f][1,2,9]triazine-6-carboxamide;
le 4-[[5-[(méthoxyamino)carbonyl]-2-méthylphényl]amino]-5-méthyl-N-(2-méthylbutyl)pyrrolo[2,1-f][1,2,4]triazine-6-carboxamide;
le N-(2-furanylméthyl)-4-[[5-[(méthoxyamino)carbonyl]-2-méthylphényl]amino]-5-méthylpyrrolo[2,1-f][1,2,4]triazine-6-carboxamide;
le 4-[[5-[(méthoxyamino)carbonyl]-2-méthylphényl)amino]-5-méthyl-N-(2-thiénylméthyl)pyrrolo[2,1-f][1,2,4]triazine-6-carboxamide;
le -4-[[5-[(méthoxyamino)carbonyl]-2-méthylphényl]amino]-5 méthyl-N-(2-phénoxyéthyl)pyrrolo[2,1-f][1,2,4]triazine-6-carboxamide;
le 4-[[5-[(méthoxyamino)carbonyl]-2-méthylphényl]amino]-5-méthyl-N-(2-méthylcyclohexyl)pyrrolo[2,1-f][1,2,4]triazine-6-carboxamide;
le N-éthyl-4-[[5-(méthoxyamino)carbonyl]-2-méthylphényl]amino]-N,5-diméthylpyrrolo[2,1-f][1,2,4]triazine-6-carboxamide;
le 4-[[5-[(méthoxyamino)carbonyl]-2-méthylphényl]amino]-5-méthyl-N-(2,2,2-trifluoroéthyl)pyrrolo[2,1-f][1,2,4]triazine-6-carboxamide;
le N-(2-fluoroéthyl)-4-[[5-[(mét-hoxyamino)carbonyl]-2-méthylphényl]amino]-5-méthylpyrrolo[2,1-f][l,2,4]triazine-6-carboxamide;
le N-(2,3-dihydro-1H-indèn-2-yl)-4-[[5-[(méthoxyamino)carbonyl]-2-méthylphényl]amino]-5-méthylpyrrolo[2,1-f][1,2,4]triazine-6-carboxamide;
le N-éthyl-4-[[5-[(méthoxyamino)carbonyl]-2-méthylphényl]amino]-5-méthylpyrrolo[2,1-f][1,2,4]triazine-6-carboxamide;
le 4-[[5-[(méthoxyamino)carbonyl]-2-méthylphényl]amino]-5-méthyl-N-(2,2,3,3,3-pentafluoropropyl)pyrrolo[2,1-f][1,2,4]triazine-6-carboxamide;
le 4-[[5-[(méthoxyamino)carbonyl]-2-méthylphényl]amino]-5,7-diméthyl-N-(1-méthyléthyl)pyrrolo [2,1-f] [1,2,4]triazine-6-carboxamide;
le N-(4-fluorophényl)-4-[[5-[(méthoxyamino)carbonyl]-2-méthyl phényl]amino]-5-méthylpyrrolo[2,1-f][1,2,4]triazine-6-carboxamide;
le 4-[[5-[(méthoxyamino)carbonyl]-2-méthylphényl]amino]-N-(2-méthoxyphényl)-5-méthylpyrrolo[2,1-f][1,2,4]triazine-6-carboxamide;
le 4-[[5-[(méthoxyamino)carbonyl]-2-méthylphényl]amino]-N-[(3-méthoxyphényl)méthyl]-5-méthylpyrrolo[2,1-f][1,2,4]triazine-6-carboxamide;
le 4-[[5-[(méthoxyamino)carbonyl]-2-méthylphényl]amino]-5-méthyl-N-[3-(trifluorométhyl)phényl]pyrrolo[2,1-f][1,2,4] triazine-6-carboxamide;
le N-[(2,6-dichlorophényl)méthyl]-4-[[5-[(méthoxyamino)carbonyl]-2-méthylphényl]amino]-5-méthylpyrrolo[2,1-f][1,2,4]triazine-6-carboxamide;
le N-[(1S)-1-cyano-2-phényléthyl]-4-[[5-[(méthoxyamino)carbonyl]-2-méthylphényl]amino]-5-méthylpyrrolo[2,1-f][1,2,4]triazine-6-carboxamide;
le 4-[[5-[(méthoxyamino)carbonyl]-2-méthylphényl]amino]-5-méthyl-N-(2-phényléthyl)pyrrolo[2,1-f][1,2,4]triazine- 6-carboxamide;
le N-méthoxy-4-méthyl-3-[[5-méthyl-6-(1-pyrrolidinylcarbonyl)pyrrolo[2,1-f][1,2,4]triazin-4-yl]amino]benzamide;
le 4-[[5-[(méthoxyamino)carbonyl]-2-méthylphényl]amino]-5-méthyl-N-(2-pyridinylméthyl)pyrrolo[2,1-f][1,2,4]triazine-6-carboxamide;
le 4-[[5-[(méthoxyamino)carbonyl]-2-méthylphényl]amino]-5-méthyl-N-(phénylméthyl)pyrrolo[2,1-f][1,2,4]triazine-6-carboxamide;
le 4-[[5-[(méthoxyamino)carbonyl]-2-méthylphényl]amino]-5-méthyl-N-(4-méthyl-2-thiazolyl)pyrrolo[2,1-f][1,2,4]triazine-6-carboxamide;
le 4-[[5-[(méthoxyamino)carbonyl]-2-méthylphényl]amino]-5-méthyl-N-[(1R)-1-méthylpropyl] pyrrolo [2,1-f][1,2,4] triazine-6-carboxamide;
le 4-[[5-[(méthoxyamino)carbonyl]-2-méthylphényl]amino]-5-méthyl-N-[(1S)-1-méthylpropyl]pyrrolo[2,1-f][1,2,4]triazine-6-carboxamide;
le N-[(3-fluorophenyl)méthyl]-4-[[5-[(méthoxyamino)carbonyl]-2-méthylphényl]amino]-5-méthylpyrrolo[2,1-f][1,2,4]triazine-6-carboxamide;
le N-[1-(4-fluorophényl)éthyl]-4-[[5-[(méthoxyamino)carbonyl]-2-méthylphényl]amino]-5-méthylpyrrolo[2,1-f][1,2,4]triazine-6-carboxamide;
le N-[(2,4-difluorophényl)méthyl]-4-[[5-[(méthoxyamino) carbonyl]-2-méthylphényl]amino]-5-méthylpyrrolo[2,1-f][1,2,4] triazine-6-carboxamide; et
le N-[(2,6-difluorophényl)méthyl]-4-[[5-[(méthoxyamino) carbonyl]-2-méthylphényl]amino]-5-méthylpyrrolo[2,1-f][1,2,4] triazine-6-carboxamide;
le 4-[[5-[[(4-cyanophényl)amino]carbonyl]-2-méthylphényl]amino]-N-éthyl-5-méthylpyrrolo[2,1-f][1,2,4]triazine-6-carboxamide;
le 4-[[5-[[(4-cyanophényl)amino]carbonyl]-2-méthylphényl]amino]-5-méthyl-N-[(1S)-1-phényléthyl]pyrrolo[2,1-f][1,2,4]triazine-6-carboxamide;
le 4-[[5-[[[(4-cyanophényl)amino]carbonyl]amino]-2-méthyl phényl]amino]-5-méthyl-N-propylpyrrolo[2,1-f][1,2,4]triazine-6-carboxamide;
ou est (ii) un sel, hydrate ou promédicament pharmaceutiquement acceptable de celui-ci.

19. Composé selon la revendication 7, qui est choisi parmi le N-éthyl-4-[[5-[(méthoxyamino)carbonyl]-2-méthylphényl] amino]-5-méthylpyrrolo[2,1-f][1,2,4]triazine-6-carboxamide;
le 4-[[5-[(méthoxyamino)carbonyl]-2-méthylphényl]amino]-5-méthyl-N-(1-méthyléthyl)pyrrolo[2,1-f][1,2,4]triazine-6-carboxamide;
le 4-[[5-[(méthoxyamino)carbonyl]-2-méthylphényl]amino]-5-méthyl-N-propylpyrrolo[2,1-f][1,2,4]triazine-6-carboxamide; le 4-[[5-[(méthoxyamino)carbonyl]-2-méthyl.phényl]amino]-5-méthyl-N-[1-phényléthyl]pyrrolo[2,1-f][1,2,4]triazine-6-carboxamide;
et le 4-[[5-[(méthoxyamino)carbonyl]-2-méthylphényl]amino]-5-méthyl-N-[1-méthylpropyl] pyrrolo[2,1-f] [1,2,4]triazine-6-carboxamide;
et un sel pharmaceutiquement acceptable de celui-ci.

20. Composé répondant à la formule (II): 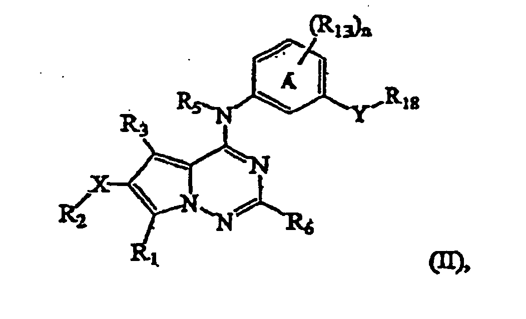
ou un sel, promédicament ou solvate pharmaceutiquement acceptable de celui-ci, dans laquelle R₃ est un groupe méthyle ou CF₃;
R₅ est un atome d'hydrogène ou un groupe alkyle inférieur;
Y est -C (=O) NR₂₃-, -NR₂₃C(=O)NR₂₃-, -NR₂₃SO₂- ou -SO₂NH₂-;
R₁₈ et R₂₃ sont choisis parmi un atome d'hydrogène, un groupe alkyle, alcoxy, aryle et aryle substitué par un à trois groupe(s) R₁₉, excepté que lorsque Y est -NR₂₃SO₂-, R₁₈ est un groupe alkyle en C_{1 à 4} ou aryle facultativement substitué par un à trois groupe (s) R₁₉;
X est choisi parmi -O-, -OC(=O)-, -S-, S(=O), -SO₂-, -C (=O) -, -CO₂-, -NR₁₀-,-NR₁₀C(=O)-,-NR₁₀C(=O)NR₁₁-, -NR₁₀CO₂-, -NR₁₀SO₂-, -NR₁₀SO₂NR₁₁-, -SO₂NR₁₀-, -C (=O) NR₁₀-, ou X est absent;
R₁ est un atome d'hydrogène -CH₃-, -OH, -OCH₃, -SH, -SCH₃, -OC (=O) R₂₁, -S (=O) R₂₂, -SO₂R₂₂, -SO₂NR₂₄R₂₅, -CO₂R₂₁, -C (=O) NR₂₄R₂₅, -NH₂-, -NR₂₁SO₂NR₂₄R₂₅, -NR₂₁SO₂R₂₂, -NR₂₄ (C=O) R₂₅, -NR₂₄CO₂R₂₅, -NR₂₁C (=O) NR₂₄R₂₅, un atome d' halogène, un groupe cyano ou nitro;
R₂ est un groupe alkyle, alkyle substitué, alcényle, alcényle substitué, alcynyle, alcynyle substitué, aryle, aryle substitué, hétérocyclo, hétérocyclo substitué, aralkyle, aralkyle substitué, hétérocycloalkyle ou hétérocycloalkyle substitué;
R₆ est un atome d'hydrogène, un groupe alkyle, alkyle substitué, aryle, aryle substitué, hétérocyclo, hétérocyclo substitué, -NR₇R₈, -OR₇ ou un atome d'halogène;
R₁₃ et R₁₉, à chaque occurrence, sont indépendamment choisis parmi un groupe alkyle, halogéno, trifluorométhoxy, trifluorométhyle, hydroxy, alcoxy, alconyle, alcanoyloxy, thiol, alkylthio, uréido, nitro, cyano, carboxy, carboxyalkyle, carbamyle, alcoxycarbonyle, alkylthiono, arylthiono, arylsulfonylamine, (alkyl en C_{1 à 4})sulfonylamine, acide sulfonique, alkylsulfonyle, sulfonamido et aryloxy, dans lequel chaque groupe R₁₃ et R₁₉ peut être en outre substitué par un groupe hydroxy, alkyle, alcoxy, aryle ou aralkyle;
R₇, R₈, R₂₁, R₂₄, et R₂₅ sont indépendamment choisis parmi un atome d'hydrogène, un groupe alkyle, alkyle substitué, aryle, aryle substitué, hétérocyclo et hétérocyclo substitué;
R₂₂ est un groupe alkyle, alkyle substitué, aryle, aryle substitué, hétérocyclo ou hétérocyclo substitué; et
n vaut 0, 1 ou 2.

21. Composé selon la revendication 20, répondant à la formule: 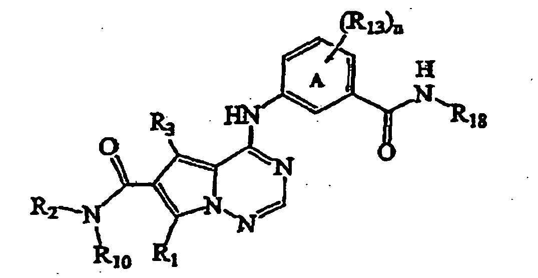
ou un sel, promédicament ou solvate pharmaceutiquement acceptable de celui-ci; dans laquelle
R₁₈ est un groupe alcoxy, aryle ou aryle substitué par un groupe R₁₉;
R₁ et R₁₀ sont un atome d'hydrogène ou -CH₃; et
R₁₃ est un groupe alkyle inférieur, un atome d'halogène, un groupe trifluorométhoxy, trifluorométhyle, hydroxy, alcoxy en C_{1 à 4}, nitro ou cyano.

22. Composé selon la revendication 20 répondant à la formule: 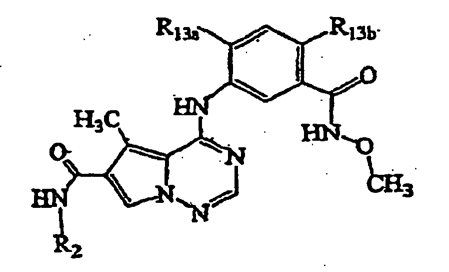
ou un sel, hydrate ou promédicament pharmaceutiquement acceptable de celui-ci, dans lequel R₂ est un groupe alkyle en C_{2 à 6} linéaire ou ramifié ou un groupe benzyle facultativement substitué, et R₁₃ₐ et R_{13b} sont choisis parmi un atome d'hydrogène, un groupe alkyle en C_{1 à 4}, hydroxy, un atome d'halogène, un groupe cyano et trifluorométhyle.

23. Composé selon la revendication 20, qui est le N-éthyl-4-[[5-[(méthoxyamino)carbonyl]-2-méthylphényl]amino]-5-méthylpyrrolo[2,1-f][1,2,4]triazine-6-carboxamide; ou un sel pharmaceutiquement acceptable comprenant un sel méthanesulfonique de celui-ci.

24. Composé selon la revendication 20, qui est le 4-[[5-[(méthoxyamino)carbonyl]-2-méthylphényl]amino]-5-méthyl-N-(1-méthyléthyl)pyrrolo[2,1-f][1,2,4]triazine-6-carboxamide; ou un sel pharmaceutiquement acceptable de celui-ci.

25. Composé selon la revendication 20, qui est le 4-[[5-[(méthoxyamino)carbonyl]-2-méthylphényl]amino]-5-méthyl-N-propylpyrrolo[2,1-f][1,2,4]triazine-6-carboxamide; ou un sel pharmaceutiquement acceptable de celui-ci.

26. Composé selon la revendication 20, qui est le 4-[[5-[(méthoxyamino)carbonyl]-2-méthylphényl]amino]-5-méthyl-N-[1-phényléthyl]pyrrolo[2,1-f][1,2;4]triazine-6-carboxamide; ou un sel pharmaceutiquement acceptable de celui-ci.

27. Composé selon la revendication 20, qui est le 4-[[5-[(méthoxyamino)carbonyl]-2-méthylphényl]amino]-5-méthyl-N-[1-méthylpropyl]pyrrolo[2,1-f][1,2,4]triazine-6-carboxamide; ou un sel pharmaceutiquement acceptable de celui-ci.

28. Composition pharmaceutique comprenant au moins un composé selon l'une quelconque des revendications 7 à 27, et un véhicule ou diluant pharmaceutiquement acceptable.

29. Utilisation d'une composition pharmaceutique selon la revendication 28 pour la fabrication d'un médicament destiné à traiter un trouble inflammatoire.

30. Utilisation selon la revendication 29, dans laquelle le trouble inflammatoire est choisi parmi l'asthme, le syndrome de détresse respiratoire de l'adulte, la bronchopneumopathie chronique obstructive, la bronchopneumo-pathie chronique inflammatoire, le diabète, l'affection abdominale inflammatoire, l'ostéoporose, le psoriasis, le rejet de greffe par l'hôte, l'athérosclérose, et l'arthrite comprenant la polyarthrite rhumatoïde, le rhumatisme psoriasique, l'arthrite traumatique, l'arthrite rubéoleuse, l'arthrite goutteuse et l'arthrose.
